# EUROPEAN PATENT SPECIFICATION

(11) **EP 2 984 107 B1**
(45) Date of publication and mention of the grant of the patent: **19.06.2019**
(21) Application number: 14715967.7
(22) Date of filing: 09.04.2014
(51) Int. Cl.: C07K 16/28, C07K 16/30, C07K 16/40, A61K 39/00

(54) **BISPECIFIC ANTIBODIES AGAINST CD3-EPSILON AND ROR1**
BISPEZIFISCHE ANTIKÖRPER GEGEN CD3-EPSILON UND ROR1
ANTICORPS BISPECIFIQUES CONTRE CD3-EPSILON ET ROR1

(30) Priority: 09.04.2013 EP 13001840
(43) Date of publication of application: 17.02.2016
(73) Proprietor: EngMab Sàrl, 2017 Boudry (CH)
(72) Inventor: VU, Minh Diem, CH-8832 Wollerau (CH); STREIN, Klaus, 69469 Weinheim (DE); MOESSNER, Ekkehard, CH-8280 Kreuzlingen (CH); HOSSE, Ralf, CH-6330 Cham (CH); AST, Oliver, CH-8303 Bassersdorf (CH); FREIMOSER-GRUNDSCHOBER, Anne, CH-8050 Zuerich (CH); FAUTI, Tanja, CH-8049 Zuerich (CH); MURR, Ramona, CH-8952 Schlieren (CH); KLEIN, Christian, CH-8906 Bonstetten (CH); UMANA, Pablo, CH-8832 Wollerau (CH); MOSER, Samuel, CH-6343 Rotkreuz (CH)
(74) Representative: Mathys & Squire LLP
(86) International application number: PCT/EP2014/057199
(87) International publication number: WO 2014/167022

(56) References cited:
- WO-A1-2011/054007
- WO-A1-2012/075158
- WO-A1-2013/026839
- D. MEZZANZANICA ET AL.: "Human ovrian carcinoma lysis by cytotoxic T cells targeted by bispecific monoclonal antibodies: Analysis of the antibody components.", INTERNATIONAL JOURNAL OF CANCER, vol. 41, 1988, pages 609-615, XP009056626, USA cited in the application
- BIOLOGICAL ABSTRACTS, vol. - 1 April 2017 (2017-04-01), Philadelphia, PA, US, abstract no.: 3629, AACR Annual Meeting, Klein et al.: "Engineering a novel asymmetric head-to-tail 2+1 T cell biospecific (2+1 TCB) IgG antibody platform with superior T cell killing compared to 1+1 asymmetric TCBs",
- DATABASE MEDLINE [Online] US NATIONAL LIBRARY OF MEDICINE (NLM), BETHESDA, MD, US; 15 November 1991 (1991-11-15), CONEY L R ET AL: "Cloning of a tumor-associated antigen: MOv18 and MOv19 antibodies recognize a folate-binding protein.", Database accession no. NLM1840502 & CANCER RESEARCH 15 NOV 1991, vol. 51, no. 22, 15 November 1991 (1991-11-15), pages 6125-6132, ISSN: 0008-5472
- MEZZANZANICA D ET AL: "HUMAN OVARIAN CARCINOMA LYSIS BY CYTOTOXIC T CELLS TARGETED BY BISPECIFIC MONOCLONAL ANTIBODIES: ANALYSIS OF THE ANTIBODY COMPONENTS", INTERNATIONAL JOURNAL OF CA, JOHN WILEY & SONS, INC, US, vol. 41, no. 4, 1 January 1988 (1988-01-01), pages 609-615, XP009056626, ISSN: 0020-7136, DOI: 10.1002/IJC.2910410422

## Description

The present invention relates to novel bispecific antibodies against CD3ε and ROR1, their manufacture and use.

### Background of the Invention

ROR1 (synonyms: tyrosine-protein kinase transmembrane receptor ROR1, EC=2.7.10.1, neurotrophic tyrosine kinase, receptor-related 1, UniPrtKB Q01973) is a tyrosine-protein kinase receptor. The receptor is described in Masiakowski P., Carroll R.D., J. Biol. Chem. 267:26181-26190(1992) "A novel family of cell surface receptors with tyrosine kinase-like domain." WO9218149 and WO9527060 mention ROR-1 as Rtk-2 and antibodies against ROR-1. WO2002087618 mentions a method of controlling the growth and differentiation of cancer by selectively inhibiting a growth factor receptor. Such a receptor would be Ror1 or Ror2. WO2005100605 mentions ROR1 as a therapeutic target for breast cancer and anti ROR1 antibodies which specifically bind to ROR1, to the extracellular region of ROR1 (M1-V406) and ROR1 fragments Q73-V139, E165-I299, K312-C391. WO2007051077 relates to an anti-ROR1 antibody and its use in lymphoma cell detection. WO2008103849 also mentions anti-ROR1 antibodies. Rabbani (Blood (ASH Annual Meeting Abstracts) 2010 116: Abstract 916) discloses the use of anti ROR1 antibodies for the treatment of chronic Lymphocytic leukemia (CLL). Rabbani used anti-ROR1 an antibody against the extracellular domain, an antibody against the CRD region (ligand binding site for Wnt proteins) and an antibody against the kringle domain. Daneshmanesh AH et al., Int. J. Cancer, 123 (2008) 1190-1195 relates to an anti ROR1 antibody that binds to the extracellular domain fragment WNISSELNKDSYLTL (SEQ ID NO:18) and an anti ROR1 antibody that binds to the intracellular domain fragment KSQKPYKIDSKQAS (SEQ ID NO:20). Also the use of such antibodies for the treatment of CLL is mentioned.

WO2011159847 relates to an anti-ROR1 antibody as a conjugate with a biologically active molecule for the treatment of ROR1 cancer like lymphoma or adenocarcinoma. WO2008076868, WO2008103849, WO201008069, WO2010124188, WO2011079902, WO2011054007, WO2011159847, WO2012076066, WO2012076727, WO 2012045085, and WO2012097313 relate also to ROR1 binding molecules or anti ROR1 antibodies. WO2012075158 relates to an anti-ROR1 antibody comprising as light chain variable domain (VL) the sequence of SEQ ID NO:2 and as variable heavy chain domain (VH) the sequence of SEQ ID NO:6, and as respective CDRs the sequences of SEQ ID NO: 3, 4, 5, 7, 8, 9.

WO2005040413 is directed to a screening method for the identification and/or validation of inhibitors of a receptor tyrosine kinase activity, including ROR1.

WO2008036449, WO2011014659 and WO2011050262 mention bispecific antibodies wherein one target can be ROR1. WO2007146968 mention multivalent single-chain binding proteins with effector function and ROR1 and CD3 are mentioned as possible targets. WO2011054007 is directed to a method of treatment of cancer administering an affinity reagent which binds to the extracellular domain of ROR1. Bispecific antibodies with CD3 are also mentioned. WO2014031174 mentions bispecific antibodies which are specific to two different epitopes of ROR1. The preferred antibody D10 strongly internalizes at 37°C in MDA MB 231 epithelial breast adenocarcinoma. Yang and Baskar PLos ONE 6 (2011) e21018, like WO2012075158, mention also anti-ROR1 antibody R12. Rebagay R. et al., Frontiers in Oncology (2012) 7, Article 34, 1-8 mention that RORs are pharmaceutical targets and a means to deliver cytotoxic agents in the cells which express the target on the cell surface. Rebagay also mention bispecific antibodies such as BiTE. Strong internalization is favorable for armed antibodies i.e. antibody drug conjugates according to Rebagay. D. MEZZANZANICA ET AL, INTERNATIONAL JOURNAL OF CANCER, 41 (1988) 609-615 investigated a therapeutic approach by retargeting CTLs by a bispecific antibody consisting of MOv18 (a poorly internalizing antibody specific for human ovarian carcinoma cells) and an anti-CD3 antibody (OKT3 or TR66). M. HUDECEK ET AL., BLOOD, 116 (2010), 4532-4541, mention that ROR1 is expressed by B cell chronic lymphocytic leukemia (B-CLL) and mantle cell lymphoma (MCL). Such cells can be targeted by activated CD8⁺ T cells transfected with, and expressing scFv from murine anti-ROR1 antibody 2A2. Such cells are useful for treatment of B cell malignancies. Baskar S. et al., mAbs 4:3 (2012) 349-361 relate to the targeting of malignant B cells with an immunotoxin BT-1 comprising scFv 2A2 anti-ROR1 conjugated to PE38 toxin. The immunotoxin is partially internalized and induces apotosis.

The TCR/CD3 complex of T-lymphocytes consists of either a TCR alpha (α)/beta (β) or TCR gamma (γ)/delta (δ) heterodimer coexpressed at the cell surface with the invariant subunits of CD3 labeled gamma (γ), delta (δ), epsilon (ε), zeta (ζ), and eta (η). Human CD3ε is described under UniProt P07766 (CD3E_HUMAN). An anti CD3ε antibody described in the state of the art is SP34 (Yang SJ, The Journal of Immunology (1986) 137; 1097-1100). SP34 reacts with both primate and human CD3. SP34 is available from Pharmingen. A further anti CD3 antibody described in the state of the art is UCHT-1 (see WO2000041474). A further anti CD3 antibody described in the state of the art is BC-3 (Fred Hutchinson Cancer Research Institute; used in Phase I/II trials of GvHD, Anasetti et al., Transplantation 54: 844 (1992)). SP34 differs from UCHT-1 and BC-3 in that SP-34 recognizes an epitope present on solely the ε chain of CD3 (see Salmeron et al., (1991) J. Immunol. 147: 3047) whereas UCHT-1 and BC-3 recognize an epitope contributed by both the ε and γ chains. An antibody with the same sequence as of antibody SP34 is mentioned in WO2008119565, WO2008119566, WO2008119567, WO2010037836, WO2010037837 and WO2010037838. An antibody VH which is 96% identical to VH of antibody SP34 is mentioned in US8236308 (WO2007042261).

A wide variety of recombinant bispecific antibody formats have been developed in the recent past, e.g. by fusion of, e.g. an IgG antibody format and single chain domains (see Kontermann RE, mAbs 4:2, (2012) 1-16). Bispecific antibodies wherein the variable domains VL and VH or the constant domains CL and CH1 or VL and VH are replaced by each other are described in WO2009080252 and WO2009080253.

An approach to engineer antibody heavy chain homodimers for heterodimerization which is known as 'knobs-into-holes', aims at forcing the pairing of two different antibody heavy chains by introducing mutations into the CH3 domains to modify the contact interface. (Ridgway JB, Presta LG, Carter P; and WO1996027011, Merchant A.M, et al, Nature Biotech 16 (1998) 677-681; Aτwell S, Ridgway JB, Wells JA, Carter P., J Mol Biol 270 (1997) 26-35). New approaches for the knobs-into-holes technology are described in e.g. in EP 1870459A1. Xie, Z., et al, J Immunol Methods 286 (2005) 95-101 refers to a format of bispecific antibody using scFvs in combination with knobs-into-holes technology for the FC part. WO 2012116927 and WO 2010/145792 mention exchanging the CH1 and CL domains. WO 2009/080254 mentions knob-into-hole constructs for producing bispecific antibodies.

WO2013026831 provides a bispecific antigen binding molecule, comprising a first Fab fragment which specifically binds to a first antigen, a second Fab fragment which specifically binds to a second antigen, and an Fc domain. WO2013026833 provides a T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain. WO2013026835 relates to bispecific antibodies comprising at least two Fab fragments, wherein the first Fab fragment comprises at least one antigen binding site specific for a first antigen; and the second Fab fragment comprises at least one antigen binding site specific for a second antigen wherein either the variable regions or the constant regions of the second Fab heavy and light chain are exchanged; and wherein the bispecific antibody is devoid of a Fc domain. Said antibody can additionally comprise a third Fab fragment. Said third Fab fragment can comprise at least one antigen binding site specific for the first or second antigen, preferably for the first antigen. WO2013026837 provides a T cell activating bispecific antigen binding molecule comprising a first and a second single chain Fv (scFv) molecule fused to each other, wherein the first scFv molecule is capable of specific binding to a target cell antigen and the second scFv molecule is capable of specific binding to an activating T cell antigen; characterized in that the T cell activating bispecific antigen binding molecule further comprises an Fc domain composed of a first and a second subunit capable of stable association.

WO2013026839 relates to bispecific antibodies that specifically bind a T-cell activating antigen and a Tumor Antigen (TA), comprising a first Fab fragment and a second Fab fragment, wherein either the variable regions or the constant regions of the second Fab heavy and light chain are exchanged; and wherein the bispecific antibody does not comprise a Fc domain. In one aspect of WO2013026839, a bispecific antibody that specifically binds a T-cell activating antigen and a Tumor Antigen (TA) is provided, comprising at least two fab fragments, wherein the first Fab fragment comprises at least one antigen binding site specific for a Tumor Antigen (TA); and the second Fab fragment comprises at least one antigen binding site specific for a T-cell activating antigen, wherein either the variable regions or the constant regions of the second Fab heavy and light chain are exchanged; and wherein the bispecific antibody is devoid of a Fc domain. WO2013026839 relates also to bispecific antibodies wherein the T-cell activating antigen is a CD3 T-Cell Co-Receptor (CD3) targeting antigen. In one embodiment the first and second Fab fragments are connected via a peptide linker. Preferably said peptide linker is a (G4S)₂ linker.

### Summary of the Invention

Based on the disclosure that is contained herein, the invention provides a bispecific antibody specifically binding to the two targets human CD3ε and the extracellular domain of human ROR1 (ROR1), wherein the bispecific antibody has the construct ROR1 Fab - Fc - CD3 Fab - ROR1 Fab.

In a further aspect the present invention provides a method for the preparation of the bispecific antibody of the invention.

In a further aspect the present invention provides a host cell comprising nucleic acid molecules encoding the antibody of the invention.

In vet another aspect, the present invention provides a pharmaceutical composition comprising the antibody of the invention and a pharmaceutically acceptable excipient.

In vet another aspect, the present invention provides the bispecific antibody or the pharmaceutical composition of the invention for use as a medicament.

In another aspect, the present invention provides the bispecific antibody or the pharmaceutical composition of the invention for use as a medicament in the treatment of a ROR1-positive hematological malignancy, a ROR1-positive solid tumor, chronic lymphocytic leukemia of B-cell lineage (B-CLL) or a plasma cell disorder.

The present invention and embodiments thereof are set out in the appended claims.

The invention relates to a bispecific antibody specifically binding to the two targets human CD3ε (further named also as "CD3") and the extracellular domain of human ROR1 (further named also as "ROR1"). The invention relates to a bispecific antibody specifically binding to the two targets human CD3ε and the extracellular domain of human ROR1 which does not internalize. The bispecific antibody according to the invention is preferably characterized in not internalizing in a concentration of lnM in primary B-CLL cells at 37°C during two hours. The bispecific antibody according to the invention is preferably characterized in that the bispecific antibody does not internalize in a cell based assay at 37°C during 2 hrs, using ROR1-positive primary B-CLL cells and used at an antibody concentration of 1 nM, whereby not internalize means, that the mean fluorescence intensity (MFI), as detected by flow cytometry, of a bispecific antibody upon
binding to ROR1-positive primary B-CLL cells measured at time 0 is not reduced more than 50%, preferably not more than 30% when re-measured after a 2hr-incubation at 37°C.

Preferably the bispecific antibody according to the disclosure is characterized in consisting of one Fab fragment of an anti-CD3 antibody (CD3 Fab), one or two Fab fragments of an anti-ROR1 antibody (ROR1 Fab) and no or one Fc fragment. Preferably the bispecific antibody according to the disclosure is characterized in comprising a monovalent anti-ROR1 antibody specifically binding to ROR1, and a monovalent antibody specifically binding to CD3. Preferably the bispecific antibody according to the disclosure is characterized in being bivalent and comprising a monovalent anti-ROR1 antibody specifically binding to ROR1, and a monovalent antibody specifically binding to CD3. Preferably the bispecific antibody according to the invention is characterized in being trivalent and comprising a bivalent anti-ROR1 antibody specifically binding to ROR1, and a monovalent Fab fragment of an antibody specifically binding to CD3

Preferably in the light chain and heavy chain of the CD3 Fab the variable domains VL and VH or the constant domains CL and CH1 are replaced by each other (CD3 crossFab). The CD3 Fab is N-terminally linked to the C-terminus to the ROR1 Fab. Preferably the VH domain of the CD3 Fab is N-terminally linked to the C-terminus of the CH1 domain of the ROR1 Fab. The Fc part is linked via its hinge region to the C-terminus of the respective Fab.

Preferably the bispecific antibody according to the disclosure is selected from the group of the constructs
a) CD3 Fab - ROR1 Fab,
b) CD3 Fab - ROR1 Fab - ROR1 Fab,
c) Fc - CD3 Fab - ROR1 Fab, and
d) ROR1 Fab - Fc - CD3 Fab - ROR1 Fab.

The preferred constructs comprise as CD3 Fab a CD3 crossFab. The two ROR1 Fabs of constructs b) and d) are derived from the same anti-ROR1 antibody and comprise at least the same CDRs or the same VH, VL, CH1, and CL domains.

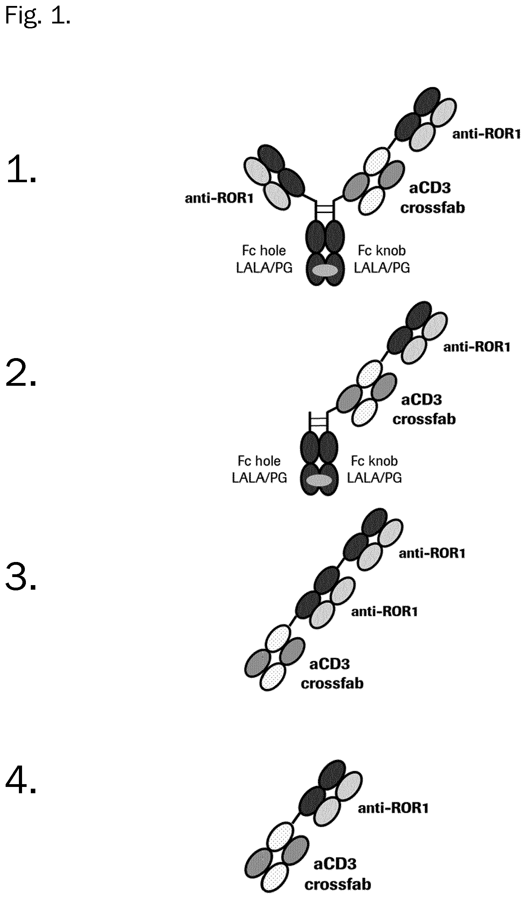

The preferred bispecific antibodies are shown in Figure 1.

The constructs are composed of the building blocks of SEQ ID NO: 29 to 36. The disclosure comprises therefore a polypeptide selected from the group consisting of the polypeptides of SEQ ID NO: 29, 30, 31, 32, 33, 34, 35, and 36, the respective nucleic acids and their use for the preparation of the constructs..

The disclosure relates further to a construct selected from the group of
a) construct consisting of building blocks SEQ ID NO:30 (2x), 31, 32, and 33 (Fig.1.1)
b) construct consisting of building blocks SEQ ID NO:30, 31, 33, and 36 (Fig.1.2)
c) construct consisting of building blocks SEQ ID NO:30 (2x), 33, and 35 (Fig.1.3)
d) construct consisting of building blocks SEQ ID NO: 30, 33, and 34 (Fig.1.4)

In a further embodiment the CD3 Mab sequences (VH and/or VL) within SEQ ID NO: 31, 33, 34, 35 are replaced by the respective VH and/or VL sequences of SEQ ID NO:10 and 11.

The invention relates to a bispecific antibody specifically binding to the two targets human CD3ε (further named also as "CD3") and the extracellular domain of human ROR1 (further named also as "ROR1"), characterized in that the bispecific antibody does not internalize in a cell based assay at 37°C during 2 hrs, using ROR1-positive primary B-CLL cells, and used at an antibody concentration of 1 nM, whereby not internalize means, that the mean fluorescence intensity (MFI), as detected by flow cytometry, of a bispecific antibody upon binding to ROR1-positive primary B-CLL cells measured at time 0 is not reduced more than 50%, preferably not more than 30% when re-measured after a 2hr-incubation at 37°C.

In a further embodiment the invention relates to a bispecific antibody specifically binding to the two targets human CD3ε (further named also as "CD3") and the extracellular domain of human ROR1 (further named also as "ROR1"), characterized in that the bispecific antibody does not internalize in a cell based assay at 37°C during 2 hrs, using EHEB B-CLL cell line DSMZ ACC-67, and used at an antibody concentration of 1 nM, whereby not internalize means, that the mean fluorescence intensity (MFI), as detected by flow cytometry, of a bispecific antibody upon binding to ROR1-positive primary B-CLL cells measured at time 0 is not reduced more than 50%, preferably not more than 30% when re-measured after a 2hr-incubation at 37°C.

Preferably the bispecific antibody according to the disclosure is devoid of a Fc domain. Preferably the bispecific antibody according to the invention comprises a Fc domain.

Alternatively the bispecific antibody can comprise instead of the Fabs single chains consisting of the same domains. In such a case the variable domains VL and VH or the constant domains CL and CH1 are not replaced by each other.

Preferably the bispecific antibody according to the disclosure is a bivalent antibody and characterized in comprising a monovalent anti-ROR1 antibody specifically binding to ROR1, and a monovalent antibody specifically binding to CD3. A bivalent antibody is preferred if its said mean fluorescence intensity (MFI), as detected by flow cytometry, upon binding to ROR1-positive cells measured at time 0 is not reduced more than 50%, preferably not more than 30% by internalization when re-measured after a 2hr-incubation at 37°C. Preferably the bispecific antibody according to the disclosure is a bivalent antibody and characterized in comprising a monovalent anti-ROR1 antibody specifically binding to ROR1, and a monovalent antibody specifically binding to CD3. Preferably the monovalent antibody specifically binding to CD3 is a Fab fragment, preferably a CD3 crossFab. Such a bivalent antibody is preferred if its said mean fluorescence intensity (MFI), as detected by flow cytometry, upon binding to ROR1-positive cells measured at time 0 is not reduced more than 50%, preferably not more than 30% by internalization when re-measured after a 2hr-incubation at 37°C. Preferably the bispecific antibody according to the invention is a trivalent antibody and characterized in comprising a bivalent anti-ROR1 antibody specifically binding to ROR1, and a monovalent antibody specifically binding to CD3. Preferably the monovalent antibody specifically binding to CD3 is a Fab fragment or preferably a CD3 crossFab. A trivalent antibody is preferred if its said mean fluorescence intensity (MFI), as detected by flow cytometry, upon binding to ROR1-positive cells measured at time 0 is not reduced more than 50%, preferably not more than 30% by internalization when re-measured after a 2hr-incubation at 37°C.

Preferably the bispecific antibody according to the invention is characterized in that the bispecific antibody does not internalize in said cell based assay at 37°C during 24 hrs.

Preferably the bispecific antibody according the invention does not internalize in said cell based assay if used in a concentration between 0.1 pM and 200 nM.

A further embodiment of the invention is an antibody according to this invention with an affinity ratio of ROR1 to CD3 of 5000:1 to 5:1, as determined by Kd values using surface plasmon resonance. Such an antibody is favorable because of its stronger binding to malignant cells over T cells. Preferably the Kd values are about 100 nM for the CD3 antibody and about 50 pM to 50 nM for the ROR1 antibody.

The invention relates to a bispecific antibody specifically binding to the two targets human CD3ε (further named also as "CD3") and the extracellular domain of human ROR1 (further named also as "ROR1"), characterized in that
i) said bispecific antibody is a fusion protein of a Fab fragment of an anti-CD3 antibody chemically linked at its N-terminus to the C-terminus of a Fab fragment of an anti-ROR1 antibody and
ii) the variable domains VL and VH or the constant domains CL and CH1 of the anti-CD3 antibody are replaced by each other.

Preferably the VH domain of the CD3 Fab is linked to the CH1 domain of the ROR1 Fab. Preferably the VL domain of the CD3 Fab is linked to the CL domain of said anti-ROR1 Fab.

The Fab fragments are chemically linked together by the use of an appropriate linker according to the state of the art. Preferably a (Gly4-Ser1)2 linker (double Gly-Ser linker, SEQ ID NO:19) or a triple Gly-Ser linker is used (Desplancq DK et al., Protein Eng. 1994 Aug;7(8):1027-33 and Mack M. et al., PNAS July 18, 1995 vol. 92 no. 15 7021-7025).

Preferably the B-CLL cells are used according to the invention in a cell concentration of 15 x 10⁶ cells/mL (primary PBMC from CLL patients) or 2 x 10⁶ cells/mL (ACC-67).

Preferably the antibody according to the invention is further characterized in that it binds also specifically to cynomolgus ROR1.

A further instance of the disclosure is a bispecific antibody specifically binding to the two targets human CD3ε (further named also as "CD3") and the extracellular domain of human ROR1 (further named also as "ROR1"), characterized in comprising one Fab fragment of an antibody specifically binding to one of said targets and one Fab fragment of an antibody specifically binding to the other one of said targets.

A further instance of the disclosure is a bispecific antibody specifically binding to the two targets human CD3ε (further named also as "CD3") and the extracellular domain of human ROR1 (further named also as "ROR1"), characterized in comprising one Fab fragment of an antibody specifically binding to one of said targets and two Fab fragment of an antibody specifically binding to the other one of said targets.

Preferably the antibody portion specifically binding to human CD3 (H2C) is characterized in comprising a variable heavy chain domain VH comprising the CDRs of SEQ ID NO: 12, 13 and 14 as respectively heavy chain CDR1, CDR2 and CDR3 and a variable domain VL comprising the CDRs of SEQ ID NO: 15, 16 and 17 as respectively light chain CDR1, CDR2 and CDR3. Preferably the antibody portion specifically binding to human CD3 is characterized in that the variable domains are of SEQ ID NO:10 (VH) and 11 (VL). Preferably the antibody portion specifically binding to human CD3 (CH2527) is characterized in comprising a variable heavy chain domain VH comprising the CDRs of SEQ ID NO: 23, 24 and 25 as respectively heavy chain CDR1, CDR2 and CDR3 and a variable domain VL comprising the CDRs of SEQ ID NO: 26, 27 and 28 as respectively light chain CDR1, CDR2 and CDR3. Preferably the antibody portion specifically binding to human CD3 is characterized in that the variable domains are of SEQ ID NO:21 (VH) and 22 (VL). Preferably the antibody portion specifically binding to CD3 is characterized in being humanized. Preferably the CD3 Mab according to the invention binds to the same epitope of CD3ε as H2C and/or CH2527.

Preferably the antibody portion specifically binding to ROR1 is characterized in comprising a light chain variable domain (VL) comprising as respective variable light chain CDRs the CDRs of SEQ ID NO: 3, 4, 5 and a heavy chain variable domain (VH) comprising as respective variable heavy chain CDRs the CDRs of SEQ ID NO:7, 8, 9. Preferably the antibody portion specifically binding to ROR1 is characterized in comprising as light chain variable domain (VL) a sequence being at least 90% identical to the sequence of SEQ ID NO:2 and as variable heavy chain domain (VH) a sequence being at least 90% identical to the sequence of SEQ ID NO:6, Preferably the antibody portion specifically binding to ROR1 is characterized in comprising as light chain variable domain (VL) the sequence of SEQ ID NO:2 and as variable heavy chain domain (VH) the sequence of SEQ ID NO:6. Preferably the antibody portion specifically binding to ROR1 is characterized in being humanized. Preferably the ROR1 Mab according to the invention binds to the same epitope of ROR1 as the Mab mentioned above.

A further embodiment of the invention is a method for the preparation of a bispecific antibody according to the invention comprising the steps of transforming a host cell with one or more vectors comprising nucleic acid molecules encoding the respective antibodies or fragments, culturing the host cell under conditions that allow synthesis of said antibody molecule; and recovering said antibody molecule from said culture.

Preferably the method for the preparation of a bispecific antibody according to the disclosure comprising the steps of transforming a host cell with one or more vectors comprising nucleic acid molecules encoding
a) a F(ab)₂ fragment of an anti-ROR1 antibody specifically binding to ROR1, wherein in one arm the variable domains VL and VH or the constant domains CL and CH1 are replaced by each other and
b) a Fab fragment of an antibody specifically binding to CD3 bound to the N-terminus of one VL or VH domain of said antibody F(ab)₂ fragment specifically binding to ROR1.
c) culturing the host cell under conditions that allow synthesis of said antibody molecule; and
d) recovering said antibody molecule from said culture.

Preferably the method for the preparation of a bispecific antibody according to the disclosure is characterized in comprising the steps of
a) transforming a host cell with vectors comprising nucleic acid molecules encoding polypeptides, which form together an antibody molecule which is a F(ab)₂ fragment of an anti-ROR1 antibody specifically binding to ROR1, wherein in one arm the variable domains VL and VH or the constant domains CL and CH1 are replaced by each other and a Fab fragment or a single chain Fv fragment of an antibody specifically binding to CD3 which is linked to the N-terminus of one VL or VH domain of said antibody F(ab)₂ fragment specifically binding to ROR1,
b) culturing the host cell under conditions that allow synthesis of said antibody molecule; and
c) recovering said antibody molecule from said culture.

A further embodiment of the invention is a host cell comprising vectors comprising nucleic acid molecules encoding an antibody according to the invention.

A further embodiment of the invention is a host cell comprising vectors comprising nucleic acid molecules encoding the light chain and heavy chain of an antibody specifically binding to the first target and vectors comprising nucleic acid molecules encoding the light chain and heavy chain of an antibody specifically binding to the second target, wherein the variable domains VL and VH or the constant domains CL and CH1 are replaced by each other.

A further preferred embodiment of the invention is a pharmaceutical composition comprising an antibody according to the invention and a pharmaceutically acceptable excipient.

A further preferred embodiment of the invention is a pharmaceutical composition comprising an antibody according to the invention for use as a medicament. A further preferred embodiment of the invention is an antibody according to the invention or a pharmaceutical composition comprising an antibody according to the invention for use as a medicament in the treatment of ROR1-positive hematological malignancies comprising chronic lymphocytic leukemia (CLL), hairy cell leukemia (HCL), acute lymphoblastic leukemia (ALL), acute myeloid leukemia (AML), chronic myeloid leukemia (CML), mantle cell lymphoma (MCL), marginal zone lymphoma (MZL), diffuse large B cell lymphoma (DLBCL), multiple myeloma (MM) and follicular lymphoma (FL). ROR1 is significantly and uniformly expressed on the cell surface of these various blood cancers. A further embodiment of the invention is an antibody according to the invention or a pharmaceutical composition comprising an antibody according to the invention for use as a medicament in the treatment of leukemias and non-Hodgkin lymphomas expressing ROR1. A preferred embodiment of the invention is an antibody according to the invention or a pharmaceutical composition comprising an antibody according to the invention for use as a medicament in the treatment of chronic lymphocytic leukemia (CLL) of B-cell lineage (B-CLL). B-CLL results from an acquired mutation to the DNA of a single marrow cell that develops into a B lymphocyte. Once the marrow cell undergoes the leukemic change, it multiplies into many cells and overtime crowd out normal cells since CLL cells grow and survive better than normal cells. The result is the uncontrolled growth of CLL cells in the bone marrow, leading to an increase in the number CLL cells in the blood. CLL symptoms usually develop over time with some patients being asymptomatic with only abnormal blood test results (e.g. increase in white blood cells). CLL patients with symptoms experience fatigue, short of breath, anemia, weight loss, decrease in appetite, lymph nodes and spleen enlargement and recurrent infections due to low immunoglobulin levels and decreased neutrophil counts (Leukemia & Lymphoma Society, 2009). A further preferred embodiment of the invention is an antibody according to the invention or a pharmaceutical composition comprising an antibody according to the invention for use as a medicament in the treatment of Multiple Myeloma. A further embodiment of the invention is an antibody according to the invention or a pharmaceutical composition comprising an antibody according to the invention for use as a medicament the treatment of plasma cell disorders like Multiple Myeloma MM or other B-cell disorders expressing ROR1. MM is a B-cell malignancy characterized by a monoclonal expansion and accumulation of abnormal plasma cells in the bone marrow compartment. MM also involves circulating clonal B cells with same IgG gene rearrangement and somatic hypermutation. MM arises from an asymptomatic, premalignant condition called monoclonal gammopathy of unknown significance (MGUS), characterized by low levels of bone marrow plasma cells and a monoclonal protein. MM cells proliferate at low rate. MM results from a progressive occurrence of multiple structural chromosomal changes (e.g. unbalanced translocations). MM involves the mutual interaction of malignant plasma cells and bone marrow microenvironment (e.g. normal bone marrow stromal cells). Clinical signs of active MM include monoclonal antibody spike, plasma cells overcrowding the bone marrow, lytic bone lesions and bone destruction resulting from overstimulation of osteoclasts (Dimopulos & Terpos, Ann Oncol 2010; 21 suppl 7: vii143-150). A further embodiment of the invention is an antibody according to the invention or a pharmaceutical composition comprising an antibody according to the invention for use as a medicament in the treatment of ROR1-positive solid tumors such as human breast cancers (Zhang S, PLoS One 2012; 7(3): e31127) and lung cancers (Yamaguchi T, Cancer Cell 2012; 21(3):348).

A further instance of the disclosure is the use of an antibody according to the invention or the pharmaceutical composition according to the invention for such treatments.

Preferably the antibody according to the invention or the pharmaceutical composition is administered once or twice a week preferably via subcutaneous administration (e.g. preferably in the dose range of 0.25 to 2.5 mg/m²/week or twice a week). Due to superior cytotoxicity activities of the antibody according to the invention it can be administered at least at the same magnitude of clinical dose range (or even lower) as compared to conventional monospecific antibodies or conventional bispecific antibodies that are not T cell bispecifics (i.e. do not bind to CD3 on one arm). It is envisaged that for an antibody according to the invention subcutaneous administration is preferred in the clinical settings (e.g. in the dose range of 100 - 1000 mg/m²/week or twice a week). An antibody according to the invention comprising an Fc part is eliminated with a half-life of about 12 days which allows at least once or twice/week administration Another advantage of the antibody according to the invention is a molecular weight (i.e. approximately 100 - 150 kDa) higher than the kidney filtration size limit (50 -70 kDa) even if the antibody has no Fc part. This molecular weight allows 6 - 72 hrs elimination half-life and makes subcutaneous administrations once or twice a week possible.

### Description of the Figures

Figure 1. Preferred bispecific antibodies comprising the Fab fragments (specific to CD3 and ROR1) with or without the Fc part as specified: (1) Fab ROR1-Fc-Fab CD3-Fab ROR1; (2) Fc-Fab CD3-Fab ROR1; (3) Fab CD3- Fab ROR1- Fab ROR1; (4) CD3 Fab-ROR1 Fab. Preferably, the Fabs CD3 include a CH1-CL crossover to reduce LC mispairing and side-products. Fab CD3 and Fab ROR1 are linked to each other with flexible linkers.

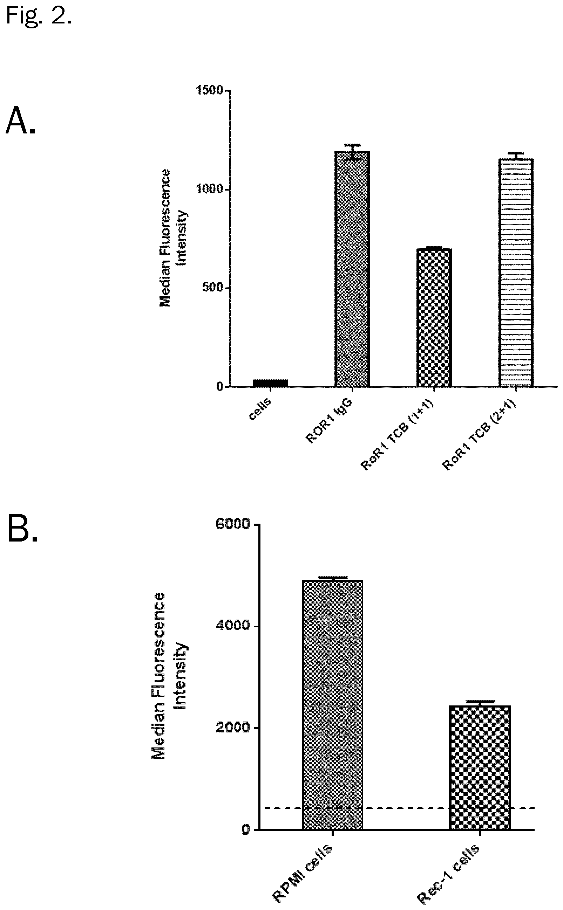

Figure 2. Detection of ROR1 on the cell surface of (A) primary CLL cells and (B) RPMI8226 MM cells and Rec-1 MCL cells using Alexa488-labelled anti-RORlIgG antibody or Alexa647-labelled anti-human Fc antibody. Graphs showing increase in MFI signal as compared to baseline.

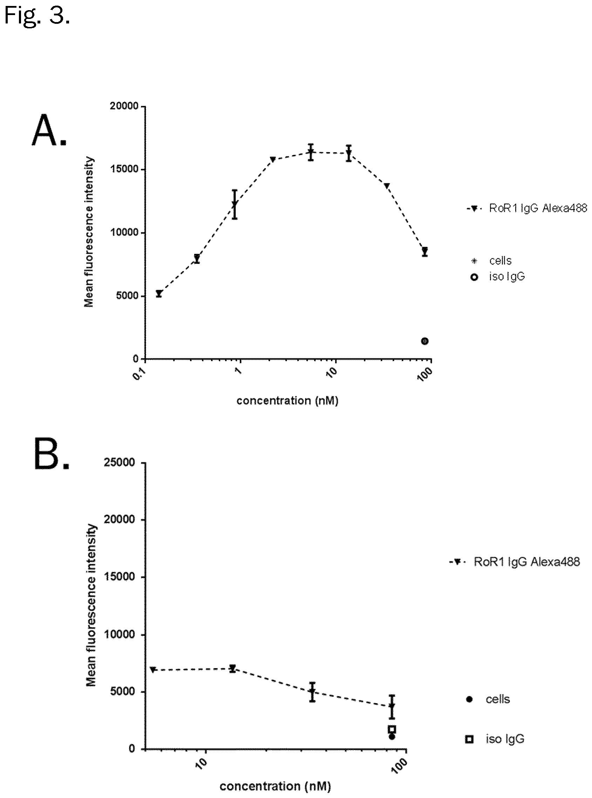

Figure 3. Binding of anti-ROR1 IgG1 antibody on ROR1-positive RPMI8226 cells (A) and non-binding to ROR1-negative MKN45 cells (B). Mean fluorescence intensity for anti-ROR1 IgG plotted in function of anti-ROR1 antibody concentrations (from 0.14 to 100 nM).

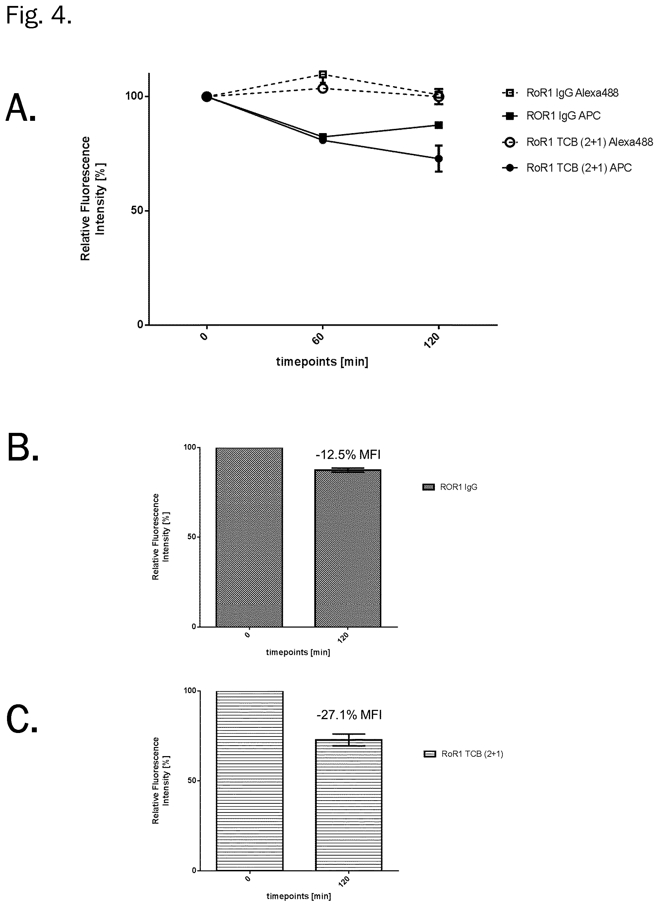

Figure 4. Internalization rate (%) of (A, B) anti-ROR1 IgG1 antibody at a concentration of 1 nM and (A, C) anti-ROR1/anti-CD3 TCB2+1 antibody on ROR1-positive primary B-CLL cells after 2h incubation at 37°C, as detected by FACS using secondary labelled anti-human Fc antibody (indirect detection). (A, B) Anti-ROR1 IgG antibody (1nM) internalized about 12.5% in primary B-CLL cells. (A, C) Anti-ROR1/anti-CD3 TCB2+1 antibody (1 nM) showed an internalization rate of 27.1% in primary B-CLL cells at the same experimental conditions as measured by FACS (indirect detection). Internalization was calculated based on the MFI value at time 0, baseline, and calculated using the previously described formula.

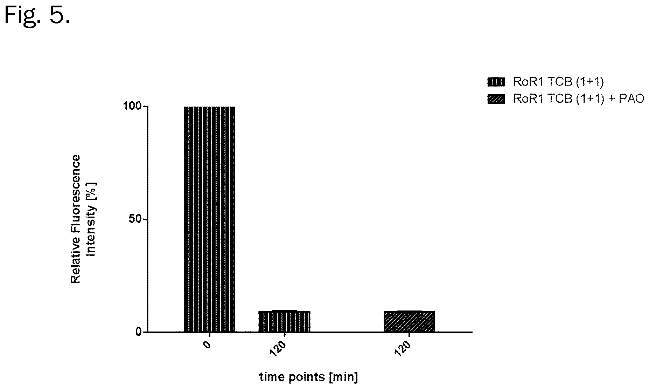

Figure 5. Internalization rate (%) of anti-ROR1/anti-CD3 TCB1+1 antibody (1 nM) in primary B-CLL cells after an incubation of 2 hrs at 37°C in the presence or absence of phenylarsine oxide (PAO) as detected by FACS using secondary labelled anti-human Fc antibody (indirect detection). A decrease of 91% in the MFI signal was observed in primary B-CLL cells after an incubation of 2 hrs at 37°C without PAO. However, when the B-CLL cells were incubated in the presence of PAO (3 µM), 90% decrease in MFI signal was still observed indicating that the loss in MFI signal was not due to internalization of the antibody but rather probably dissociation.

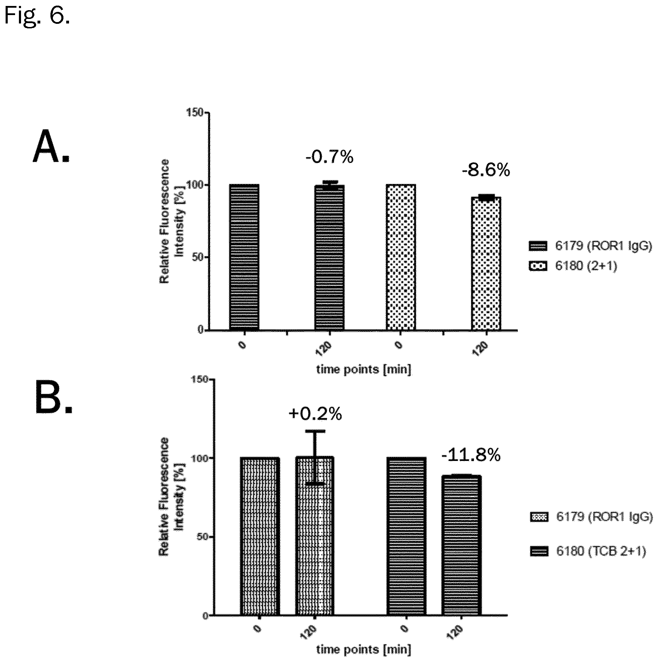

Figure 6. Internalization rates of TCB2+1 antibodies and anti-ROR1 IgG antibody (1 nM) in RPMI8226 MM cells after an incubation of 2 hrs at 37°C, as measured in two independent experiments. The results demonstrate that anti-ROR1/anti-CD3 TCB2+1 has an internalization rate of less than 15% in RPMI cells.

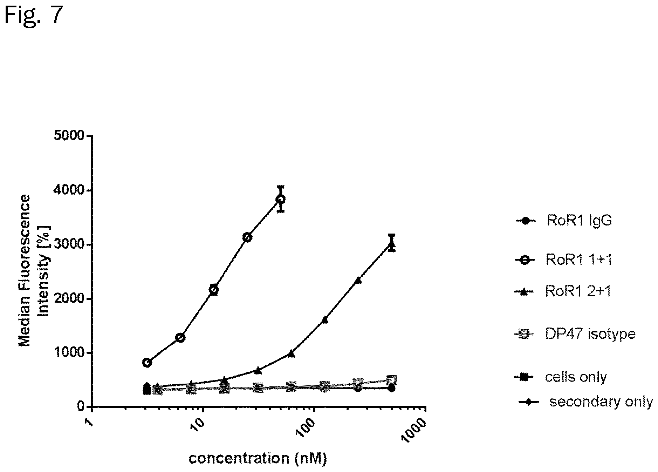

Figure 7. Binding of anti-ROR1/anti-CD3 TCB antibodies on Jurkat T cells. Mean fluorescence intensity for anti-ROR1/anti-CD3 T cell bispecific antibodies plotted in function of antibody concentrations (from 3 to 500 nM); anti-ROR1/anti-CD3 TCB1+1 and anti-ROR1/anti-CD3 TCB2+1 antibodies on Jurkat cells. EC50 values and maximal binding of anti-ROR1/anti-CD3 TCB1+1 and anti-ROR1/anti-CD3 TCB2+1 antibodies to Jurkat cells were not reached. DP47 isotype control antibody or anti-ROR1 IgG antibody did not bind to Jurkat T cells.

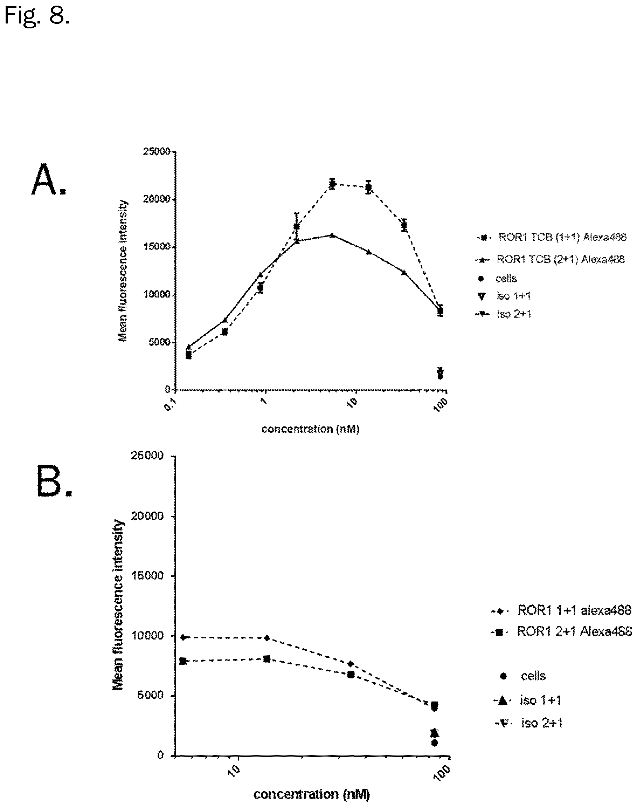

Figure 8. Binding of anti-ROR1/anti-CD3 TCB antibodies on (A) ROR1-positive RPMI8226 cells and non-binding to (B) ROR1-negative MKN45 cells. Mean fluorescence intensity plotted in function of antibody concentrations (from 0.14 to 100 nM).

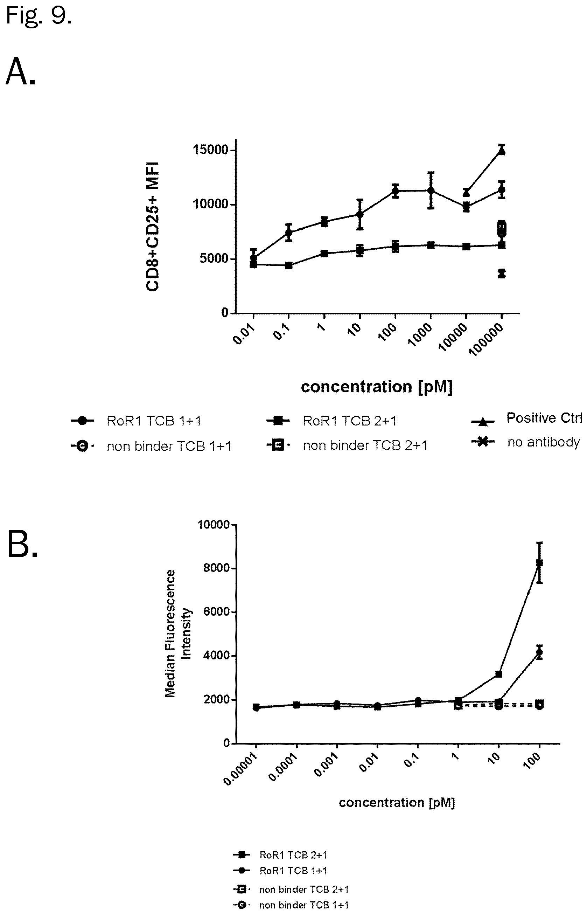

Figure 9. Up-regulation of activation markers by anti-ROR1/anti-CD3 TCB antibodies on ROR1-positive (A) Rec-1 cells and (B) RPMI8226 cells. Mean fluorescence intensity plotted in function of antibody concentrations (from 0.01 pM to 100 nM). (A) A concentration dependent increase in the mean fluorescence intensity of the late activation marker CD25 gated on CD8 T cells was observed in Rec-1 cells. Significant concentration dependent activation of CD8 T cells by anti-ROR1/anti-CD3 TCB1+1 antibody in the presence of ROR1-positive Rec-1 cells. Maximum signal reached at 100 pM of antibody. Unspecific activation of CD8 T cells was minimal upon binding of CD3 on T cells but without binding on ROR1-positive target cells by using non-binder TCB constructs. Activation of CD8 T cells was weak with anti-ROR1/anti-CD3 TCB2+1 antibody as shown by a faint but noticeable increase in CD25 mean fluorescence intensity. However, unspecific T cell activation could not be ruled out. (B) Concentration dependent upregulation of CD25 on CD8 T cells mediated by anti-ROR1/anti-CD3 TCB1+1 and anti-ROR1/anti-CD3 TCB2+1 antibodies in the presence of ROR1-positive RPMI8226 MM cells. At the highest concentration (100 pM) of TCB antibodies tested there was no unspecific activation of CD8 T cells as shown with non-binder TCB constructs.

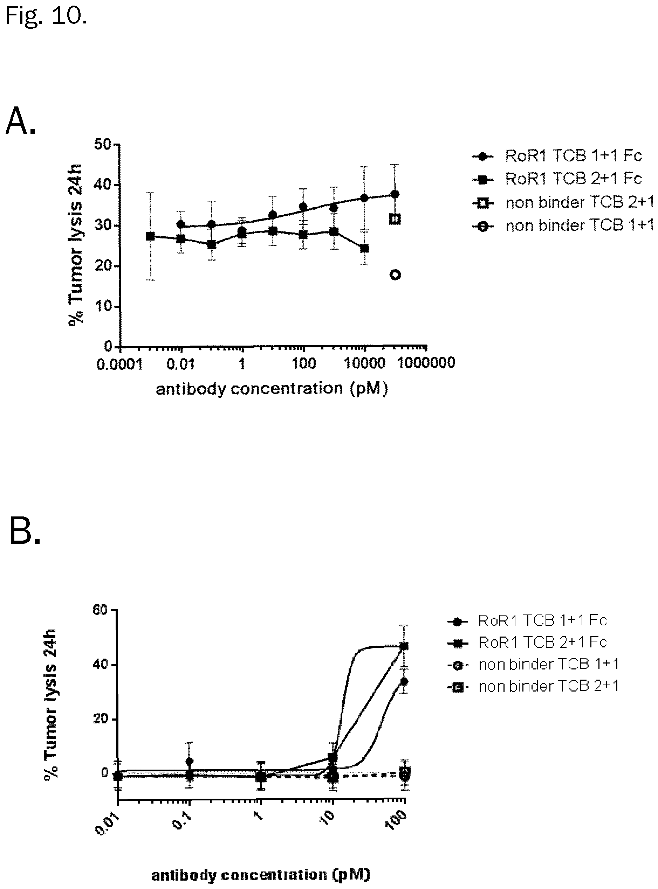

Figure 10. Redirected T cell killing of ROR1-positive RPMI8226 MM target cells by CD8 T cells activated by anti-ROR1/anti-CD3 TCB antibodies. Specific cytotoxicity of target cells (tumor lysis) induced by anti-ROR1/anti-CD3 TCB antibodies was measured by LDH release. (A) Experiment 1 (14h time point): 30% of tumor lysis was already observed with the lowest concentration tested of 0.01 pM anti-ROR1/anti-CD3 TCB1+1 antibody and up to 37.5% tumor lysis was reached with 30 nM of anti-ROR1/anti-CD3 TCB antibodies in experimental conditions reflecting clinically relevant E:T ratio of 3:1 i.e. 3 CD8 T cells for 1 RPMI 8226 target cell. The 37.5% tumor lysis observed at 30 nM as detected by LDH release could not have been attributed only to unspecific killing of target cells as there was only 17% unspecific target cell lysis with 30 nM of non-binder TCB1+1 (i.e. binds to effector cells but not to target cells). For anti-ROR1/anti-CD3 TCB2+1 antibody, a maximum target cell lysis of 30% was already observed at the lowest concentration tested of 0.2 fM and there was no concentration dependent response with increasing concentrations for up to 10 nM. 30 nM non-binding TCB2+1 had close to 30% tumor lysis. (B) Experiment 2 (20h time point): 30-40% target cell lysis was observed with anti-ROR1/anti-CD3 TCB1+1 and TCB2+1 antibodies at a concentration of 100 pM while non-binder TCB controls did not induce any tumor lysis at the same concentration.

### Detailed Description of the Invention

The term "ROR1" as used herein relates to humanROR1 (synonyms: tyrosine-protein kinase transmembrane receptor ROR1, EC=2.7.10.1, neurotrophic tyrosine kinase, receptor-related 1, UniPrtKB Q01973) which is a tyrosine-protein kinase receptor. The extracellular domain of ROR1 consists according to UniProt of amino acids 30 - 406. The term "antibody against ROR1, anti ROR1 antibody or ROR1 Mab" as used herein relates to an antibody specifically binding to human ROR1. The antibody binds specifically to the extracellular domain of ROR1 (amino acids M1-V406 of SEQ ID NO:1). The antibody binds specifically to fragments of the extracellular domain, which are the Ig-like C2-type domain (amino acids Q73-V139 of SEQ ID NO:1), the frizzled domain (amino acids E165-I299 of SEQ ID NO: 1), or the kringle domain (amino acids K312-C391 of SEQ ID NO:1). These fragments are mentioned in WO2005100605. It is further preferred that the antibody binds specifically to the extracellular domain fragment WNISSELNKDSYLTL (SEQ ID NO.18) of ROR1. This fragment is mentioned in Daneshmanesh AH et al., Int. J. Cancer, 123 (2008) 1190-1195.

The term "CD3ε or CD3" as used herein relates to human CD3ε described under UniProt P07766 (CD3E_HUMAN). The term "antibody against CD3, anti CD3 antibody" relates to an antibody binding to CD3ε. Preferably the antibody comprises a variable domain VH comprising the heavy chain CDRs of SEQ ID NO: 12, 13 and 14 as respectively heavy chain CDR1, CDR2 and CDR3 and a variable domain VL comprising the light chain CDRs of SEQ ID NO: 15, 16 and 17 as respectively light chain CDR1, CDR2 and CDR3. Preferably the antibody comprises the variable domains of SEQ ID NO:10 (VH) and SEQ ID NO:11 (VL). Preferably the antibody comprises a variable domain VH comprising the heavy chain CDRs of SEQ ID NO: 23, 24 and 25 as respectively heavy chain CDR1, CDR2 and CDR3 and a variable domain VL comprising the light chain CDRs of SEQ ID NO: 26, 27 and 28 as respectively light chain CDR1, CDR2 and CDR3. Preferably the antibody comprises the variable domains of SEQ ID NO:21 (VH) and SEQ ID NO:22 (VL).The anti-CD3 antibodies shown in SEQ ID NO:10 and 11 and 21 and 22 are derived from SP34 and have similar sequences and the same properties in regard to epitope binding as antibody SP34.

"Specifically binding to CD3 or ROR1" refer to an antibody that is capable of binding CD3 or ROR1 (the targets) with sufficient affinity such that the antibody is useful as a therapeutic agent in targeting CD3 or ROR1. In some embodiments, the extent of binding of an anti-CD3 or ROR1 antibody to an unrelated, non-CD3 or non-ROR1 protein is about 10-fold preferably >100-fold less than the binding of the antibody to CD3 or ROR1 as measured, e.g., by surface plasmon resonance (SPR) e.g. Biacore®, enzyme-linked immunosorbent (ELISA) or flow cytometry (FACS). Preferably the antibody that binds to CD3 or ROR1 has a dissociation constant (Kd) of 10⁻⁸ M or less, preferably from 10⁻⁸ M to 10⁻¹³ M, preferably from 10⁻⁹ M to 10⁻¹³ M. Preferably the bispecific antibody according to the invention binds to an epitope of ROR1 that is conserved among ROR1 from different species and/or an epitope of CD3 that is conserved among CD3 from different species, preferably among human and cynomolgus. "Bispecific antibody specifically binding to CD3 and ROR1" or "antibody according to the invention" refers to a respective definition for binding to both targets. An antibody specifically binding to ROR1 (or CD3 or ROR1 and CD3) does not bind to other human antigens. Therefore in an ELISA, OD values for such unrelated targets will be equal or lower to that of the limit of detection of the specific assay, preferably equal or lower as 1.5 pM, or equal or lower to OD values of control samples without plate-bound-ROR1 or with untransfected HEK293 cells.

Anti-ROR1 antibodies are analyzed by ELISA for binding to human ROR1 using plate-bound ROR1. For this assay, an amount of plate-bound ROR1 preferably or 1.5 nM and concentration(s) preferably ranging from 1 pM to 200 nM of anti-ROR1 antibody are used. A ROR1 antibody for which its ROR1 binding is at least 20% higher than the OD values of the control samples without plate-bound ROR1 or with untransfected HEK293 cells according to the invention is an anti-ROR1 antibody "binding to human ROR1 in an ELISA assay". An exemplary antibody is an anti-ROR1 antibody, characterized in being of human IgG1 kappa (κ) type comprising as light chain variable domain (VL) the sequence of SEQ ID NO:2 and as variable heavy chain domain (VH) the sequence of SEQ ID NO:6, or the respective CDRs shown in SEQ ID NO: 3, 4, 5, 7, 8, 9 (further named also as "said anti-ROR1 bivalent antibody").

The term "antibody according to the invention which does not internalize" as used herein means a bispecific antibody according to the invention with MFI reduction properties characterized in that in a cell based assay at 37°C during 2 hrs., using ROR1-positive B-CLL cells, and used at an antibody concentration of 1 nM, whereby not internalize means, that the mean fluorescence intensity (MFI), as detected by flow cytometry, upon binding to ROR1-positive cells measured at time 0 is not reduced more than 50%, preferably not more than 30% by internalization when re-measured after a 2hr-incubation at 37°C. The bispecific antibody according to the invention does not internalize in ROR1-positive B-CLL cells, therefore the binding of the said anti-ROR1 antibody to ROR1-positive B-CLL cells is not reduced more than 50%, preferably not more than 30%, when the said antibody is incubated at 37°C for 2 h in such cell based assay as described herein.

It is also preferred, that a bispecific antibody according to the invention shows in a cell based assay at 37°C during 2 hrs, using ROR1-positive B-CLL cells, and at an antibody concentration of 1 nM, a decrease in the mean fluorescence intensity by internalization from time 0 to 2 hrs at 37°C (ΔMFI), as measured by flow cytometry is between 120% to 0%, preferably from 100% to 0%, of the ΔMFI of an anti-ROR1 bivalent antibody of human IgG1 kappa (κ) type comprising as light chain variable domain (VL) the sequence of SEQ ID NO:2 and as variable heavy chain domain (VH) the sequence of SEQ ID NO:6, in the same concentration and experimental conditions.

The cell line ACC-67 is described in Saltman, D. et al., Leukemia research 14 (1990) 381-387 and available from Leibniz Institute DSMZ-German Collection of Microorganisms and Cell Cultures, Braunschweig, Germany, in the open collection.

For a therapy using a T cell bispecific antibody comprising an anti-ROR1 antibody, it is preferred that the antibody does not internalize as defined above for facilitating a stable immune synapse between the tumor cell and the T cell and effective T cell-mediated redirected cytotoxicity.

The term "reduction of mean fluorescence intensity" (ΔMFI) reflecting the internalization of the said anti-ROR1 antibody to ROR1-positive cells" or "MFI reduction" as used herein refers to the percentage of MFI reduction as calculated for each ROR1 antibodies relative to the unspecific human IgG control (MFI_{background}) and ROR1 antibodies maintained on ice (MFIₘₐₓ) by using the formula ΔMFI= 100 - 100 X [(MFIₑₓₚₑᵣᵢₘₑₙₜₐₗ - MFI_{background}) / (MFIₘₐₓ - MFI_{background})]. MFIₑₓₚₑᵣᵢₘₑₙₜₐₗ is the MFI measured with said ROR1 antibody after 2h incubation at 37°C. An MFI reduction which is at least 75% blocked and reversed by 10 µM endocytosis inhibitor phenylarsine oxide is for example caused by antibody internalization while an MFI reduction which is not blocked by phenylarsine oxide is caused by antibody dissociation. Internalizing anti-ROR1 antibodies are known in the state of the art (Baskar et al., Clin. Cancer Res., 14(2): 396-404 (2008)).

Preferably the bispecific antibody according to the invention is characterized in that an increase in MFI value at time 2hrs in the presence of 3µM phenylarsine oxide (PAO) as compared to MFI value at time 2hrs in the absence of PAO is not more than 30% , preferably not more than 20%, preferably not more that 10%, even not more than detection level of the MFI value at time 0.

The term "target" as used herein means either ROR1 or CD3. The term "first target and second target" means either CD3 as first target and ROR1 as second target or means ROR1 as first target and CD3 as second target.

The term "antibody" as used herein refers to a monoclonal antibody or a fragment thereof. An antibody consists of two pairs of a "light chain" (LC) and a "heavy chain" (HC) (such light chain (LC) /heavy chain pairs are abbreviated herein as LC/HC). The light chains and heavy chains of such antibodies are polypeptides consisting of several domains. Each heavy chain comprises a heavy chain variable region (abbreviated herein as HCVR or VH) and a heavy chain constant region. Each light chain comprises a light chain variable domain VL and a light chain constant domain CL. The variable domains VH and VL can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The "constant domains" of the heavy chain and of the light chain are not involved directly in binding of an antibody to a target, but exhibit various effector functions.

The term "devoid of the Fc domain" as used herein means that the bispecific antibodies according to the disclosure do not comprise a CH2, CH3 or CH4 domain; i.e. the constant heavy chain consists solely of one or more CH1 domains.

The term "antibody" includes e.g. mouse antibodies, human antibodies, chimeric antibodies, humanized antibodies and genetically engineered antibodies (variant or mutant antibodies) as long as their characteristic properties are retained. Especially preferred are human or humanized antibodies, especially as recombinant human or humanized antibodies.

The term "comprising" in regard to the bispecific antibody as used herein means that the bispecific antibody comprises as CD3 and ROR1 binders only those binders mentioned. Therefore a bispecific antibody according the disclosure comprising a monovalent anti-ROR1 antibody specifically binding to ROR1, and a monovalent antibody specifically binding to CD3 has in regard to CD3 and ROR1 binding only one binding valence for CD3 and only one valence for ROR1 and is therefore bivalent. A bispecific antibody according the invention comprising a bivalent anti-ROR1 antibody specifically binding to ROR1, and a monovalent antibody specifically binding to CD3 has in regard to ROR1 binding two binding valences and in regard to CD3 binding one valence and is therefore trivalent. Preferably the monovalent antibody specifically binding to CD3 is covalently linked at its C-terminus to the N-terminus of one variable chain of the antibody specifically binding to ROR1.

As used herein, "Fab fragment" refers to an antibody fragment comprising a light chain fragment comprising a VL domain and a constant domain of a light chain (CL), and a VH domain and the first constant domain (CH1) of a heavy chain. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as crossFab (VLVH). On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as crossFab (CLCH1). The term "Fab fragment" also includes parts or all of the hinge region, like Fab' fragment. As used herein, "F(ab)₂ fragment" refers to a bivalent monospecific antibody fragment without an Fc part. Preferably a F(ab)₂ fragment is linked at the C-terminus by disulphide bond(s) in the hinge region and usually such a "F(ab)₂ fragment" is a F(ab')₂ fragment.

The term "ROR1 Fab" as used within the invention denotes a Fab fragment of the antibody specifically binding to ROR1. Due to the exchange of either the variable regions or the constant regions in the anti-ROR1 antibody Fab fragment (ROR1 Fab), such ROR1 Fab is referred to as " RORlcross Fab" or "crossover ROR1 Fab fragment" According to the invention the ROR1 Fab is not a ROR1crossFab. By "connected" is meant that the Fab fragments are preferably linked by peptide bonds, either directly or via one or more peptide linker. The term "CD3 Fab" as used within the invention denotes a Fab fragment of the antibody specifically binding to CD3. The CD3 Fab is linked at its N-terminus the C-terminus of the ROR1 Fab. Due to the exchange of either the variable regions or the constant regions in the CD3 Fab, such CD3 Fab is referred to as "CD3 crossFab" or "crossover CD3 Fab fragment". According to the invention the CD3 Fab is preferably a crossFab.

The term "peptide linker" as used within the invention denotes a peptide with amino acid sequences, which is preferably of synthetic origin. These peptide linkers according to invention are used to connect one of the Fab fragments to the C-or N-terminus of the other Fab fragment to form a multispecific antibody according to the invention. Preferably said peptide linkers are peptides with an amino acid sequence with a length of at least 5 amino acids, preferably with a length of 5 to 100, more preferably of 10 to 50 amino acids. In one embodiment said peptide linker is (GxS)n or (GxS)nGm with G = glycine, S = serine, and (x = 3, n= 3, 4, 5 or 6, and m= 0, 1, 2 or 3) or (x = 4,n= 2, 3, 4 or 5 and m= 0, 1, 2 or 3), preferably x = 4 and n= 2 or 3, more preferably with x = 4, n= 2. Additionally, linkers may comprise (a portion of) an immunoglobulin hinge region. In one embodiment said peptide linker is (G₄S)₂ (SEQ ID: NO 19).

There are five types of mammalian antibody heavy chains denoted by the Greek letters: α, δ, ε, γ, and µ (Janeway CA, Jr et al (2001). Immunobiology. 5th ed., Garland Publishing). The type of heavy chain present defines the class of antibody; these chains are found in IgA, IgD, IgE, IgG, and IgM antibodies, respectively (Rhoades RA, Pflanzer RG (2002). Human Physiology, 4th ed., Thomson Learning). Distinct heavy chains differ in size and composition; α and γ contain approximately 450 amino acids, while µ and ε have approximately 550 amino acids. Each heavy chain has two regions, the constant region and the variable region. The constant region is identical in all antibodies of the same isotype, but differs in antibodies of different isotype. Heavy chains γ, α and δ have a constant region composed of three constant domains CH1, CH2, and CH3 (in a line), and a hinge region for added flexibility (Woof J, Burton D Nat Rev Immunol 4 (2004) 89-99); heavy chains µ and ε have a constant region composed of four constant domains CH1, CH2, CH3, and CH4 (Janeway CA, Jr et al (2001). Immunobiology. 5th ed., Garland Publishing). The variable region of the heavy chain differs in antibodies produced by different B cells, but is the same for all antibodies produced by a single B cell or B cell clone. The variable region of each heavy chain is approximately 110 amino acids long and is composed of a single antibody domain. In mammals there are only two types of light chain, which are called lambda (λ) and kappa (κ). A light chain has two successive domains: one constant domain CL and one variable domain VL. The approximate length of a light chain is 211 to 217 amino acids.

A bispecific antibody according to the invention, which comprises a Fc part, can be of any class (e.g. IgA, IgD, IgE, IgG, and IgM, preferably IgG or IgE), or subclass (e.g., IgG1, IgG2, IgG3, IgG4, IgA1 and IgA2, preferably IgG1), whereby both antibodies, from which the bivalent bispecific antibody according to the invention is derived, have an Fc part of the same subclass(e.g. IgG1, IgG4 and the like, preferably IgG1), preferably of the same allotype (e.g. Caucasian).

A "Fc part of an antibody" is a term well known to the skilled artisan and defined on the basis of papain cleavage of antibodies. The antibodies according to the invention, which comprise an Fc part, contain as Fc part, preferably a Fc part derived from human origin and preferably all other parts of the human constant regions. The Fc part of an antibody is directly involved in complement activation, C1q binding, C3 activation and Fc receptor binding. While the influence of an antibody on the complement system is dependent on certain conditions, binding to C1q is caused by defined binding sites in the Fc part. Such binding sites are known in the state of the art and described e.g. by Lukas, TJ., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat, see below). Antibodies of subclass IgG1, IgG2 and IgG3 usually show complement activation, C1q binding and C3 activation, whereas IgG4 do not activate the complement system, do not bind C1q and do not activate C3. Preferably the Fc part is a human Fc part.

The constant heavy chain of an antibody according to the invention is preferably of human IgGltype and the constant light chain is preferably of human lambda (λ) or kappa (κ) type, preferably of human kappa (κ) type.

The terms "monoclonal antibody" or "monoclonal antibody composition" as used herein refer to a preparation of antibody molecules of a single amino acid composition.

The term "chimeric antibody" refers to an antibody comprising a variable region, i.e., binding region, from one source or species and at least a portion of a constant region derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See, e.g., Morrison, S.L., et al., Proc. Natl. Acad. Sci. USA 81 (1984) 6851-6855; US Patent Nos. 5,202,238 and 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the targets noted above for chimeric antibodies. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon target challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. Mol. Biol. 227 (1992) 381-388; Marks, J.D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95).

The term "recombinant human antibody", as used herein, is intended to include all human antibodies that are prepared, expressed, created or isolated by recombinant means, such as antibodies isolated from a host cell such as a NSO or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The "variable domain" (variable domain of a light chain (VL), variable region of a heavy chain (VH)) as used herein denotes each of the pair of light and heavy chains which is involved directly in binding the antibody to the target. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the target binding site. The antibody heavy and light chain CDR3 regions play a particularly important role in the binding specificity/affinity of the antibodies according to the invention and therefore provide a further object of the invention.

The terms "hypervariable region" or "target-binding portion of an antibody" when used herein refer to the amino acid residues of an antibody which are responsible for target-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. CDRs on each chain are separated by such framework amino acids. Especially, CDR3 of the heavy chain is the region which contributes most to target binding. CDR and FR regions are determined according to the standard definition of Kabat et al., Sequences of Proteins of Immunological Interest, 5th ed., Public Health Service, National Institutes of Health, Bethesda, MD (1991).

The term "target" or "target molecule" as used herein are used interchangeable and refer to human ROR1 and human CD3ε.

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of a target that is bound by an antibody.

In general there are two vectors encoding the light chain and heavy chain of said antibody specifically binding to the first target, and further two vectors encoding the light chain and heavy chain of said antibody specifically binding to the second target. One of the two vectors is encoding the respective light chain and the other of the two vectors is encoding the respective heavy chain. However in an alternative method for the preparation of a bispecific antibody according to the invention, only one first vector encoding the light chain and heavy chain of the antibody specifically binding to the first target and only one second vector encoding the light chain and heavy chain of the antibody specifically binding to the second target can be used for transforming the host cell.

The term "nucleic acid or nucleic acid molecule", as used herein, is intended to include DNA molecules and RNA molecules. A nucleic acid molecule may be single-stranded or double-stranded, but preferably is double-stranded DNA.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen SN, et al, PNAS 1972, 69 (8): 2110-2114.

Recombinant production of antibodies using transformation is well-known in the state of the art and described, for example, in the review articles of Makrides, S. C, Protein Expr. Purif. 17 (1999) 183-202; Geisse, S., et al., Protein Expr. Purif. 8 (1996) 271-282; Kaufman, RJ., Mol. Biotechnol. 16 (2000) 151-161; Werner, R.G., et al., Arzneimittelforschung 48 (1998) 870-880 as well as in US6331415 and US4816567.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product.

The bispecific antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NSO cells, SP2/0 cells, HEK293 cells, COS cells, yeast, or E.coli cells, and the antibody is recovered from the cells (supernatant or cells after lysis). The bispecific antibodies may be present in whole cells, in a cell lysate, or in a partially purified or substantially pure form. Purification is performed in order to eliminate other cellular components or other contaminants, e.g. other cellular nucleic acids or proteins, by standard techniques, including alkaline/SDS treatment, column chromatography and others well known in the art. See Ausubel, F., et al., ed., Current Protocols in Molecular Biology, Greene Publishing and Wiley Interscience, New York (1987).

Expression in NS0 cells is described by, e.g., Barnes, L.M., et al., Cytotechnology 32 (2000) 109-123; and Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK293) is described by Schlaeger, E.- J., and Christensen, K., in Cytotechnology 30 (1999) 71-83 and by Schlaeger, E.-J., in J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the

DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The bispecific antibodies are suitably separated from the culture medium by conventional immunoglobulin purification procedures such as, for example, protein A-Sepharose, hydroxylapatite chromatography, gel electrophoresis, dialysis, or affinity chromatography. DNA or RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells such as HEK293 cells, CHO cells, or myeloma cells that do not otherwise produce immunoglobulin protein, to obtain the synthesis of recombinant monoclonal antibodies in the host cells.

Amino acid sequence variants (or mutants) of the bispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and target binding, but may improve the yield of the recombinant production, protein stability or facilitate the purification.

T cell bispecific (TCB) binders have very high concentration/tumor-cell-receptor-occupancy dependent potency in cell killing (e.g. EC₅₀ in in vitro cell killing assays in the sub- or low picomolar range; Dreier et al. Int J Cancer 2002), T-cell bispecific binder (TCB) are given at much lower doses than conventional monospecific antibodies. For example, blinatumomab (CD19xCD3) is given at a continuous intravenous dose of 5 to 15 µg/m²/day (i.e. only 0.35 to 0.105 mg/m²/week) for treatment of acute lymphocytic leukemia or 60 µg/m²/day for treatment of Non Hodgkin Lymphoma, and the serum concentrations at these doses are in the range of 0.5 to 4 ng/ml (Klinger et al., Blood 2012; Topp et al., J Clin Oncol 2011; Goebeler et al. Ann Oncol 2011). Because low doses of TCB can exert high efficacy in patients, it is envisaged that for an antibody according to the invention subcutaneous administration is possible and preferred in the clinical settings (preferably in the dose range of 0.25 to 2.5 mg/m²/week or twice a week). Even at these low concentrations/doses/receptor occupancies, TCB can cause considerable adverse events (Klinger et al., Blood 2012).

In principle it is possible to produce bispecific antibodies against CD3 and ROR1 in all formats known in the state of the art. A wide variety of recombinant bispecific antibody formats have been developed in the recent past, e.g. by fusion of, e.g. an IgG antibody format and single chain domains (see e.g. Kontermann RE, mAbs 4:2, (2012) 1-16). Bispecific antibodies wherein the variable domains VL and VH or the constant domains CL and CH1 are replaced by each other are described in WO2009080251 and WO2009080252. Antibody formats and formats of bispecific and multispecific antibodies are also pepbodies (WO200244215), Novel Antigen Receptor ("NAR") (WO2003014161), diabody-diabody dimers "TandAbs" (WO2003048209), polyalkylene oxide-modified scFv (US7150872), humanized rabbit antibodies (WO2005016950), synthetic immunoglobulin domains (WO2006072620), covalent diabodies (WO2006113665), flexibodies (WO2003025018), domain antibodies, dAb (WO2004058822), vaccibody (WO2004076489), antibodies with new world primate framework (WO2007019620), antibody-drug conjugate with cleavable linkers (WO2009117531), IgG4 antibodies with hinge region removed (WO2010063785), bispecific antibodies with IgG4 like CH3 domains (WO2008119353), camelid Antibodies (US6838254), nanobodies (US7655759), CAT diabodies (US5837242), bispecific scFv2 directed against target antigen and CD3 (US7235641),), sIgA plAntibodies (US6303341), minibodies (US5837821), IgNAR (US2009148438), antibodies with modified hinge and Fc regions (US2008227958, US20080181890), trifunctional antibodies (US5273743), triomabs (US6551592), troybodies (US6294654).

However an antibody according to the disclosure that is devoid of an Fc part has a safety advantage because of the lack of possibility for inducing Fc-mediated infusion reactions. Because of the strong potency of a bispecific antibody according to the invention in killing of target cells in vitro (e.g. in the subnanomolar and even picomolar range) and the fact that ROR1 is also expressed on normal cells (e.g. normal human adipocytes), it is preferred that the bispecific antibody can be eliminated rapidly from the circulation after being administrated. A T cell bispecific devoid of an Fc part according to the disclosure has therefore a safety advantage and is eliminated rapidly from the circulation in case of toxicity.

An antibody according to the disclosure which has as two or three Fab fragments a molecular weight of about 100 kDa and 143 kDa respectively, i.e. bigger than the kidney filtration size limit but without an Fc, gives the TCB a half-life of 6 to 72 hours enabling once or twice a week sc administration but being shorter in half life than a Fc containing antibody (10 to 14 days), has safety advantages over a TCB with Fc and does not show Fc-related infusion reactions.

A bispecific trivalent antibody according to the invention has advantages on the potency, predictability for efficacy and safety.

An antibody according to the invention with bivalency to ROR1 and monovalency to CD3 favors binding to the tumor target ROR1 on malignant cells over CD3ε on T cells in circulation and avoids CD3 sink, thus increasing drug exposure in the tumor.

The following examples, sequence listing and figures are provided to aid the understanding of the present invention, the true scope of which is set forth in the appended claims. It is understood that modifications can be made in the procedures set forth without departing from the spirit of the invention.

**Sequence listing**

| **SEQ NO:** | **Name** |
|---|---|
| 1 | ROR1 extracellular domain |
| 2 | Mab ROR1 VL |
| 3 | CDR1L |
| 4 | CDR2L |
| 5 | CDR3L |
| 6 | Mab ROR1 VH |
| 7 | CDR1H |
| 8 | CDR2H |
| 9 | CDR3H |
| 10 | Mab CD3 VH (H2C) |
| 11 | Mab CD3 VL (H2C) |
| 12 | CDR1H (H2C) |
| 13 | CDR2H (H2C) |
| 14 | CDR3H (H2C) |
| 15 | CDR1L (H2C) |
| 16 | CDR2L (H2C) |
| 17 | CDR3L (H2C) |
| 18 | Extracellular fragment of ROR1 |
| 19 | Linker |
| 20 | Intracellular fragment of ROR1 |
| 21 | Mab CD3 VH (CH2527) |
| 22 | Mab CD3 VL (CH2527) |
| 23 | CDR1H (CH2527) |
| 24 | CDR2H (CH2527) |
| 25 | CDR3H (CH2527) |
| 26 | CDRL1 (CH2527) |
| 27 | CDRL2 (CH2527) |
| 28 | CDRL3 (CH2527) |
| 29 | ROR1 hum IgG1 HC LALA PG |
| 30 | ROR1 hum IgG1 LC |
| 31 | ROR1 x CD3 VH_CL HC knob LALA PG |
| 32 | ROR1 HC hole LALA PG |
| 33 | CD3 VL_CH1 |
| 34 | ROR1 x CD3 VH_CL |
| 35 | (ROR1)2 x CD3 VH_CL |
| 36 | Fc hole LALA PG |

### Materials & general methods

General information regarding the nucleotide sequences of human immunoglobulins light and heavy chains is given in: Kabat, E. A., et al., Sequences of Proteins of Immunological Interest, 5th ed., Public Health Service, National Institutes of Health, Bethesda, MD (1991). Amino acids of antibody chains are numbered and referred to according to EU numbering (Edelman, G.M., et al., Proc. Natl. Acad. Sci. USA 63 (1969) 78-85; Kabat, E.A., et al., Sequences of Proteins of Immunological Interest, 5th ed., Public Health Service, National Institutes of Health, Bethesda, MD, (1991)).

### Recombinant DNA techniques

Standard methods are used to manipulate DNA as described in Sambrook, J. et al., Molecular cloning: A laboratory manual; Cold Spring Harbor Laboratory Press, Cold Spring Harbor, New York, 1989. The molecular biological reagents are used according to the manufacturer's instructions. General information regarding the nucleotide sequences of human immunoglobulins light and heavy chains is given in: Kabat, E.A. et al., (1991) Sequences of Proteins of Immunological Interest, 5th ed., NIH Publication No. 91-3242.

### Gene synthesis

a) Desired gene segments are prepared from oligonucleotides made by chemical synthesis. The 600 - 1800 bp long gene segments, which are flanked by singular restriction endonuclease cleavage sites, are assembled by annealing and ligation of oligonucleotides including PCR amplification and subsequently cloned via the indicated restriction sites e.g. Kpnl/ Sad or Ascl/Pacl into a pPCRScript (Stratagene) based pGA4 cloning vector. The DNA sequences of the subcloned gene fragments are confirmed by DNA sequencing. Gene synthesis fragments are ordered according to given specifications at Geneart (Regensburg, Germany).
b) Desired gene segments where required were either generated by PCR using appropriate templates or were synthesized by Geneart AG (Regensburg, Germany) from synthetic oligonucleotides and PCR products by automated gene synthesis. The gene segments flanked by singular restriction endonuclease cleavage sites were cloned into standard expression vectors or into sequencing vectors for further analysis. The plasmid DNA was purified from transformed bacteria using commercially available plasmid purification kits. Plasmid concentration was determined by UV spectroscopy. The DNA sequence of the subcloned gene fragments was confirmed by DNA sequencing. Gene segments were designed with suitable restriction sites to allow sub-cloning into the respective expression vectors. If required, protein coding genes were designed with a 5'-end DNA sequence coding for a leader peptide which targets proteins for secretion in eukaryotic cells.

### DNA sequence determination

DNA sequences are determined by double strand sequencing.

### DNA and protein sequence analysis and sequence data management

The GCG's (Genetics Computer Group, Madison, Wisconsin) software package version 10.2 and Infomax's Vector NTl Advance suite version 8.0 is used for sequence creation, mapping, analysis, annotation and illustration.

### Expression vectors

a) For the expression of the described antibodies variants of expression plasmids for transient expression (e.g. in HEK293 EBNA or HEK293-F) cells based either on a cDNA organization with a CMV-Intron A promoter or on a genomic organization with a CMV promoter are applied. Beside the antibody expression cassette the vectors contain an origin of replication which allows replication of this plasmid in E. coli, and- a β-lactamase gene which confers ampicillin resistance in E. coli. The transcription unit of the antibody gene is composed of the following elements:
   - unique restriction site(s) at the 5' end - the immediate early enhancer and promoter from the human cytomegalovirus,
   - followed by the Intron A sequence in the case of the cDNA organization,
   - a 5'-untranslated region of a human antibody gene,
   - a immunoglobulin heavy chain signal sequence,
   - the human antibody chain (wildtype or with domain exchange) either as cDNA or as genomic organization with an the immunoglobulin exon-intron organization
   - a 3' untranslated region with a polyadenylation signal sequence, and
   - unique restriction site(s) at the 3' end.

   The fusion genes comprising the described antibody chains as described below are generated by PCR and/or gene synthesis and assembled with known recombinant methods and techniques by connection of the according nucleic acid segments e.g. using unique restriction sites in the respective vectors. The subcloned nucleic acid sequences are verified by DNA sequencing. For transient transfections larger quantities of the plasmids are prepared by plasmid preparation from transformed E. coli cultures (Nucleobond AX, Macherey-Nagel).
b) For the generation of anti-ROR1 antibody expression vectors, the variable regions of heavy and light chain DNA sequences were subcloned in frame with either the human IgG1 constant heavy chain or the hum IgG1 constant light chain pre-inserted into the respective generic recipient expression vector optimized for expression in mammalian cell lines. The antibody expression is driven by a chimeric MPSV promoter comprising a CMV enhancer and a MPSV promoter followed by a 5' UTR, an intron and a Ig kappa MAR element. The transcription is terminated by a synthetic polyA signal sequence at the 3' end of the CDS. All vectors carry a 5'-end DNA sequence coding for a leader peptide which targets proteins for secretion in eukaryotic cells. In addition each vector contains an EBV OriP sequence for episomal plasmid replication in EBV EBNA expressing cells.
c) For the generation of ROR1xCD3 bispecific antibody vectors, the IgG1 derived bispecific molecules consist at least of two antigen binding moieties capable of binding specificly to two distinct antigenic determinants CD3 and ROR1. The antigen binding moieties are Fab fragments composed of a heavy and a light chain, each comprising a variable and a constant region. At least one of the Fab fragments is a "Crossfab" fragment, wherein the constant domains of the Fab heavy and light chain are exchanged. The exchange of heavy and light chain constant domains within the Fab fragment assures that Fab fragments of different specificity do not have identical domain arrangements and consequently do not interchange light chains. The bispecific molecule design can be monovalent for both antigenic determinants (1+1) or monovalent for CD3 and bivalent for ROR1 where one Fab fragment is fused to the N-terminus of the inner CrossFab (2+1). It can be either Fc containing or non-Fc containing in order to modulate half-life of the molecule. A schematic representation of the constructs is given in Figure 1; Sequences of the constructs are shown in SEQ ID NOs 1 to 36. The molecules were produced by co-transfecting HEK293 EBNA cells growing in suspension with the mammalian expression vectors using polyethylenimine (PEI). For preparation of 1+1 CrossFab-IgG constructs, cells were transfected with the corresponding expression vectors in a 1:1:1:1 ratio ("vector Fc(knob)" : "vector light chain" : "vector light chain CrossFab" : "vector heavy chain-CrossFab"). For preparation of 2+1 CrossFab-IgG constructs, cells were transfected with the corresponding expression vectors in a 1:2:1:1 ratio ("vector Fc(knob)" : "vector light chain" : "vector light chain CrossFab" : "vector heavy chain-CrossFab").

### Cell culture techniques

Standard cell culture techniques are used as described in Current Protocols in Cell Biology (2000), Bonifacino, J. S., Dasso, M., Harford, J.B., Lippincott-Schwartz, J. and Yamada, K.M. (eds.), John Wiley & Sons, Inc.

### Transient expression in HEK293 cells

Bispecific antibodies are expressed by transient co-transfection of the respective expression plasmids in adherently growing HEK293-EBNA or in HEK293-F cells growing in suspension as described below.

### a) Transient transfections in HEK293-EBNA system

a) Bispecific antibodies are expressed by transient co-transfection of the respective expression plasmids (e.g. encoding the heavy and modified heavy chain, as well as the corresponding light and modified light chain) in adherently growing HEK293-EBNA cells (human embryonic kidney cell line 293 expressing Epstein-Barr-Virus nuclear target; American type culture collection deposit number ATCC # CRL- 10852, Lot. 959 218) cultivated in DMEM (Dulbecco's modified Eagle's medium, Gibco) supplemented with 10% Ultra Low IgG FCS (fetal calf serum, Gibco), 2 mM L-Glutamine (Gibco), and 250 µg/ml Geneticin (Gibco). For transfection FuGENE™ 6 Transfection Reagent (Roche Molecular Biochemicals) is used in a ratio of FuGENE™ reagent (µl) to DNA (µg) of 4:1 (ranging from 3:1 to 6:1).
   Proteins are expressed from the respective plasmids using a molar ratio of (modified and wildtype) light chain and heavy chain encoding plasmids of 1:1 (equimolar) ranging from 1:2 to 2:1, respectively. Cells are feeded at day 3 with L-Glutamine ad 4 mM, Glucose [Sigma] and NAA [Gibco]. Bispecific antibody containing cell culture supernatants are harvested from day 5 to 11 after transfection by centrifugation and stored at -200C. General information regarding the recombinant expression of human immunoglobulins in e.g. HEK293 cells is given in: Meissner, P. et al., Biotechnol. Bioeng. 75 (2001) 197-203.
b) The recombinant anti-ROR1 human antibody and bispecific antibodies were produced in suspension by co-transfecting HEK293-EBNA cells with the mammalian expression vectors using polyethylenimine (PEI). The cells were transfected with two or four vectors, depending in the format. For the human IgG1 one plasmid encoded the heavy chain and the other plasmid the light chain. For the bispecific antibodies four plasmids were co-transfected. Two of them encoded the two different heavy chains and the other two encoded the two different light chains. One day prior to transfection the HEK293-EBNA cells were seeded at 1.5 Mio viable cells/mL in F17 Medium, supplemented with 6 mM of L-Glutamine.

### b) Transient transfections in HEK293-F system

Bispecific antibodies are generated by transient transfection of the respective plasmids (e.g. encoding the heavy and modified heavy chain, as well as the corresponding light and modified light chain) using the HEK293-F system (Invitrogen) according to the manufacturer's instruction. Briefly, HEK293-F cells (Invitrogen) growing in suspension either in a shake flask or in a stirred fermenter in serumfree FreeStyle 293 expression medium (Invitrogen) are transfected with a mix of the four expression plasmids and 293fectin or fectin (Invitrogen). For 2 L shake flask (Corning) HEK293-F cells are seeded at a density of 1.0 x 10⁶ cells/mL in 600 mL and incubated at 120 rpm, 8% CO2. The day after the cells are transfected at a cell density of ca. 1.5 x 10⁶ cells/mL with ca. 42 mL mix of A) 20 mL Opti-MEM (Invitrogen) with 600 µg total plasmid DNA (1 µg/mL) encoding the heavy or modified heavy chain, respectively and the corresponding light chain in an equimolar ratio and B) 20 ml Opti-MEM + 1.2 mL 293 fectin or fectin (2 µl/mL). According to the glucose consumption glucose solution is added during the course of the fermentation. The supernatant containing the secreted antibody is harvested after 5-10 days and antibodies are either directly purified from the supernatant or the supernatant is frozen and stored.

### Protein determination

The protein concentration of purified antibodies and derivatives is determined by determining the optical density (OD) at 280 nm, using the molar extinction coefficient calculated on the basis of the amino acid sequence according to Pace et al., Protein Science, 1995, 4, 2411-1423.

### Antibody concentration determination in supernatants

a) The concentration of antibodies and derivatives in cell culture supernatants is estimated by immunoprecipitation with Protein A Agarose-beads (Roche). 60 µL Protein A Agarose beads are washed three times in TBS-NP40 (50 mM Tris, pH 7.5, 150 mM NaCl, 1% Nonidet-P40). Subsequently, 1 -15 mL cell culture supernatant is applied to the Protein A Agarose beads pre-equilibrated in TBS-NP40. After incubation for at 1 h at room temperature the beads are washed on an Ultrafree-MC-filter column (Amicon] once with 0.5 mL TBS-NP40, twice with 0.5 mL 2x phosphate buffered saline (2xPBS, Roche) and briefly four times with 0.5 mL 100 mM Na-citrate pH 5,0. Bound antibody is eluted by addition of 35 µl NuPAGE® LDS Sample Buffer (Invitrogen). Half of the sample is combined with NuPAGE® Sample Reducing Agent or left unreduced, respectively, and heated for 10 min at 70°C. Consequently, 5-30 µl are applied to an 4-12% NuPAGE® Bis-Tris SDS-PAGE (Invitrogen) (with MOPS buffer for non-reduced SDS-PAGE and MES buffer with NuPAGE® Antioxidant running buffer additive (Invitrogen) for reduced SDS-PAGE) and stained with Coomassie Blue.
   The concentration of antibodies and derivatives in cell culture supernatants is quantitatively measured by affinity HPLC chromatography. Briefly, cell culture supernatants containing antibodies and derivatives that bind to Protein A are applied to an Applied Biosystems Poros A/20 column in 200 mM KH₂PO₄, 100 mM sodium citrate, pH 7.4 and eluted from the matrix with 200 mM NaCl, 100 mM citric acid, pH 2,5 on an Agilent HPLC 1100 system. The eluted protein is quantified by UV absorbance and integration of peak areas. A purified standard IgG1 antibody served as a standard.
   Alternatively, the concentration of antibodies and derivatives in cell culture supernatants is measured by Sandwich-IgG-ELISA. Briefly, StreptaWell High Bind Strepatavidin A-96 well microtiter plates (Roche) are coated with 100 µL/well biotinylated anti-human IgG capture molecule F(ab')2<h-Fcγ> BI (Dianova) at 0.1 µg/mL for 1 h at room temperature or alternatively over night at 4°C and subsequently washed three times with 200 µL/well PBS, 0.05% Tween® (PBST, Sigma). 100 µL/well of a dilution series in PBS (Sigma) of the respective antibody containing cell culture supernatants is added to the wells and incubated for 1-2 h on a micro titerplate shaker at room temperature. The wells are washed three times with 200 µL/well PBST and bound antibody is detected with 100 µl F(ab')2<hFcγ>POD (Dianova) at 0.1 µg/mL as detection antibody for 1-2 h on a microtiterplate shaker at room temperature. Unbound detection antibody is washed away three times with 200 µL/well PBST and the bound detection antibody is detected by addition of 100 µL ABTS/well. Determination of absorbance is performed on a Tecan Fluor Spectrometer at a measurement wavelength of 405 nm (reference wavelength 492 nm).
b) For every mL of final Production volume 2.0 Mio viable cells were centrifuged (5 minutes at 210 x g). The supernatant was aspirated and the cells resuspended in 100 uL of CD CHO medium. The DNA for every mL of final Production volume was prepared by mixing 1 ug of DNA (light chain DNA : heavy Chain DNA = 1 : 1) in 100 uL of CD CHO medium. After addition of 0.27 uL of PEI solution (1 mg/mL) the mixture was vortexed for 15 seconds and left at RT for 10 minutes. After 10 minutes the resuspended cells and DNA/PEI mixture were put together. This was then transferred into an appropriate container which was placed in a shaking device (37°C, 5% CO2). After a 3 hour incubation time 800 uL of F17 Medium, supplemented with 6 mM L-Glutamine, 1.25 mM valproic acid and 12.5% Pepsoy (50 g/L), was added for every mL of final Production volume. After 24 hours 70 uL of Feed (SF40, Lonza) was added for every mL of final Production volume. After 7 days or when the cell viability was equal or lower than 70% the cells were separated from the supernatant by centrifugation and sterile filtration.

### Protein purification

a) Proteins are purified from filtered cell culture supernatants referring to standard protocols. In brief, antibodies are applied to a Protein A Sepharose column (GE healthcare) and washed with PBS. Elution of antibodies is achieved at pH 2.8 followed by immediate neutralization of the sample. Aggregated protein is separated from monomeric antibodies by size exclusion chromatography (Superdex 200, GE Healthcare) in PBS or in 20 mM Histidine, 150 mM NaCl pH 6.0. Monomeric antibody fractions are pooled, concentrated if required using e.g. a MILLIPORE Amicon Ultra (30 MWCO) centrifugal concentrator, frozen and stored at -20°C. Part of the samples are provided for subsequent protein analytics and analytical characterization e.g. by SDS-PAGE, size exclusion chromatography or mass spectrometry.
b) The antibodies were purified by a two one-step methods. The supernatant was loaded on a protein A column, (HiTrap Protein A FF (5 mL, GE Healthcare)), equilibrated with 6 CV 20 mM sodium phosphate, 20 mM sodium citrate, pH 7.5. After a washing step the antibody was eluted from the column by step elution with 20 mM sodium phosphate, 100 mM sodium chloride, 100 mM Glycine, pH 3.0. The appropriate fractions with eluted antibody were neutralized by 0.5 M Sodium Phosphate, pH 8.0 (1:10), pooled and concentrated by centrifugation. The concentrate was sterile filtered and injected onto a XK16/60 HiLoad Superdex 200 column (GE Healthcare). The formulation buffer was 20 mM Histidine, 140 mM NaCl, 0.01% Tween20, pH 6.0. The fractions containing the monomers were pooled, concentrated by centrifugation and sterile filtered into a sterile vial. Determination of the concentration was done by measurement of the absorbance at 280 nm, using the theoretical value of the absorbance of a 0.1% solution of the antibody. This value was based on the amino acid sequence.

### SDS-PAGE

The NuPAGE® Pre-Cast gel system (Invitrogen) is used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer is used.

### Analytical size exclusion chromatography

a) Size exclusion chromatography for the determination of the aggregation and oligomeric state of antibodies is performed by HPLC chromatography. Briefly, Protein A purified antibodies are applied to a Tosoh TSKgel G3000SW column in 300 mM NaCl, 50 mM KH₂PO₄/K₂HPO₄, pH 7.5 on an Agilent HPLC 1100 system or to a Superdex 200 column (GE Healthcare) in 2 x PBS on a Dionex HPLC-System. The eluted protein is quantified by UV absorbance and integration of peak areas. BioRad Gel Filtration Standard 151-1901 served as a standard.
b) Purity and monomer content of the final protein preparation was determined by CE-SDS (Caliper LabChip GXII system (Caliper Life Sciences) resp. HPLC (TSKgel G3000 SW XL analytical size exclusion column (Tosoh) in a 25 mM potassium phosphate, 125 mM Sodium chloride, 200 mM L-arginine monohydrochloride, 0.02 % (w/v) Sodium azide, pH 6.7 buffer.

### Mass spectrometry

The total deglycosylated mass of crossover antibodies is determined and confirmed via electrospray ionization mass spectrometry (ESI-MS). Briefly, 100 µg purified antibodies are deglycosylated with 50 mil N-Glycosidase F (PNGaseF, ProZyme) in 100 mM KH₂PO₄/K₂HPO₄, pH 7 at 37°C for 12-24 h at a protein concentration of up to 2 mg/ml and subsequently desalted via HPLC on a Sephadex G25 column (GE Healthcare). The mass of the respective heavy and light chains is determined by ESI-MS after deglycosylation and reduction. In brief, 50 µg antibody in 115 µl are incubated with 60 µl IM TCEP and 50 µl 8 M Guanidine-hydrochloride subsequently desalted. The total mass and the mass of the reduced heavy and light chains is determined via ESI-MS on a Q-Star Elite MS system equipped with a NanoMate® source.

### Examples

### Example 1 - Generation of anti-ROR1 antibodies

The protein sequences of the VH and VL regions for an ROR1 antibody of SEQ ID NOs: 2-9 are described in WO2012/075158.

Briefly, oliogonucleotides encoding the above sequences are joined together via PCR to synthesize cDNAs encoding the VH are VL sequences, respectively, of the anti-ROR1 antibody.

### Example 1A. Recombinant, soluble, human ROR1 extracellular domain

Recombinant, soluble, human ROR1 extracellular domain is produced as a fusion protein to the C-terminus of human IgG1 Fc ("ROR1-ECD"). Briefly, using splice-overlap-extension PCR, a cDNA encoding the fusion protein is synthesized. The cDNA encodes, from N- to C-terminus, a fusion protein consisting of a secretory leader sequence, hinge region, CH2 and CH3 domains of human IgG1, a flexible linker with the sequence Gly-Gly-Gly-Gly-Ser-Gly-Gly-Gly-Gly-Ser, (SEQ ID NO:19) and the extracellular domains of human ROR1 (amino acid residues 24 - 403). The cDNA is subcloned into a mammalian expression vector and the recombinant fusion protein is produced and purified using the same methods as for human IgG1 antibodies described below in Example 2. The template used for PRC-amplifying the human ROR1 extracellular domain-encoding cDNA portion is a human full-length cDNA clone EN1031_D08 Ror1 gene (Origene Technologies, Rockville, MD).

A biotinylated variant of the same human Fc-ROR1 ECD fusion ("ROR1-ECD-biot") is produced as described above using the same procedures with the following modifications. A DNA sequence encoding an Avi-His tag is added, via PCR amplification, in frame downstream at the 3' end of the first PCR product described above. This new, second PCR product is then subcloned into the mammalian expression vector and then co-transfected in mammalian cells together with a vector for expression of BirA enzyme for in vivo biotinylation of the Avi tag. During cell culture for production of ROR1-ECD-biot, cell culture medium is supplemented with biotin to allow for proper biotinylation. The remaining production and purification steps are performed as indicated above for ROR1-ECD.

### Example 1B. Recombinant cells expressing human ROR1 on their surface

Recombinant cells expressing human ROR1 on their surface ("HEK293-ROR1 cells") are generated as described in example 1A. The amplification product is cloned into an E. coli expression vector, comprising human cytomegalovirus (CMV) immediate early enhancer/promoter, a polyhistidine (6xHis), and a neomycin resistance gene, linearized and transfected into human embryonic kidney 293 (HEK293) cells. These cells are selected which express human ROR1 on their surface high expressing stable clones are chosen by fluorescence-activated cell sorting analysis.

### Example 1C. Human B-CLL cell line or primary B-CLL cells, multiple myeloma cell line or Mantle cell lymphoma cell line expressing ROR1 on their surface

a) Human B-CLL cell line EHEB was acquired from Leibniz Institute DSMZ-German Collection of Microorganisms and Cell Cultures (ACC-67). EHEB cells were grown in DMEM, 10% FCS, 1 % Glutamine. ROR1 expression on EHEB CLL cell lines was evaluated by flow cytometry using fluorochrome-conjugated anti-human ROR1 antibodies (BD Biosciences). ROR expression was previously reported to be expressed on EHEB CLL cell lines (Daneshmanesh et al. Leukemia 2012, 26(6):1348-55).
b) Cryopreserved human primary B-CLL cells (CD19⁺ CD5⁺) was acquired from Allcells (Alameda, CA, USA). The primary B-CLL cells from patients were lawfully obtained and comply with ethical requirements: (i) obtaining samples from patients diagnosed with CLL is approved by an Institute Reviewing Board (IRB) or Human Subject Committee; (ii) a signed and witnessed informed consent form is obtained from the patient before taking part in the Allcells Diseased Cells Program; (iii) all of the patients diagnosed with the above mentioned diseases are reasonably compensated for their commitment to the program and the compensation is approved by the IRB or Human Subject Committee; (iv) all of the patients are aware that the donated samples may be used for any research applications and waived any rights generated from the research applications.
   Primary B-CLL cells were grown in RPMI supplemented with 10% fetal bovine serum. ROR1 expression on primary CD19⁺ CD5⁺ B-CLL cells was confirmed by flow cytometry using fluorochrome-conjugated anti-human ROR-1 antibodies (see Example 1H).
c) Human B lymphocyte multiple myeloma cell line RPMI8226 was acquired from ATCC (ATCC CCL-155). RPMI8226 myeloma cells were cultured in DMEM, 10% FCS, 1 % Glutamine. ROR1 expression on RPMI8226 cell lines was confirmed by flow cytometry using fluorochrome-conjugated anti-human ROR1 antibodies (see Example 1H).
d) Human Mantle cell lymphoma (B cell non-Hodgkin's lymphoma) Rec-1cell line was acquired from ATCC (ATCC CRL-3004). Rec-1 cells were cultured in DMEM, 10% FCS, 1 % Glutamine. ROR1 expression on Rec-1 cell lines was confirmed by flow cytometry using fluorochrome-conjugated anti-human ROR1 antibodies (see Example 1H).

### Example 1D. Obtaining anti-ROR1 antibodies via immunization

Anti-ROR1 antibodies are generated by immunization of rats with ROR1 ECD. Briefly, Sprague-Dawley rats are immunized subcutaneously with keyhole limpet hemocyanin-conjugated ROR1 ECD (amino acids 5-54; NP_001183) using TiterMax® adjuvant (Sigma). Keyhole limpet hemocyanin conjugation is performed with a lysine residue using Imject mcKLHV (Pierce). Due to the high sequence homology between human and mouse ROR1 proteins, rats are preferred for antibody production. B cells are harvested from immunized spleens and fused to P3-X63.Ag8 myeloma cells using a standard polyethylene glycol fusion protocol (Goding 1996; Monoclonal antibodies: principles and practice. 3rd ed. Academic Press). Hybridomas are cultured in 80% Iscove's modified Dulbecco's medium supplemented with 10% fetal clone I, 4 mmol/L L-glutamine, 10% cloning factor and also including penicillin, streptomycin and 1× sodium hypoxanthine, aminopterin, and thymidine. ELISA testing is performed to detect binding of hybridoma culture supernatants to ROR1. Positive ROR1-binding hybridomas are further screened by flow cytometry for cell-based binding to ROR1 transfectants (HEK293-ROR1 cells). Chosen hybridomas undergo two rounds of limiting dilution cloning and are further expanded for purification. In addition, antibodies from those same chosen hybridomas are converted to chimeric antibodies with human constant regions by standard methods. Briefly, cDNAs encoding the heavy and light chain variable regions are amplified bz RT-PCR out of mRNA from the hybridomas and then joined in frame with cDNAs coding the heavy constant region of human IgG1 and the human kappa light chain constant region, respectively. These cDNAs are cloned into mammalian transient expression vectors and plasmid DNA is produced in E. coli and purified for transfection. HEK293 cells are transfected by a standard transfection method (calcium phosphate-based transfection) and 7 days later IgG1 antibodies are purified from culture supernatants by affinity chromatography on a Protein A column followed by isolation of the monomeric antibody fraction via size exclusion chromatography.

### Example IE. Obtaining anti-ROR1 antibodies out of an in vitro, recombinant library

### Example 1E1. Construction of Generic Fab-Libraries

Generic antibody libraries in the Fab-format are constructed on the basis of human germline genes using the following V-domain pairings: Vk3_20 kappa light chain with VH3_23 heavy chain for the DP47-3 library and Vk1_17 kappa light chain with VH1_69 heavy chain for the DP88-3 library. Both libraries are randomized in CDR3 of the light chain (L3) and CDR3 of the heavy chain (H3) and are assembled from 3 fragments per library by splicing by overlapping extension (SOE) PCR. Fragment 1 comprises the 5' end of the antibody gene including randomized L3, fragment 2 is a central constant fragment spanning from L3 to H3, whereas fragment 3 comprises randomized H3 and the 3' portion of the antibody gene. The following primer combinations are used to generate library fragments for DP47-3 library: fragment 1 (LMB3-LibL1b_new), fragment 2 (MS63-MS64), fragment 3 (Lib2H-fdseqlong) (see table 1 of WO2012020038). The following primer combinations are used to generate library fragments for the DP88-3 library: fragment 1 (LMB3-RJH_LIB3), fragment 2 (RJH31-RJH32) and fragment 3 (LIB88_2-fdseqlong). See tables 3 and 4 of WO2012020038.

The PCR protocol for the production of library fragments includes: 5 min of initial denaturation at 94°C; 25 cycles of 1 min at 94°C, 1 min at 58° C, and 1 min at 72°C; and terminal elongation for 10 min at 72°C. For assembly PCR, equimolar ratios of the 3 fragments are used as template. The assembly PCR protocol includes: 3 min of initial denaturation at 94°C; and 5 cycles of 30 seconds at 94°C, 1 min at 58° C, and 2 min at 72°C. At this stage, primers complementary to sequence outside fragments 1-3 are added and an additional 20 cycles are performed prior to a terminal elongation for 10 min at 72°C. After assembly of sufficient amounts of full length randomized Fab constructs, the Fab constructs are digested with NcoI/NotI for the DP47-3 library and with NcoI/NheI for the DP88-3 library alongside with similarly treated acceptor phagemid vector. For the DP47-3 library, 22.8 µg of Fab library is ligated with 16.2 µg of phagemid vector. For the DP88-3 library, 30.6 µg of Fab library is ligated with 30.6 µg of phagemid vector.

Purified ligations are used for 68 transformations for the DP47-3 library and 64 transformations for the DP88-3 library, respectively, to obtain final DP47-3 and DP88-3 libraries. Phagemid particles displaying the Fab libraries are rescued and purified by PEG/NaCl purification to be used for selection of anti-ROR1 Fab clones.

### Example 1E2. Selection of Anti-ROR1 Fab Clones

Selections are carried out against ROR1-ECD-biot. The antigen is biotinylated in vivo upon expression. Selections are carried out in solution according to the following protocol: (i) binding of ^{∼}10¹² phagemid particles of library DP88-3 and 100 nM ROR1-ECD-biot for 0.5 hours in a total volume of 1 ml; (ii) capture of ROR1-ECD-biot and attached phage by the addition of 5.4×10⁷ streptavidin-coated magnetic beads for 10 min; (iii) washing of beads using 5×1 ml PBS/Tween®20 and 5×1 ml PBS; (iv) elution of phage particles by the addition of 1 mL 100 mM TEA (triethylamine) for 10 min and neutralization by the addition of 500 µL 1M Tris/HCl pH 7.4; and (v) re-infection of log-phase *E. coli* TG1 cells (Zymo Research), infection with helper phage VCSM13 (Stratagene) and subsequent PEG/NaCl precipitation of phagemid particles to be used in subsequent selection rounds.

Selections are carried out over 3 rounds using constant ROR1-ECD-biot concentrations at 100 nM. In round 2, capture of antigen:phage complexes is performed on neutravidin plates instead of streptavidin beads. Specific binders are identified by ELISA as follows using: 100 µl of 100 nM ROR1-ECD-biot is coated in each well of neutravidin plates.

Fab-containing bacterial supernatants are added and binding Fabs are detected via their Flag-tags by using an anti-Flag/HRP secondary antibody. Once identified, anti-ROR1 ECD clones are bacterially expressed in a 0.5 litre culture volume, affinity purified and further characterized by SPR-analysis using a BIACORE® instrument.

### Example 1F. ROR1 binding assays: surface plasmon resonance

To measure binding affinities of ROR1 antibody to immobilized ROR1, surface plasmon resonance measurements are performed on a Biacore® 3000 instrument (Pharmacia Biosensor). The receptor ROR1 (ROR1-ECD) is coupled to the sensor chip at a level of 400 resonance units using the amine coupling protocol as provided by manufacturer. Alternative ROR1-ECD-biot is coupled to a streptavidin-sensor chip, also at a level of 400 resonance units, using the protocol as provided by the manufacturer. In all experiments, flow cell 1 is used as the reference cell. Sensorgrams are recorded for Fab solutions ranging in concentrations from 0.1 pM to 200 nM. Nonlinear regression analysis is used to calculate kinetic constants and binding constants simultaneously with the use of the manufacturer's software. Fab clones with monovalent binding affinities to ROR1-ECD of ≤ 100 nM are converted into IgGs by standard methods. Briefly, cDNAs encoding the heavy and light chain variable regions are joined in frame with cDNAs coding the heavy constant region of human IgG1 and the human kappa light chain constant region, respectively. These cDNAs are cloned into mammalian transient expression vectors and plasmid DNA is produced in E. coli and purified for transfection. HEK293 cells are transfected by a standard transfection method (calcium phosphate-based transfection) and 7 days later IgG1 antibodies are purified from culture supernatants by affinity chromatography on a Protein A column followed by isolation of the monomeric antibody fraction via size exclusion chromatography.

### Example 1H. Binding to ROR1 on HEK293-ROR1 cells or plate-bound-RORl, primary B-CLL cells, RPMI8226 myeloma cells or Rec-1 MCL cells (Flow cytometry and/or ELISA)

a) Anti-ROR1 antibodies coming either from the immunization approach and/or from the screening of the recombinant in vitro library described above are analyzed by flow cytometry for binding to human ROR1 on HEK293-ROR1 cells. Briefly, cultured cells are harvested, counted and cell viability is evaluated using the Trypan Blue exclusion method. Viable cells are then adjusted to 2 x 10⁶ cells per ml in BSA-containing FACS Stain Buffer (BD Biosciences). 90 µl of this cell suspension are further aliquoted per well into a round-bottom 96-well plate. 10 µl of the anti-ROR1 antibodies or corresponding IgG control are added to the cell-containing wells to obtain final concentrations of 0.1 pM to 200 nM. All constructs and control IgG are used at the same molarity. After incubation for 30 min at 4°C, the cells are centrifuged (5 min, 350 x g), washed with 150 µl/well FACS Stain Buffer (BD Biosciences), resuspended and incubated for an additional 30 min at 4°C with 12 µl/well fluorochrome-conjugated AffiniPure F(ab')₂ Fragment goat anti-human IgG Fcγ Fragment Specific (Jackson Immuno Research Lab; working solution: 1:20). Cells are then washed with Stain Buffer (BD Biosciences) 120 µl/well and pelleted down by centrifugation at 350 x g for 5 min. A second washing step is performed using FACS Stain Buffer 150 µl/well. The samples are resuspended in 200 µl/well FACS Stain Buffer and acquired and analyzed using an LSR II flow cytometer with FACSDiva® software (BD Biosciences). The mean fluorescence intensity (MFI) is plotted as a function of anti-ROR1 antibody concentration to obtain the binding curve and to calculate the effective antibody concentration to reach 50% of maximal binding (EC₅₀). Anti-ROR1 antibodies that bind to ROR1 on cells as judged from this assay are selected for the next screening step, namely the internalization assay (step (Example 1I) below).
The properties of antibodies that show binding to human ROR1 on HEK293-ROR1 cells are confirmed using a conventional ELISA method. Briefly, immunosorb 96-well plates are coated with 1.5 µg/mL of GST-ROR1-ECD, washed with PBS + 1% Tween (PBS-T), and blocked with PBS-T plus 1% serum albumin. ROR1-coated plates are incubated with hybridoma culture supernatants for 2 h at room temperature, washed 5 times with PBS-T, and incubated with peroxidase-conjugated goat-anti-rat IgG. Following incubation with secondary antibody, plates are washed, incubated with 3,3,5,5-tetramethylbenzidine substrate, and stopped with an equal volume of 1 mol/L H₂SO₄.
b) ROR1 expression was then assessed on primary CD19⁺ CD5⁺ CLL cells by flow cytometry. Briefly, cells were harvested, washed, counted for viability, resuspended at 50 000 cells/well of a 96-well round bottom plate and incubated with Alexa488-labeled anti human ROR1 antibody at 10 µg/ml for 30 min at 4°C (to prevent internalization). At the end of incubation time, cells were centrifuged (5 min at 350 x g), washed twice with FACS buffer, resuspended in 100 ul FACS buffer and analyzed on a CantoII device running FACS Diva software. Figure 2A shows an increase of median fluorescence intensity upon binding of the anti-ROR1 antibody to primary B-CLL cells, indicating that ROR1 is expressed on primary CLL cells.
c) ROR1 expression was then assessed on B lymphocyte myeloma RPMI8226 cell lines by flow cytometry, using the methods described above. Figure 3 shows increase of median fluorescence intensity upon binding of increasing concentrations of the anti-ROR1 antibody to RPMI8226 cells, but not to ROR1-negative MKN45 cells (DSMZ ACC 409). Table 1 shows the binding EC50 of anti-ROR1 antibody to ROR1-positive RPMI8226 cell lines.

**Table 1: EC50 values for binding of anti-ROR1 antibody to RPMI8226 cells**

| | **Anti-ROR1 antibody** |
|---|---|
| **EC50 (nM)** | 0.087 |
| **EC50 (µg/ml)** | 0.013 |

d) ROR1 expression was also tested on MCL Rec-1 cell lines by flow cytometry, using the methods describe above. Figure 2B shows increase of median fluorescence intensity upon binding of the anti-ROR1 antibody to Rec-1 MCL cells.

### Example 1I. Internalization of anti-ROR1 antibody on EHEB B-CLL cell line or primary PBMC from CLL patients or RPMI8226 MM cells (Flow cytometry)

Anti-ROR1 antibodies selected in step (Example 1H) above are further tested in the internalization assay. Briefly, cryopreserved human ROR1-expressing primary B-CLL target cells were thawed, harvested with Cell Dissociation Buffer, washed and resuspended in RPMI supplemented with 10% FCS at a concentration of 1 x 10⁶ (1 x 10⁶/mL) of cryopreserved PBMC from untreated CLL patients or 2 x10⁶cells/mL of EHEB B-CLL cell line or 1 x10⁶ cells/mL RPMI8226 cells after determination of cell viability using ViCell. The cell suspension was transferred in a 15 ml Falcon tube for each tested IgG/TCB and each concentration. 0.5 ml of diluted anti-ROR1 IgG or anti-ROR1/anti-CD3 TCBs conjugated with Alexa488 (diluted to 1 nM in RPMI + 10% FCS) were added to the tubes and incubated for 30 min in the cold room on a shaker. After incubation and washing the cells three times with cold PBS to remove unbound antibody, the cells were either left on ice or transferred (0.1 x 10⁶ cells) in 96-well FACS plate in pre-warmed medium and incubated at 37°C for 15 min, 30 min, 1 h, 2 h, and 24 h to facilitate internalization. In addition, sample of cells were also incubated at 37°C for 2 h and/or 24 h in the presence of 3 µM phenylarsine oxide (Sigma-Aldrich) to inhibit internalization. Subsequently, the cells were washed once with cold PBS and incubated with Alexa647-labeled anti-human Fc secondary antibody (F(ab)²) for 30 min at 4 °C. After three final washes with PBS, the cells were centrifuged 4 min at 400 x g and resuspended in FACS buffer with or without propidium iodide (1:4000) (Sigma). The mean fluorescence intensity (MFI) of the cells for anti-ROR1 IgG and anti-ROR1/anti-CD3 TCBs wais measured using a FACS CantoII flow cytometer (BD Biosciences) and FlowJo analytical software.

MFI reduction can represent antibody internalization, antibody dissociation or a combination of both. The percentage of MFI reduction is calculated for each ROR1 antibodies relative to the unspecific human IgG control (MFI_{background}) and ROR1 antibodies maintained on ice (MFIₘₐₓ) by using the formula ΔMFI= 100 - 100 X [(MFIₑₓₚₑᵣᵢₘₑₙₜₐₗ - MFI_{background}) / (MFIₘₐₓ - MFI_{background})]. An MFI reduction which is blocked by endocytosis inhibitor phenylarsine oxide indicates antibody internalization while an MFI reduction which is not blocked by phenylarsine oxide reflects antibody dissociation. Internalizing anti-ROR1 antibodies are known in the state of the art (Baskar et al., Clin. Cancer Res., 14(2): 396-404 (2008)).

For antibody-based therapies such as T cell bispecifics, it is important that the antibody or antibody fragment specific to the tumor target do not internalize, or slowly internalize, or slightly internalize for facilitating a stable immune synapse between the tumor cell and the T cell and effective T cell-mediated redirected cytotoxicity. Thus, anti-ROR1 antibodies selected in step (Example 1H) above which does not internalize or slowly internalize or slightly internalize are selected for the next step (Example 2) below, namely the production of anti-ROR1 antibodies.

The internalization values of anti-ROR1 IgG antibody in primary CLL cells and RPMI8226 cells are further summarized in Figures 4 and 6 and Tables 4 and 6.

### Example 2 - Production of anti-ROR1 antibodies

a) Selected antibodies that are derived from immunization are in a rat-human chimeric format and are then humanized to be able to apply them for therapy. In that case, standard antibody humanization methods are applied by transferring the complementarity-determining regions of those rat variable regions into human antibody variable region frameworks. Additional mutations are introduced into the variable regions, if necessary, to recover binding to ROR1 as compared to the chimeric, parental antibody.
   For the production of the antibody, the cells are co-transfected with two plasmids, (one for expression of the heavy chain of the antibody and another for expression of the light chain of the antibody), at a ratio of 1:1, respectively. Cells are grown as adherent monolayer cultures in T flasks using DMEM culture medium supplemented with 10% FCS, and are transfected when they are between 50 and 80% confluent. For the transfection of a T75 flask, 8 million cells are seeded 24 hours before transfection in 14 ml DMEM culture medium supplemented with FCS (at 10% V/V final), 250 µg/ml neomycin, and cells are placed at 37°C in an incubator with a 5% CO₂ atmosphere overnight. For each T75 flask to be transfected, a solution of DNA, CaCl₂ and water is prepared by mixing 47 µg total plasmid vector DNA divided equally between the light and heavy chain expression vectors, 235 µl of a 1M CaCl₂ solution, and adding water to a final volume of 469 µl. To this solution, 469 µl of a 50 mM HEPES, 280 mM NaCl, 1.5 mM Na₂ HPO₄ solution at pH 7.05 are added, mixed immediately for 10 sec and left to stand at room temperature for 20 sec. The suspension is diluted with 12 ml of DMEM supplemented with 2% FCS, and added to the T75 in place of the existing medium. The cells are incubated at 37°C., 5% CO₂ for about 17 to 20 hours, then medium is replaced with 12 ml DMEM, 10% FCS. The conditioned culture medium is harvested 5 to 7 days post-transfection centrifuged for 5 min at 1200 rpm, followed by a second centrifugation for 10 min at 4000 rpm and kept at 4°C.
   The secreted antibodies are purified by Protein A affinity chromatography, followed by cation exchange chromatography and a final size exclusion chromatographic step on a Superdex® 200 column (Amersham Pharmacia) exchanging the buffer to phosphate buffer saline and collecting the pure monomeric IgG1 antibodies. Antibody concentration is estimated using a spectrophotometer from the absorbance at 280 nm. The antibodies were formulated in a 25 mM potassium phosphate, 125 mM sodium chloride, 100 mM glycine solution of pH 6.7.
b) For the generation of anti-ROR1 antibody expression vectors, the variable regions of heavy and light chain DNA sequences were subcloned in frame with either the human IgG1 constant heavy chain or the hum IgG1 constant light chain pre-inserted into the respective generic recipient expression vector optimized for expression in mammalian cell lines. The antibody expression is driven by a chimeric MPSV promoter comprising a CMV enhancer and a MPSV promoter followed by a 5' UTR, an intron and a Ig kappa MAR element. The transcription is terminated by a synthetic polyA signal sequence at the 3' end of the CDS. All vectors carry a 5'-end DNA sequence coding for a leader peptide which targets proteins for secretion in eukaryotic cells. In addition each vector contains an EBV OriP sequence for episomal plasmid replication in EBV EBNA expressing cells.

### Example 3 - Generation of anti-ROR1/anti-CD3 T cell bispecific F(ab)₂ antibodies

### Example 3A. Generation of anti-CD3 antibodies

The following protein sequences of the VH and VL regions are used to generate human and cynomolgus monkey cross reactive CD3ε antibodies as described in WO2007/042261.

### H2C_VH (SEQ ID NO:7):

### H2C_VL (SEQ ID NO:8)

Briefly, oligonucleotides encoding the above sequences are joined together via PCR to synthesize cDNAs encoding the VH are VL sequences, respectively, of the anti-CD3 antibody.

Anti-CD3 antibody CH2527 (SEQ ID NO:21-28) was used to generate the T cell bispecific antibodies which were used in the following examples.

### Example 3B. Generation of anti-ROR1/anti-CD3 T cell bispecific 1+1 format (i.e. bispecific (Fab) x (Fab) antibody monovalent for ROR1 and monovalent for CD3) with or without Fc

a) An anti-ROR1/anti-CD3 T cell bispecific antibody that is devoid of an Fc part would have a safety advantage because of the lack of possibility for inducing Fc-mediated infusion reactions. In addition, because of the strong potency of T cell bispecific antibodies in killing of target cells in vitro (e.g. subnanomlolar and even picomolar range), it is desirable that for T cell bispecific antibodies specific for a tumor target that is also expressed on normal cells, such as ROR1 on normal human adipocytes, the T cell bispecific antibodies could be eliminated rapidly from the circulation after being administrated. A T cell bispecific devoid of an Fc would have a safety advantage and would be eliminated more rapidly from the circulation in case of toxicity.
   An anti-ROR1/anti-CD3 T cell bispecific antibody containing an Fc part would have the advantage of an elimination half-life of about 1 to 12 days which allows at least once or twice/week administration as compared to TCBs without an Fc portion (e.g. blinatumomab) which are required to be given intravenously and continuously with a pump carried by patients.
   Anti-ROR1/anti-CD3 T cell bispecific of the 1+1 format (i.e. bispecific (Fab)x(Fab) antibody monovalent for ROR1 and monovalent for CD3 with or without Fc) are produced fromr the human or humanized anti-ROR1 antibodies selected after step (Example 1). cDNAs encoding the full Fabs (heavy chain VH and CH1 domains plus light chain VL and CL domains) of the corresponding anti-ROR1 IgG1 antibodies, as described in Example 1, as well as the anti-CD3 VH and VL cDNAs described in Example 3A, are used as the starting materials. For each bispecific antibody, four protein chains are involved comprising the heavy and light chains of the corresponding anti-ROR1 antibody and the heavy and light chains of the anti-CD3 antibody described above, respectively, with or without any Fc regions.
   Briefly, each bispecific antibody is produced by simultaneous cotransfection of three mammalian expression vectors encoding, respectively: a) the full light chain cDNA of the corresponding ROR1 antibody, b) a fusion cDNA generated by standard molecular biology methods, such as splice-overlap-extension PCR, encoding a fusion protein made of (in N- to C-terminal order) secretory leader sequence, Fab (VH followed by CH1 domains) of the corresponding anti-ROR1 antibody described above, a flexible glycine(Gly)-serine(Ser) linker with the sequence Gly-Gly-Gly- Gly-Ser-Gly-Gly- Gly- Gly-Ser, the VH of the anti-CD3 antibody described above and the constant kappa domain of a human light chain cDNA, c) a fusion cDNA generated by standard molecular biology methods, such as splice-overlap-extension PC, encoding a fusion protein made of (in N- to C-terminal order) secretory leader sequence, VL of the anti-CD3 antibody described above, constant CH1 domain of a human IgG1 cDNA. Co-transfection of mammalian cells and antibody production and purification using the methods described above for production of human or humanized IgG1 antibodies (see Example 2), with one modification: for purification of antibodies, the first capture step is not done using ProteinA, but instead is done using an affinity chromatography column packed with a resin binding to human kappa light chain constant region, such as KappaSelect (GE Healthcare Life Sciences). In addition, a disulfide can be included to increase the stability and yields as well as additional residues forming ionic bridges and increasing the heterodimerization yields (EP 1870459A1).
b) For the generation of ROR1xCD3 bispecific antibody vectors, the IgG1 derived bispecific molecules consist at least of two antigen binding moieties capable of binding specifically to two distinct antigenic determinants CD3 and ROR1. The antigen binding moieties are Fab fragments composed of a heavy and a light chain, each comprising a variable and a constant region. At least one of the Fab fragments is a "Crossfab" fragment, wherein the constant domains of the Fab heavy and light chain are exchanged. The exchange of heavy and light chain constant domains within the Fab fragment assures that Fab fragments of different specificity do not have identical domain arrangements and consequently do not interchange light chains. The bispecific molecule design can be monovalent for both antigenic determinants (1+1) or monovalent for CD3 and bivalent for ROR1 where one Fab fragment is fused to the N-terminus of the inner CrossFab (2+1). It can be either Fc containing or non-Fc containing in order to modulate half-life of the molecule. A schematic representation of the constructs is given in Figure 1. Sequences of the constructs are shown in SEQ ID NOs 2 to 36. The molecules were produced by co-transfecting HEK293 EBNA cells growing in suspension with the mammalian expression vectors using polyethylenimine (PEI). For preparation of 1+1 CrossFab-IgG constructs, cells were transfected with the corresponding expression vectors in a 1:1:1:1 ratio ("vector Fc(knob)" : "vector light chain" : "vector light chain CrossFab" : "vector heavy chain-CrossFab").

### Example 3C. Generation of anti-ROR1/anti-CD3 T cell bispecific 2+1 format (i.e. bispecific (Fab)₂ x (Fab) antibody bivalent for ROR1 and monovalent for CD3) with or without Fc

a) An anti-ROR1/anti-CD3 T cell bispecific antibody with a 2+1 format i.e. bispecific (Fab)₂ x (Fab) antibody that is bivalent for ROR1 and monovalent for CD3 would have advantages on potency, predictability for efficacy and safety because it would preferentially bind to the tumor target ROR1 and avoid CD3 antibody sink, thus higher probability for drug exposure focused to the tumor.
   Anti -ROR1/anti-CD3 T cell bispecific of the 2+1 format (i.e. bispecific (Fab)₂ x (Fab) antibody bivalent for ROR1 and monovalent for CD3 with or without Fc are produced for the human or humanized anti-ROR1 antibodies selected after step (Example 1). cDNAs encoding the full Fabs (heavy chain VH and CH1 domains plus light chain VL and CL domains)of the corresponding anti-ROR1 IgG1 antibodies, as described in Example 1, as well as the anti-CD3 VH and VL cDNAs described in Example 3A, are used as the starting materials. For each bispecific antibody, four protein chains are involved comprising the heavy and light chains of the corresponding anti-ROR1 antibody and the heavy and light chains of the anti-CD3 antibody described above, respectively, with or without any Fc regions.
   Briefly, each bispecific antibody is produced by simultaneous cotransfection of three mammalian expression vectors encoding, respectively: a) the full light chain cDNA of the corresponding ROR1 antibody, b) a fusion cDNA generated by standard molecular biology methods, such as splice-overlap-extension PCR, encoding a fusion protein made of (in N- to C-terminal order) secretory leader sequence, Fab (VH followed by CH1 domains) of the corresponding anti-ROR1 antibody described above, a flexible glycine(Gly)-serine(Ser) linker with the sequence Gly-Gly-Gly- Gly-Ser-Gly-Gly- Gly- Gly-Ser, Fab (VH followed by CH1 domains) of the corresponding anti-ROR1 antibody described above, a flexible glycine(Gly)-serine(Ser) linker with the sequence Gly-Gly- Gly- Gly-Ser-Gly-Gly- Gly- Gly-Ser, the VH of the anti-CD3 antibody described above and the constant kappa domain of a human light chain cDNA, c) a fusion cDNA generated by standard molecular biology methods, such as splice-overlap-extension PC, encoding a fusion protein made of (in N- to C-terminal order) secretory leader sequence, VL of the anti-CD3 antibody described above, constant CH1 domain of a human IgG1 cDNA. Co-transfection of mammalian cells and antibody production and purification using the methods described above for production of human or humanized IgG1 antibodies (see Example 2), with one modification: for purification of antibodies, the first capture step is not done using ProteinA, but instead is done using an affinity chromatography column packed with a resin binding to human kappa light chain constant region, such as KappaSelect (GE Healthcare Life Sciences). In addition, a disulfide can be included to increase the stability and yields as well as additional residues forming ionic bridges and increasing the heterodimerization yields (EP 1870459A1).
b) For the generation of ROR1xCD3 bispecific antibody vectors, the IgG1 derived bispecific molecules consist at least of two antigen binding moieties capable of binding specifically to two distinct antigenic determinants CD3 and ROR1. The antigen binding moieties are Fab fragments composed of a heavy and a light chain, each comprising a variable and a constant region. At least one of the Fab fragments is a "Crossfab" fragment, wherein the constant domains of the Fab heavy and light chain are exchanged. The exchange of heavy and light chain constant domains within the Fab fragment assures that Fab fragments of different specificity do not have identical domain arrangements and consequently do not interchange light chains. The bispecific molecule design can be monovalent for both antigenic determinants (1+1) or monovalent for CD3 and bivalent for ROR1 where one Fab fragment is fused to the N-terminus of the inner CrossFab (2+1). It can be either Fc containing or non-Fc containing in order to modulate half-life of the molecule. A schematic representation of the constructs is given in Figure 1; Sequences of the constructs are shown in SEQ ID NOs 1 to 36. The molecules were produced by co-transfecting HEK293 EBNA cells growing in suspension with the mammalian expression vectors using polyethylenimine (PEI). For preparation of 2+1 CrossFab-IgG constructs, cells were transfected with the corresponding expression vectors in a 1:2:1:1 ratio ("vector Fc(knob)" : "vector light chain" : "vector light chain CrossFab" : "vector heavy chain-CrossFab").

### Example 4 - Simultaneous binding of anti-ROR1/anti-CD3 T cell bispecific antibodies to ROR1 and CD3 (Surface plasmon resonance)

The binding properties to ROR1 and CD3 of bispecific anti-ROR1/anti-CD3 T cell bispecific antibodies generated in Example 3 are analyzed by surface plasmon resonance (SPR) technology using a Biacore® T100 instrument (Biacore AB) with HBS-EP as running buffer (0.01 M HEPES pH 7.4, 0.15 M NaCl, 3 mM EDTA, 0.005% Surfactant P20, Biacore). This system is well established for the study of molecule interactions. It allows a continuous real-time monitoring of ligand/analyte bindings and thus the determination of association rate constants (ka), dissociation rate constants (kd), and equilibrium constants (KD) in various assay settings. SPR-technology is based on the measurement of the refractive index close to the surface of a gold coated biosensor chip. Changes in the refractive index indicate mass changes on the surface caused by the interaction of immobilized ligand with analyte injected in solution. If molecules bind to immobilized ligand on the surface the mass increases, in case of dissociation the mass decreases.

Capturing anti-His tag antibody is immobilized on the surface of a CM5 biosensorchip using amine-coupling chemistry. Flow cells are activated with a 1:1 mixture of 0.1 M N-hydroxysuccinimide and 0.1 M 3-(N,N-dimethylamino)propyl-N-ethylcarbodiimide at a flow rate of 5 µl/min anti-human IgG antibody is injected in sodium acetate, pH 5.0 at 10 µg/ml, which resulted in a surface density of approximately 12000 resonance units (RU). A reference control flow cell is treated in the same way but with vehicle buffers only instead of the capturing antibody. Surfaces are blocked with an injection of 1 M ethanolamine/HCl pH 8.5. The anti-ROR1/anti-CD3 T cell bispecific antibodies are diluted in HBS-P and injected at a flow rate of 5 (µl/min. The contact time (association phase) is 1 min for the antibodies at a concentration between 1 and 100 nM for the ROR1-ECD binding and 1 and 200 nM for the CD3 interaction. ROR1-ECD is injected at increasing concentrations of 3.125, 6.25, 12.5, 25, 50 and 100 nM, CD3 at concentrations of 0.21, 0.62, 1.85, 5.6, 16.7, 50, 100 and 200 nM. The contact time (association phase) is 3 min, the dissociation time (washing with running buffer) 5 min for both molecules at a flowrate of 30 (µl/min. All interactions are performed at 25°C (standard temperature). The regeneration solution of 3 M Magnesium chloride is injected for 60 s at 5 (µl/min flow to remove any non-covalently bound protein after each binding cycle. Signals are detected at a rate of one signal per second. Samples are injected at increasing concentrations. SPR graphs showing the rate of signal (i.e. resonance unit) plotted against contact time are determined.

### Example 5 - Binding of anti-ROR1/anti-CD3 T cell bispecific antibodies to ROR1-positive B-CLL cells or myeloma cells or CD3 on T cells (Flow cytometry)

a) Anti-ROR1/anti-CD3 T cell bispecific antibodies generated in Example 3 were also analyzed by flow cytometry for their binding properties to human ROR1 expressed on primary B-CLL cells or human CD3 expressed on human leukemic T cells Jurkat (ATCC TIB-152). Jurkat T cells were cultured in RPMI supplemented with 10% fetal calf serum. Briefly, cultured cells were harvested, counted and cell viability was evaluated using ViCell. Viable cells were then adjusted to 2 x 10⁶ cells per ml in FACS Stain Buffer (BD Biosciences) containing 0.1% BSA. 100 µl of this cell suspension were further aliquoted per well into a round-bottom 96-well plate. 30 µl of the Alexa488-labelled anti-ROR1/anti-CD3 T cell bispecific antibodies or corresponding IgG control were added to the cell-containing wells to obtain final concentrations of 3 nM to 500 nM or 0.1 pM to 200 nM. Anti-ROR1/anti-CD3 T cell bispecific antibodies and control IgG were used at the same molarity. After incubation for 30 min at 4°C, cells were centrifuged (5 min, 350 x g), washed twice with 150 µl/well BSA-containing FACS Stain Buffer (BD Biosciences), then cells were fixed using 100 ul BD Fixation buffer per well (#BD Biosciences, 554655) at 4°C for 20 min, resuspended in 120 µl FACS buffer and analyzed using BD FACS CantoII. Binding of the anti-ROR1/anti-CD3 T cell bispecific antibodies to B-CLL cells and T cells were evaluated and the mean fluorescence intensity was determined gated on either ROR1-expressing B-CLL cells or CD3-expressing Jurkat T cells and plotted in histograms or dot plots. Figure 7 shows the mean fluorescence intensity for anti-ROR1/anti-CD3 T cell bispecific antibodies binding to Jurkat T cells and plotted in function of antibody concentration. EC50 values and maximal binding of anti-ROR1/anti-CD3 TCB1+1 and anti-ROR1/anti-CD3 TCB2+1 antibodies to Jurkat cells were not reached. Interestingly, ROR1/anti-CD3 TCB1+1 antibody binds more efficiently to Jurkat T cells than ROR1/anti-CD3 TCB2+1 antibody does. DP47 isotype control antibody or anti-ROR1 IgG antibody did not bind to Jurkat T cells.
b) Anti-ROR1/anti-CD3 T cell bispecific antibodies were analyzed by flow cytometry for binding to human ROR1 on ROR1-expressing myeloma RPMI8226 cells. MKN45 (human gastric adenocarcinoma cell line that does not express ROR1) was used as negative control. Briefly, cultured cells were harvested, counted and cell viability was evaluated using ViCell. Viable cells were then adjusted to 2 x 10⁶ cells per ml in BSA-containing FACS Stain Buffer (BD Biosciences). 100 µl of this cell suspension was further aliquoted per well into a round-bottom 96-well plate and incubated with 30 µl of the Alexa488-labelled anti-ROR1/anti-CD3 T cell bispecific antibodies or corresponding IgG control for 30 min at 4°C. All anti-RORl/anti-CD3 T cell bispecific antibodies (and isotype control) were titrated and analyzed in final concentration range between 0.25 - 100 nM. For samples using non-labelled antibodies, cells were centrifuged (5 min, 350 x g), washed with 120µl/well FACS Stain Buffer (BD Biosciences), resuspended and incubated for an additional 30 min at 4°C with fluorochrome-conjugated PE-conjugated AffiniPure F(ab')2 Fragment goat anti-human IgG Fc Fragment Specific (Jackson Immuno Research Lab; 109-116-170). Cells were then washed twice with Stain Buffer (BD Biosciences), fixed using 100 ul BD Fixation buffer per well (#BD Biosciences, 554655) at 4°C for 20 min, resuspended in 120 µl FACS buffer and analyzed using BD FACS CantoII. Figure 8 shows the mean fluorescence intensity for anti-ROR1/anti-CD3 T cell bispecific antibodies plotted in function of antibody concentration; (A) anti-ROR1/anti-CD3 TCB1+1 and anti-ROR1/anti-CD3 TCB2+1 antibodies on RPMI8226 cells, (B) anti-ROR1/anti-CD3 TCB1+1 and anti-ROR1/anti-CD3 TCB2+1 antibodies on MKN45 cells. EC50 values (denoting the antibody concentration required to reach 50% of the maximal binding) for the binding of anti-RORl/anti-CD3 TCB1+1 and anti-ROR1/anti-CD3 TCB2+1 antibodies to RPMI8226 cells are summarized in Table 3. Surprisingly, despite being monovalent to ROR1 anti-ROR1/anti-CD3 TCB1+1 antibody seems to bind to ROR1-positive RPMI8226 myeloma cells more efficiently than anti-ROR1/anti-CD3 TCB2+1 antibody which is bivalent to ROR1, as detected by FACS (Figure 8). Anti-ROR1/anti-CD3 TCB antibodies were also shown to bind to primary B-CLL cells as detected by flow cytometry using a fluorochrome-conjugated secondary anti-human Fc antibody (Figure 2A).

**Table 3: EC50 values for binding of anti-ROR1/anti-CD3 T cell bispecific antibodies to RPMI8226 cells**

| | **Anti-ROR1/anti-CD3 TCB1+1 antibody** | **Anti-ROR1/anti-CD3 TCB2+1 antibody** |
|---|---|---|
| **EC50 (nM)** | 0.007 | 1.26 |
| **EC50 (µg/ml)** | 0.001 | 0.24 |

### Example 6. - Internalization of anti-ROR1/anti-CD3 T cell bispecific antibodies on EHEB B-CLL cell line or primary PBMC from CLL patients or RPMI8226 MM cells (Flow cytometry).

Anti-ROR1/anti-CD3 T cell bispecific antibodies selected in step (Example 5) above were further tested in the internalization assay. Briefly, cryopreserved human ROR1-expressing primary B-CLL target cells were thawed, harvested with Cell Dissociation Buffer, washed and resuspended in RPMI supplemented with 10% FCS at a concentration of 1 x 10⁶ (1 x 10⁶/mL) of cryopreserved PBMC from untreated CLL patients or 2 x10⁶cells/mL of EHEB B-CLL cell line or 1 x10⁶ cells/mL RPMI8226 cells after determination of cell viability using ViCell. The cell suspension was transferred in a 15 ml Falcon tube for each tested IgG/TCB and each concentration. 0.5 ml of diluted anti-ROR1 IgG or anti-ROR1/anti-CD3 TCBs conjugated with Alexa488 (diluted to 1 nM in RPMI + 10% FCS) were added to the tubes and incubated for 30 min in the cold room on a shaker. After incubation and washing the cells three times with cold PBS to remove unbound antibody, the cells were either left on ice or transferred (0.1 x 10⁶ cells) in 96-well FACS plate in pre-warmed medium and incubated at 37°C for 15 min, 30 min, 1 h, 2 h, and 24 h to facilitate internalization. In addition, cell samples were incubated at 37°C for 2 h and/or 24 h in the presence of 3 µM phenylarsine oxide (Sigma-Aldrich) to inhibit internalization. Subsequently, the cells were washed once with cold PBS and incubated with Alexa647-labeled anti-human Fc secondary antibody (F(ab)²) for 30 min at 4 °C. After three final washes with PBS, the cells were centrifuged 4 min at 400 x g and resuspended in FACS buffer with or without propidium iodide (1:4000) (Sigma). The mean fluorescence intensity (MFI) of the cells for anti-ROR1 IgG and anti-ROR1/anti-CD3 TCBs wais measured using a FACS CantoII flow cytometer (BD Biosciences) and FlowJo analytical software.

The term "reduction of mean fluorescence intensity" (ΔMFI) reflecting the internalization of the said anti-ROR1 antibody into ROR1-positive cells" or "MFI reduction" as used herein refers to the percentage of MFI reduction as calculated for each ROR1 antibodies relative to the unspecific human IgG control (MFI background) and ROR1 antibodies maintained on ice (MFIₘₐₓ) by using the formula ΔMFI= 100 - 100 X [(MFIₑₓₚₑᵣᵢₘₑₙₜₐₗ - MFI_{background}) / (MFIₘₐₓ - MFI_{background})]. MFIₑₓₚₑᵣᵢₘₑₙₜₐₗ is the MFI measured with said ROR1 antibody after 2h incubation at 37°C. MFI reduction can represent antibody internalization, antibody dissociation or a combination of both. An MFI reduction which is blocked by endocytosis inhibitor phenylarsine oxide indicates antibody internalization while an MFI reduction which is not blocked by phenylarsine oxide reflects antibody dissociation. Internalizing anti-ROR1 antibodies are known in the state of the art (Baskar et al., Clin. Cancer Res., 14(2): 396-404 (2008)).

In some studies, the internalization rate of anti-ROR1/anti-CD3 T cell antibodies was then compared to that of anti-ROR1 bivalent IgG antibody.

For antibody-based therapies such as T cell bispecifics, it is important that the antibody or antibody fragment specific to the tumor target does not internalize, or slowly internalizes, or slightly internalizes for facilitating a stable immune synapse between the tumor cell and the T cell and effective T cell-mediated redirected cytotoxicity and T cell activation.

As shown in Figures 4A and 4B and summarized in Table 4, anti-ROR1 IgG antibody (1nM) internalized about 12.5% in primary B-CLL cells after an incubation of 2 hrs at 37°C while anti-ROR1/anti-CD3 TCB2+1 antibody (1 nM) showed an internalization rate of 27.1% in primary B-CLL cells at the same experimental conditions (Figures 4A and 4C) as measured by FACS (indirect detection of secondary fluorochrome-conjugated antibody). Internalization was calculated based on the MFI value at time 0, baseline, and calculated using the previously described formula. The results show that anti-ROR1/anti-CD3 TCB2+1 has an internalization rate of less than 30% in B-CLL cells.

Figure 5 shows the internalization rate of anti-ROR1/anti-CD3 TCB1+1 antibody (1 nM) in primary B-CLL cells after an incubation of 2 hrs at 37°C in the presence or absence of phenylarsine oxide (PAO). Because reduction of MFI signal can be due to internalization and/or dissociation of the antibody, it is important to verify if a reduction of MFI signal is caused by internalization or not by using an endocytosis inhibitor to block internalization. This is particularly important for monovalent antibodies that have lower binding avidity to cells than bivalent antibodies. As shown in Figure 5, there was a decrease of 91% in the MFI signal in primary B-CLL cells after an incubation of 2 hrs at 37°C without PAO. However, when the B-CLL cells were incubated in the presence of PAO (3 µM), 90% decrease in MFI signal was still observed indicating that the loss in MFI signal was not due to internalization of the antibody but rather probably dissociation (Table 5). Internalization rate could then be calculated to 0%. The results demonstrate that anti-ROR1/anti-CD3 TCB1+1 does not internalize in B-CLL cells, which is a preferred feature for a TCB antibody.

Figure 6 and Table 6 summarize the internalization rates of TCB2+1 antibodies and anti-ROR1 IgG antibody (1 nM) in RPMI8226 MM cells after an incubation of 2 hrs at 37°C, as measured in two independent experiments. The results demonstrate that anti-ROR1/anti-CD3 TCB2+1 has an internalization rate of less than 15% in RPMI cells.

**Table 4: Internalization values for anti-ROR1/anti-CD3 2+1 T cell bispecific antibody and ROR1 IgG in primary B-CLL cells**

| | **Internalization of anti-ROR1 antibody (%)** | **Internalization of anti-ROR1/anti-CD3 TCB2+1 antibody (%)** |
|---|---|---|
| **Time 0** | 0 (baseline) | 0 (baseline) |
| **Time 2 hrs** | 12.5 | 27.1 |

**Table 5: Internalization values for anti-ROR1/anti-CD3 1+1 T cell bispecific antibody and ROR1 IgG in primary B-CLL cells**

| | **Internalization of anti-ROR1/anti-CD3 TCB1+1 antibody (%)** |
|---|---|
| **Time 0** | 0 (baseline) |
| **Time 2 hrs** | 0 |

**Table 6: Internalization values for anti-ROR1/anti-CD3 T cell bispecific antibodies and ROR1 IgG in RPMI8226 MM cells**

| **Experiment 1** | **Internalization of anti-ROR1 antibody (%)** | **Internalization of anti-ROR1/anti-CD3 TCB1+1 antibody (%)** |
|---|---|---|
| **Time 0** | 0 (baseline) | 0 (baseline) |
| **Time 2 hrs** | 0.7 | 8.6 |
| | | |

| **Experiment 2** | **Internalization of anti-ROR1 antibody (%)** | **Internalization of anti-ROR1/anti-CD3 TCB1+1 antibody (%)** |
|---|---|---|
| **Time 0** | 0 (baseline) | 0 (baseline) |
| **Time 2 hrs** | 0 | 11.8 |

### Example 7 - Activation of T cells upon engagement of anti-ROR1/anti-CD3 T cell bispecific antibodies (Flow cytometry)

a) Anti-ROR1/anti-CD3 T cell bispecific antibodies generated in Example 3 were also analyzed by flow cytometry for their potential to induce T cell activation by evaluating the surface expression of the early activation marker CD69, or the late activation marker CD25 on CD4⁺ and CD8⁺ T cells in the presence or absence of human ROR1-positive cells. Briefly, ROR1-positive cells were harvested with Cell Dissociation buffer, counted and cell viability is verified using ViCell. Viable B-CLL cells were adjusted to 0.2 x 10⁶ cells/mL in RPMI supplemented with 10% FCS, 100 µl of this cell suspension per well was pipetted into a round-bottom 96-well plate. 50 µl of the T cell bispecific constructs were added to the ROR1-positive cells-containing wells to obtain a final concentration of 0.01 fM to 100 pM or 0.01 pM to 100 nM. The 96-well plate was set aside and kept at 37°C, 5% CO₂ until further manipulations.

PBMC were isolated from fresh blood using density gradient centrifugation using Cell Preparation Tubes with Sodium citrate (Vacutainer CPT tubes, BD Biosciences). Total human T cells were then isolated using the Pan T Cell Isolation Kit II (Miltenyi Biotec), according to the manufacturer's instructions. In some studies, CD8 T cell clones were used as effectors. CD8 T cells specific to NLV (a CMV specific peptide recognized by HLA-A2) were purified from HLA-A2+ healthy donor PBMCs using aCD8 antibodies and tetramers specific to HLA-A2 complexed with NLV peptide and sorted with a cell sorter. The purified cells were expanded on irradiated feeder preparations obtained from healthy donor PBMC and HLA-A2+LCLs (lymphoblastoid cells) pulsed with NLV peptide in media (RPMI1640+10%FBS+1%L-glutamine) with 400IU IL2. The NLV specific CD8 T cell clones were maintained in the same media with 400IU IL2 and regularly reactivated on feeder preparations. Human total T cells or CD8 T cell clones (effectors) were then adjusted to 2 x 10⁶ cells per ml in RPMI supplemented with 10% FCS. 50 µl of this cell suspension was added per well in the assay plate containing already ROR1-positive target cells to obtain a final E:T ratio of 3:1 (CD8 T cells as effectors) or 10:1 (PBMC as effectors). To test whether the T cell bispecific constructs were able to activate T cells in the presence of only ROR1-positive tumor target cells, wells containing final concentration(s) in the range of 0.01 fM to 100 pM or 0.01 pM to 100 nM of the respective bispecific molecules with effector cells but without ROR1-positive tumor target cells were also included. After incubation for 6 to 24h (CD8 T cell clones as effectors) or 24 to 48 hrs (PBMC as effectors) at 37°C, 5% CO₂, cells were pelleted down by centrifugation (5 min, 350 x g) and washed twice with 150 µl/well of FACS Stain Buffer (BD Biosciences). Surface staining of the effector cells with selected fluorochrome-conjugated antibodies against human CD4, CD8, CD69 or CD25 (BD Biosciences) was performed at 4°C for 30 min, protected from light, in FACS Stain Buffer (BD Biosciences) according to the manufacturer's protocol. Cells were washed twice with 150 µl/well FACS Stain Buffer then fixed using 100 ul BD Fixation buffer per well (#BD Biosciences, 554655) at 4°C for 20 min, resuspended in 120 µl FACS buffer and analyzed using BD FACS CantoII. The expression of CD69 or CD25 activation markers were determined by measuring the mean fluorescence intensity gated on CD4⁺ and CD8⁺ T cell populations as represented in histograms or dot plots.

Figure 9A shows the concentration dependent increase in the mean fluorescence intensity of the late activation marker CD25 gated on CD8 T cells. The results indicates that anti-RORl/anti-CD3 TCB1+1 antibody induced a significant concentration dependent activation of CD8 T cells in the presence of ROR1-positive Rec-1 cells and the maximum signal was reached with 100 pM of antibody. Unspecific activation of CD8 T cells was minimal upon binding of CD3 on T cells but without binding on ROR1-positive target cells obtained by non-binder TCB constructs. Although the activation of CD8 T cells was not as pronounced with anti-ROR1/anti-CD3 TCB2+1 antibody, there was a faint but noticeable increase in CD25 mean fluorescence intensity. However, unspecific actvation could not be ruled out.

Figure 9B shows the concentration dependent upregulation of CD25 on CD8 T cells mediated by anti-ROR1/anti-CD3 TCB1+1 and anti-ROR1/anti-CD3 TCB2+1 antibodies in the presence of ROR1-positive RPMI8226 MM cells. At the highest concentration (100 pM) of TCB antibodies tested there was no unspecific activation of CD8 T cells as shown in comparison to the non-binder TCB constructs.

### Example 8 - Proliferation of T cells upon engagement of anti-ROR1/anti-CD3 T cell bispecific antibodies (CFSE dilution)

Anti-ROR1/anti-CD3 T cell bispecific antibodies generated in Example 3 are also analyzed by flow cytometry for their potential to induce proliferation of CD8⁺ or CD4⁺ T cells in the presence or absence of human ROR1-positive cells. Briefly, ROR1-positive cells are harvested with Cell Dissociation buffer, counted and looked for viability using Trypan Blue. Viable B-CLL cells are adjusted to 0.2 x 10⁶ cells per ml in complete RPMI medium, 100 µl of this cell suspension are pipetted per well into a round-bottom 96-well plate. 50 µl of the T cell bispecific constructs are added to the target cells-containing wells to obtain final concentration(s) in the range of 0.1 fM to 200 nM. The well plate is set aside and kept at 37°C, 5% CO₂.

PBMC are isolated from fresh blood using density gradient centrifugation using Cell Preparation Tubes with Sodium citrate (Vacutainer CPT tubes, BD Biosciences). Total human T cells are then isolated using the Pan T Cell Isolation Kit II (Miltenyi Biotec), according to the manufacturer's instructions. The total T cells are then adjusted to 1 million cells per ml in pre-warm RPMI without serum (37°C) and stained with 1 µM CFSE at room temperature for 6 min, protected from light. The staining volume is then doubled by addition of RPMI-1640 medium supplemented with 10% FCS and 1% GlutaMax to stop CFSE staining. After incubation at room temperature for further 20 min, the cells are washed three times with pre-warmed serum-containing medium to remove remaining CFSE. CFSE-stained total T cells (effector) are then adjusted to 2 x 10⁶ cells/mL in complete RPMI-1640 medium. 50 µl of this cell suspension is added per well in the assay plate already containing ROR1-positive cells to obtain a final E:T ratio of 5:1. To test whether the T cell bispecific constructs are able to activate T cells only in the presence of ROR1-positive tumor target cells, wells containing 1 nM of the T cell bispecific antibodies with effector cells but without tumor target cells are also included. After incubation for five days at 37°C, 5% CO₂, cells are pelleted down by centrifugation (5 min, 350 x g) and washed twice with 150 µl/well of FACS Stain Buffer (BD Biosciences). Surface staining of the effector cells with selected fluorochrome-conjugated antibodies against human CD4, CD8 or CD25 (BD) is performed at 4°C for 30 min, protected from light, in FACS Stain Buffer according to the manufacturer's protocol. Cells are washed twice with 150 µl/well FACS Stain Buffer, resuspended in 200 µl/well FACS Stain Buffer, and acquired and analyzed using a LSR II flow cytometer complemented with FACSDiva® software (BD). The percentage of non-proliferating cells is determined by gating on the far right undiluted CFSE peak in the group which the wells contain ROR1-positive cells and CFSE-stained T cells but without the T cell bispecific antibodies, and compared that to other experimental groups (wells). The percentage of proliferating cells is measured by gating all the diluted CFSE peaks excluding the far right peak (if observable). The proliferation level of CD4⁺ and CD8⁺ T cells is determined by gating on that population first then to further look at the CFSE dilution peaks.

### Example 9 - Cytokine production from activated T cells upon engagement of anti-ROR1/anti-CD3 T cell bispecific antibodies

### Example 9A. Interferon-y production

Anti-ROR1/anti-CD3 T cell bispecific antibodies generated in Example 3 are also analyzed for their potential to induce interferon-y (IFN-y) production by the T cells in the presence or absence of human ROR1-positive cells. Briefly, ROR1-positive cells are harvested with Cell Dissociation buffer, counted and looked for viability using Trypan Blue. Approximately 20,000 viable cells per well are plated in a round-bottom 96-well-plate and the respective antibody dilution is added to obtain final concentration(s) in the range of 0.1 pM to 200 nM. Anti-human ROR1 and anti-CD3 IgGs adjusted to the same molarity are used as controls. Human total T effector cells are added to obtain a final E:T ratio of 5:1. After 20 h incubation at 37°C, 5% CO₂, human IFN-γ levels in the supernatant are measured by ELISA, according to the manufacturer's instructions (human IFN-γELISA Kit II, BD Biosciences). The levels of IFN-γproduced by T cells in the presence of anti-ROR1/anti-CD3 T cell bispecific antibody and ROR1-positive cells is measured and plotted in histograms and compared to that produced by T cells in the presence of anti-ROR1/anti-CD3 T cell bispecific antibody and but without ROR1-positive cells.

### Example 9B. Cytokine release assay (CBA analysis)

Anti-ROR1/anti-CD3 T cell bispecific antibodies generated in Example 3 are also analyzed for their potential to induce T-cell mediated cytokine production in the presence or absence of human ROR1-positive cells. PBMC are isolated from fresh blood using density gradient centrifugation using Cell Preparation Tubes with Sodium citrate (Vacutainer CPT tubes, BD Biosciences) and a final cell concentration of 0.3 million cells/well are plated into a round-bottom 96-well plate. ROR1-positive cells are then added to obtain a final E:T-ratio of 10:1, as well as T cell bispecific constructs and IgG controls are added to obtain final concentration(s) in the range of 0.1 pM to 200 nM, for a 24 h incubation at 37°C, 5% CO₂. The next day, the cells are centrifuged for 5 min at 350 x g and the supernatant is transferred into a new deep-well 96-well-plate for the further analysis. The CBA analysis is performed according to manufacturer's instructions for LSR II flow cytometer, using the Human Th1/Th2 Cytokine Kit II (BD Biosciences) including human IL-2, human IL-4, human IL-6, human IL-10, human TNF-α, and human IFN-γ. The levels of cytokines produced by T cells in the presence of anti-RORl/anti-CD3 T cell bispecific antibody and ROR1-positive cells is measured and plotted in histograms and compared to that produced by T cells in the presence of anti-ROR1/anti-CD3 T cell bispecific antibody and but without ROR1-positive cells.

### Example 10 - Redirected T cell cytotoxicity of B-CLL Cells upon cross-linking of anti-ROR1/anti-CD3 T cell bispecific antibodies to CD3 on T cells and ROR1 on B-CLL cells (LDH release assay)

Anti-ROR1/anti-CD3 T cell bispecific antibodies generated in Example 3 were also analyzed for their potential to induce T cell-mediated apoptosis in ROR1-expressing B-CLL cells upon crosslinking of the construct via binding of the antigen binding moieties to ROR1 on cells. Briefly, cryopreserved human ROR1-expressing primary B-CLL target cells were thawed, harvested with Cell Dissociation Buffer, washed and resuspended in RPMI supplemented with 10% FCS. Approximately, 30,000 cells per well were plated in a round-bottom 96-well plate and the respective dilution of the construct is added for a desired final concentration (in triplicates); final concentrations ranging from 0.01 fM to 100pM or 0.2 nM to 30 nM. For an appropriate comparison, all T cell bispecific constructs and controls were adjusted to the same molarity. Human total T cells or CD8 T cell clones (effectors) were added into the wells to obtain a final E:T ratio of 3:1. When human PBMC were used as effector cells, a final E:T ratio of 10:1 was used. PHA-L (Sigma) was used as positive control for human T cell activation at a concentration of 1 µg/ml. Negative control groups were represented by effectors or target cells only. For normalization, maximal lysis of the B-CLL target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 6 to 24 h incubation (CD8 T cell clones as effectors) or 24h to 48h (PBMC as effectors) at 37°C, 5% CO₂, lactate dehydrogenase (LDH) release from the apoptotic/necrotic B-CLL target cells into the supernatant was then measured with the LDH detection kit (Roche Applied Science), following the manufacturer's instructions. The percentage of LDH release was plotted against the concentrations of anti-ROR1/anti-CD3 T cell bispecific antibodies in concentration-response curves. The IC₅₀ values were measured using Prism software (GraphPad) and determined as the T cell bispecific antibody concentration that results in 50% of LDH release.

### Example 11 - Redirected T cell cytotoxicity of multiple myeloma cells upon cross-linking of anti-ROR1/anti-CD3 T cell bispecific antibodies to CD3 on T cells and ROR1 on multiple myeloma cells (LDH release assay)

Anti-ROR1/anti-CD3 T cell bispecific antibodies generated in Example 3 were also analyzed for their potential to induce T cell-mediated apoptosis in ROR1-expressing multiple myeloma cells upon crosslinking of the construct via binding of the antigen binding moieties to ROR1 on cells. Briefly, human ROR1-expressing RPMI-8226 multiple myeloma target cells (available from American Type Culture Collection; ATCC CCL-155) were harvested with Cell Dissociation Buffer, washed and resuspended in RPMI supplemented with 10% FCS. Approximately, 30,000 cells per well were plated in a round-bottom 96-well plate and the respective dilution of the construct was added for a desired final concentration (in triplicates); final concentrations ranging from 0.01 fM to 100 pM or 0.2 nM to 30 nM. For an appropriate comparison, all T cell bispecific constructs and controls were adjusted to the same molarity. Human total T cells or CD8 T cell clones (effectors) were added into the wells to obtain a final E:T ratio of 3:1. When human PBMC were used as effector cells, a final E:T ratio of 10:1 was used. PHA-L (Sigma) was used as positive control for human T cell activation at a concentration of 1 µg/ml. Negative control groups were represented by effector or target cells only. For normalization, maximal lysis of the RPMI-8226 multiple myeloma target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 6 to 24 hrs incubation (CD8 T cell clones as effectors) or 24 to 48 hrs incubation (PBMC as effectors) at 37°C, 5% CO₂, LDH release from the apoptotic/necrotic ROR1-positive target cells into the supernatant was then measured with the LDH detection kit (Roche Applied Science), following the manufacturer's instructions. The percentage of LDH release was plotted against the concentrations of anti-ROR1/anti-CD3 T cell bispecific antibodies in concentration-response curves. The IC₅₀ values were measured using Prism software (GraphPad) and determined as the T cell bispecific antibody concentration that results in 50% of LDH release.

Figure 10 shows the redirected T cell killing of ROR1-positive RPMI8226 MM target cells by CD8 T cells activated by anti-ROR1/anti-CD3 TCB antibodies. Specific cytotoxicity of target cells (tumor lysis) induced by anti-ROR1/anti-CD3 TCB antibodies was measured by LDH release. (A) Experiment 1 (14h time point): A very slight concentration dependent increase of tumor lysis response was observed with anti-ROR1/anti-CD3 TCB1+1 antibody. 30% of tumor lysis was already observed with the lowest concentration tested of 0.01 pM anti-ROR1/anti-CD3 TCB1+1 antibody and up to 37.5% tumor lysis was reached with 30 nM of anti-ROR1/anti-CD3 TCB antibodies in experimental conditions reflecting clinically relevant E:T ratio of 3:1 i.e. 3 CD8 T cells for 1 RPMI 8226 target cell. EC50 could not be calculated. The 37.5% tumor lysis observed at 30 nM as detected by LDH release could not have been attributed only to unspecific killing of target cells as there was only 17% unspecific target cell lysis with 30 nM of non-binder TCB1+1 (i.e. binds to effector cells but not to target cells). For anti-ROR1/anti-CD3 TCB2+1 antibody, a maximum target cell lysis of 30% was already observed at the lowest concentration tested of 0.2 fM and there was no concentration dependent response with increasing concentrations for up to 10 nM. However, cell lysis with the non-binder TCB 2+1 in a concentration of 30 nM was already close to 30%. (B) Experiment 2 (20h time point): The study was repeated in ROR1-positive RPMI8226 and measurement of LDH release was assessed after 20h incubation. 30-40% target cell lysis was observed with anti-ROR1/anti-CD3 TCB1+1 and TCB2+1 antibodies at a concentration of 100 pM while non-binder TCB controls at 100 pM did not induce any tumor lysis. The results corroborate with an increase in T cell activation as measured by upregulation of CD25 marker on the CD8 T cells (Figure 9B).

The overall in vitro results with ROR1-positive blood cancer cells (CLL, MM, and MCL) clearly show that anti-ROR1/anti-CD3 TCB1+1 and anti-ROR1/anti-CD3 TCB2+1 molecules act like T cell bispecific antibodies as they 1) bind to ROR1-positive target cells; 2) bind to CD3-postive T cells; 3) mediate T cell activation upon simultaneous binding to target cells and T cells; and 4) induce redirected T cell cytotoxicity of ROR1-positive target cells in a concentration-dependent manner which corroborate with the upregulation of CD25 on T cells.

### Example 12 - Redirected T cell cytotoxicity of cell lines from solid tumors upon cross-linking of anti-ROR1/anti-CD3 T cell bispecific antibodies to CD3 on T cells and ROR1 on solid tumor cells (LDH release assay)

Anti-ROR1/anti-CD3 T cell bispecific antibodies generated in Example 3 are also analyzed for their potential to induce T cell-mediated apoptosis in ROR1-expressing malignant cells from solid tumors (i.e. target cells) upon crosslinking of the construct via binding of the antigen binding moieties to ROR1 on cells. Briefly, human ROR1-expressing lung cancer cell line A549 (available from American Type Culture Collection; ATCC CCL-185) or breast cancer cell line MDA-MB-468 (available from American Type Culture Collection; ATCC HTB-132) are harvested with Cell Dissociation Buffer, washed and resuspended in RPMI supplemented with 10% FCS. Approximately, 30,000 cells per well are plated in a round-bottom 96-well plate and the respective dilution of the construct is added for a desired final concentration (in triplicates); final concentrations ranging from 0.1 fM to 200 nM. For an appropriate comparison, all T cell bispecific constructs and controls are adjusted to the same molarity. Human total T cells (effector) are added into the wells to obtain a final E:T ratio of 3:1. When human PBMC are used as effector cells, a final E:T ratio of 10:1 is used. PHA-L (Sigma) is used as positive control for human T cell activation at a concentration of 1 µg/ml. Negative control groups are represented by effector or target cells only. For normalization, maximal lysis of the lung cancer cell line A549 or breast cancer cell line MDA-MB-468 target cells (= 100%) is determined by incubation of the target cells with a final concentration of 1% Triton X-100, inducing cell death. Minimal lysis (= 0%) is represented by target cells co-incubated with effector cells only, i.e. without any T cell bispecific antibody. After 20 h incubation at 37°C, 5% CO₂, LDH release from the apoptotic/necrotic ROR1-positive target cells into the supernatant is then measured with the LDH detection kit (Roche Applied Science), following the manufacturer's instructions. The percentage of LDH release is plotted against the concentrations of **anti-ROR1/anti-CD3** T cell bispecific antibodies in concentration-response curves. The IC₅₀ values are measured using Prism software (GraphPad) and determined as the T cell bispecific antibody concentration that results in 50% of LDH release.

### Example 13 - Evaluation of therapeutic efficacy of anti-ROR1/anti-CD3 T cell bispecific antibody in CLL xenograft SCID beige mouse model

Adult female CB17-severe combined immunodeficient (SCID) beige mice (Charles River Laboratories), are maintained under specific pathogen-free conditions according to guidelines. Continuous health monitoring is carried out on a regular basis. Mice are monitored daily for clinical symptoms and detection of adverse effects. Throughout the experimental period, the body weight of animals is recorded twice weekly and tumor volume is measured by caliper after staging. CB17-SCID mice are first reconstituted with human PBMC via tail vein injection then they are inoculated subcutaneously into their lower dorsum with EHEB B-CLL cells in 100 µL PBS and 100 µL Matrigel basement membrane matrix (Becton Dickinson). When tumors are palpable and reach ∼300 mm³, mice are randomly assigned into cohorts of 10 mice each and treated with ROR1-TCB 10-50 µg/mouse/ twice a week administered intravenously via the tail vein or an equal amount of control IgGs. Animals are sacrificed, according to institutional guidelines, when the diameter of their tumors reaches 3 cm or when significant toxicity is observed. Animal body weight and any sign of morbidity are monitored.

### Example 14 - Evaluation of therapeutic efficacy of anti-ROR1/anti-CD3 T cell bispecific antibody in the Vk*MYC multiple myeloma mouse model

Murine cross-reactive anti-ROR1/anti-CD3 T cell bispecific antibodies are tested for their potential to prevent multiple myeloma in Vk*MYC multiple myeloma prone mice as described in Chesi, 2012 (Chesi et al. 2012; Blood 120: 376-385). Multiple myeloma is a hematological malignancy involving an uncontrolled expansion of plasma cells in the bone marrow. Since ROR1 is strongly expressed on malignant plasma cells, we hypothesize that an anti-RORl/anti-CD3 T cell bispecific will be efficacious for the treatment of multiple myeloma. The Vk*MYC multiple myeloma mouse model is highly representative of human myeloma and predictive of drug response used in the clinic; representing an excellent tool for testing the preclinical proof-of-concept of anti-ROR1/anti-CD3 T cell bispecific antibodies. Briefly, Vk*MYC mice obtained from an academic collaboration at Mayo Clinic Arizona are crossed with human CD3ε transgenic (huCD3ε Tg) mice. T cells from huCD3ε Tg x Vk*MYC mice express both human CD3ε and mouse CD3ε on the cell surface and the mice are therefore responsive to anti-ROR1/anti-CD3 T cell bispecifics. Vk*MYC mice uniformly develop a monoclonal gammopathy initiated at around 30 weeks of age that progresses slowly over time associated with clinical signs representative of human myeloma such as anemia, osteoporosis and renal disease. Mice are periodically bled by tail grazing and blood is collected into Microtainer tubes (BD Biosciences), let coagulate at room temperature then spun for 10 min at 2,300g. Sera are diluted 1:2 in normal saline buffer and analyzed on a QuickGel Chamber apparatus using pre-casted QuickGels (Helena Laboratories) according to manufacturer's instruction. Gamma/albumin ratio and serum fractions are measured by densitometric analysis.

For therapeutic studies, Vk*MYC mice are enrolled and randomized into different treatment groups (n=5-8/group): for example, 1) control IgGs; 2) anti-ROR1/anti-CD3 T cell bispecific antibodies; 500 (µg/kg/week or 10 µg/mouse/week administered intravenously via the tail vein; 3) bortezomib 1 mg/kg/ i.p. on days 1,4,8,11 used as standard of care. Preferably, the dose(s) of anti-ROR1/anti-CD3 T cell bispecific antibodies could be multiple and range from 200 to 1000 µg/kg/week. In each group, at least three aged (>1 year old) Vk*MYC mice with gamma/albumin ratio between 0.3-2.0, corresponding to a predominant M-spike between approximately 10-70 g/l as measured by densitometry. Serum protein electrophoresis (SPEP) is performed on day 0 and day 14 post treatment to measure treatment-mediated reduction in the M-spike as a marker of tumor response, as done in the clinic. In some therapeutic studies, transplanted Vk*MYC mice with an M-spike approximately 10-70 g/l and a bone marrow plasmacytosis of greater than 5% are enrolled and assigned to different treatment groups. The efficacy of anti-ROR1/anti-CD3 T cell bispecific antibodies to reduce M-spike is evaluated.

## Claims

1. A bispecific antibody specifically binding to the two targets human CD3ε and the extracellular domain of human ROR1 (ROR1), wherein the bispecific antibody has the construct ROR1 Fab - Fc - CD3 Fab - ROR1 Fab.

2. The bispecific antibody according to claim 1, wherein the variable domains VL and VH or the constant domains CL and CH1 of the CD3 Fab are replaced by each other.

3. The bispecific antibody according to claim 1 or 2, wherein the variable domains VL and VH of the CD3 Fab are replaced by each other.

4. The bispecific antibody according to any one of the preceding claims, wherein the antibody does not internalize at a concentration of 1 nM in an assay using ROR1-positive primary B-CLL cells at 37°C for two hours, wherein not internalize means that the mean fluorescence intensity detected by flow cytometry of the antibody binding at time 0 is not reduced by more than 50%, preferably not more than 30%, when re-measured after a two-hour incubation at 37°C.

5. The bispecific antibody according to any one of the preceding claims, wherein the CD3 Fab comprises the CDRs of SEQ ID NO: 12, 13 and 14 as respectively heavy chain CDR1, CDR2 and CDR3 and the CDRs of SEQ ID NO: 15, 16 and 17 as respectively light chain CDR1, CDR2 and CDR3, optionally wherein the CD3 Fab comprises the variable domain VH of SEQ ID NO: 10 and the variable domain VL of SEQ ID NO: 11.

6. The bispecific antibody according to any one of claims 1 to 4, wherein the CD3 Fab comprises the CDRs of SEQ ID NO: 23, 24 and 25 as respectively heavy chain CDR1, CDR2 and CDR3 and the CDRs of SEQ ID NO: 26, 27 and 28 as respectively light chain CDR1, CDR2 and CDR3, optionally wherein the CD3 Fab comprises the variable domain VH of SEQ ID NO: 21 and the variable domain VL of SEQ ID NO: 22.

7. The bispecific antibody according to any one of the preceding claims, wherein each ROR1 Fab comprises the CDRs of SEQ ID NO: 7, 8 and 9 as respectively heavy chain CDR1, CDR2 and CDR3 and the CDRs of SEQ ID NO: 3, 4 and 5 as respectively light chain CDR1, CDR2 and CDR3.

8. The bispecific antibody according to claim 1, wherein the construct consists of building blocks SEQ ID NO: 30 (2x), 31, 32, and 33.

9. A method for the preparation of the bispecific antibody according to any one of claims 1 to 8 comprising the steps of transforming a host cell with one or more vectors comprising nucleic acid molecules encoding the respective antibodies and/or fragments and culturing the host cell under conditions that allow synthesis of said antibody molecule; and recovering said antibody molecule from said culture.

10. A host cell comprising vectors comprising nucleic acid molecules encoding the antibody according to any one of claims 1 to 8.

11. A pharmaceutical composition comprising the antibody according to any one of claims 1 to 8 and a pharmaceutically acceptable excipient.

12. The bispecific antibody according to any one of claims 1 to 8 or the pharmaceutical composition according to claim 11 for use as a medicament.

13. The bispecific antibody according to any one of claims 1 to 8 or the pharmaceutical composition according to claim 11 for use as a medicament in the treatment of a ROR1-positive hematological malignancy selected from chronic lymphocytic leukemia (CLL), hairy cell leukemia (HCL), acute lymphoblastic leukemia (ALL), acute myeloid leukemia (AML), chronic myeloid leukemia (CML), mantle cell lymphoma (MCL), marginal zone lymphoma (MZL), diffuse large B cell lymphoma (DLBCL), multiple myeloma (MM) and follicular lymphoma (FL).

14. The bispecific antibody according to any one of claims 1 to 8 or the pharmaceutical composition according to claim 11 for use as a medicament in the treatment of a ROR1-positive solid tumor, optionally wherein the ROR1-positive tumor is breast cancer or lung cancer.

15. The bispecific antibody according to any one of claims 1 to 8 or the pharmaceutical composition according to claim 11 for use as a medicament in the treatment of chronic lymphocytic leukemia of B-cell lineage (B-CLL) or plasma cell disorder, optionally wherein the plasma cell disorder is Multiple Myeloma (MM) or another B-cell disorder expressing ROR1.

## Patentansprüche

1. Ein bispezifischer Antikörper, der spezifisch an die zwei Ziele humanes CD3ε und die extrazelluläre Domäne von humanem ROR1 (ROR1) bindet, wobei der bispezifische Antikörper das Konstrukt ROR1 Fab - Fc - CD3 Fab - ROR1 Fab aufweist.

2. Bispezifischer Antikörper nach Anspruch 1, wobei die variablen Domänen VL und VH oder die konstanten Domänen CL und CH1 des CD3 Fab miteinander ausgetauscht sind.

3. Bispezifischer Antikörper nach Anspruch 1 oder 2, wobei die variablen Domänen VL und VH des CD3 Fab miteinander ausgetauscht sind.

4. Bispezifischer Antikörper nach einem der vorhergehenden Ansprüche, wobei der Antikörper bei einer Konzentration von 1 nM in einem Assay unter Verwendung von ROR1-positiven primären B-CLL-Zellen bei 37 °C für zwei Stunden nicht internalisiert, wobei nicht internalisiert bedeutet, dass die anhand Durchflusszytometrie detektierte mittlere Fluoreszenzintensität der Antikörperbindung zur Zeit 0 um nicht mehr als 50 %, bevorzugt um nicht mehr als 30 % reduziert ist, wenn sie nach einer zweistündigen Inkubation bei 37 °C erneut gemessen wird.

5. Bispezifischer Antikörper nach einem der vorhergehenden Ansprüche, wobei das CD3 Fab die CDRs von SEQ ID NO: 12, 13 und 14 als schwerkettige CDR1, CDR2 bzw. CDR3 und die CDRs von SEQ ID NO: 15, 16 und 17 als leichtkettige CDR1, CDR2 bzw. CDR3 beinhaltet, wobei optional das CD3 Fab die variable Domäne VH von SEQ ID NO: 10 und die variable Domäne VL von SEQ ID NO: 11 beinhaltet.

6. Bispezifischer Antikörper nach einem der Ansprüche 1 bis 4, wobei das CD3 Fab die CDRs von SEQ ID NO: 23, 24 und 25 als schwerkettige CDR1, CDR2 bzw. CDR3 und die CDRs von SEQ ID NO: 26, 27 und 28 als leichtkettige CDR1, CDR2 bzw. CDR3 beinhaltet, wobei optional das CD3 Fab die variable Domäne VH von SEQ ID NO: 21 und die variable Domäne VL von SEQ ID NO: 22 beinhaltet.

7. Bispezifischer Antikörper nach einem der vorhergehenden Ansprüche, wobei jedes ROR1 Fab die CDRs von SEQ ID NO: 7, 8 und 9 als schwerkettige CDR1, CDR2 bzw. CDR3 und die CDRs von SEQ ID NO: 3, 4 und 5 als leichtkettige CDR1, CDR2 bzw. CDR3 beinhaltet.

8. Bispezifischer Antikörper nach Anspruch 1, wobei das Konstrukt aus Bausteinen SEQ ID NO: 30 (2x), 31, 32 und 33 besteht.

9. Ein Verfahren zur Herstellung des bispezifischen Antikörpers nach einem der Ansprüche 1 bis 8, das die folgenden Schritte beinhaltet: Transformieren einer Wirtszelle mit einem oder mehreren Vektoren, die Nukleinsäuremoleküle beinhalten, die für die jeweiligen Antikörper und/oder Fragmente kodieren, und Kultivieren der Wirtszelle unter Bedingungen, welche die Synthese des genannten Antikörpermoleküls erlauben; und Wiedergewinnen des genannten Antikörpermoleküls aus der genannten Kultur.

10. Eine Wirtszelle, die Vektoren beinhaltet, die Nukleinsäuremoleküle beinhalten, die für den Antikörper nach einem der Ansprüche 1 bis 8 kodieren.

11. Eine pharmazeutische Zusammensetzung, die den Antikörper nach einem der Ansprüche 1 bis 8 und einen pharmazeutisch akzeptablen Hilfsstoff beinhaltet.

12. Bispezifischer Antikörper nach einem der Ansprüche 1 bis 8 oder pharmazeutische Zusammensetzung nach Anspruch 11 zur Verwendung als ein Medikament.

13. Bispezifischer Antikörper nach einem der Ansprüche 1 bis 8 oder pharmazeutische Zusammensetzung nach Anspruch 11 zur Verwendung als ein Medikament bei der Behandlung einer ROR1-positiven malignen hämatologischen Erkrankung, ausgewählt aus chronischer lymphatischer Leukämie (CLL), Haarzell-Leukämie (HCL), akuter lymphatischer Leukämie (ALL), akuter myeloischer Leukämie (AML), chronischer myeloischer Leukämie (CML), Mantelzell-Lymphom (MCL), Marginalzonen-Lymphom (MZL), diffusem großem B-Zell-Lymphom (DLBCL), multiplem Myelom (MM) und follikulärem Lymphom (FL).

14. Bispezifischer Antikörper nach einem der Ansprüche 1 bis 8 oder pharmazeutische Zusammensetzung nach Anspruch 11 zur Verwendung als ein Medikament bei der Behandlung eines ROR1-positiven soliden Tumors, wobei optional der ROR1-positive Tumor Brustkrebs oder Lungenkrebs ist.

15. Bispezifischer Antikörper nach einem der Ansprüche 1 bis 8 oder pharmazeutische Zusammensetzung nach Anspruch 11 zur Verwendung als ein Medikament bei der Behandlung von chronischer lymphatischer Leukämie der B-Zelllinie (B-CLL) oder einer Plasmazellstörung, wobei optional die Plasmazellstörung multiples Myelom (MM) oder eine andere B-Zellstörung ist, die ROR1 exprimiert.

## Revendications

1. Anticorps bispécifique qui se lie spécifiquement aux deux cibles que sont le gène CD3_{ε} humain et le domaine extracellulaire du récepteur ROR1 humain (ROR1), l'anticorps bispécifique ayant la construction ROR1 Fab - Fc - CD3 Fab - ROR1 Fab.

2. Anticorps bispécifique selon la revendication 1, dans lequel les domaines variables VL et VH ou les domaines constants CL et CH1 de CD3 Fab sont remplacés l'un par l'autre.

3. Anticorps bispécifique selon la revendication 1 ou 2, dans lequel les domaines variables VL et VH de CD3 Fab sont remplacés l'un par l'autre.

4. Anticorps bispécifique selon l'une quelconque des revendications précédentes, l'anticorps ne s'internalisant pas à une concentration de 1 nM dans un essai pour lequel on utilise des cellules B-CLL primaires ROR1-positives à 37 °C pendant deux heures, le terme « ne s'internalisant pas » signifiant que l'intensité de fluorescence moyenne détectée par cytométrie en flux correspondant à la liaison de l'anticorps au temps 0 n'est pas réduite de plus de 50 %, et n'est préférablement pas réduite de plus de 30 %, lorsqu'elle est mesurée de nouveau après une incubation de deux heures à 37 °C.

5. Anticorps bispécifique selon l'une quelconque des revendications précédentes, dans lequel CD3 Fab comprend les CDR des SÉQ. ID n^{os} 12, 13 et 14, correspondant respectivement aux régions CDR1, CDR2 et CDR3 de chaîne lourde, et les CDR des SÉQ. ID n^{OS} 15, 16 et 17, correspondant respectivement aux régions CDR1, CDR2 et CDR3 de chaîne légère, optionnellement dans lequel CD3 Fab comprend le domaine variable VH de la SÉQ. ID n° 10 et le domaine variable VL de la SÉQ. ID n° 11.

6. Anticorps bispécifique selon l'une quelconque des revendications 1 à 4, dans lequel CD3 Fab comprend les CDR des SÉQ. ID n^{os} 23, 24 et 25, correspondant respectivement aux régions CDR1, CDR2 et CDR3 de chaîne lourde, et les CDR des SÉQ. ID n^{os} 26, 27 et 28, correspondant respectivement aux régions CDR1, CDR2 et CDR3 de chaîne légère, optionnellement dans lequel CD3 Fab comprend le domaine variable VH de la SÉQ. ID n° 21 et le domaine variable VL de la SÉQ. ID n° 22.

7. Anticorps bispécifique selon l'une quelconque des revendications précédentes, dans lequel chaque ROR1 Fab comprend les CDR des SÉQ. ID n^{os} 7, 8 et 9, correspondant respectivement aux régions CDR1, CDR2 et CDR3 de chaîne lourde et les CDR des SÉQ. ID n^{os} 3, 4 et 5, correspondant respectivement aux régions CDR1, CDR2 et CDR3 de chaîne légère.

8. Anticorps bispécifique selon la revendication 1, dans lequel la construction se compose d'éléments constituants des SÉQ. ID n^{os} 30 (2x), 31, 32 et 33.

9. Procédé de préparation de l'anticorps bispécifique selon l'une quelconque des revendications 1 à 8, comprenant les étapes de transformation d'une cellule hôte avec un ou plusieurs vecteurs comprenant des molécules d'acide nucléique codant les anticorps et/ou fragments respectifs et de mise en culture de la cellule hôte dans des conditions qui permettent la synthèse de ladite molécule d'anticorps ; et la récupération de ladite molécule d'anticorps à partir de ladite culture.

10. Cellule hôte comprenant des vecteurs comprenant des molécules d'acide nucléique codant l'anticorps selon l'une quelconque des revendications 1 à 8.

11. Composition pharmaceutique comprenant l'anticorps selon l'une quelconque des revendications 1 à 8 et un excipient pharmaceutiquement acceptable.

12. Anticorps bispécifique selon l'une quelconque des revendications 1 à 8 ou composition pharmaceutique selon la revendication 11, destiné(e) à une utilisation comme médicament.

13. Anticorps bispécifique selon l'une quelconque des revendications 1 à 8 ou composition pharmaceutique selon la revendication 11, destiné(e) à une utilisation comme médicament dans le traitement d'une malignité hématologique ROR1-positive sélectionnée parmi la leucémie lymphocytaire chronique (LLC), la leucémie à tricholeucocytes (LT), la leucémie lymphoblastique aiguë (LLA), la leucémie myéloïde aiguë (LMA), la leucémie myéloïde chronique (LMC), le lymphome à cellules du manteau (LCM), le lymphome de la zone marginale, (LZM), le lymphome diffus à grandes cellules B (LDGCB), le myélome multiple (MM) et le lymphome folliculaire (LF) .

14. Anticorps bispécifique selon l'une quelconque des revendications 1 à 8 ou composition pharmaceutique selon la revendication 11, destiné(e) à une utilisation comme médicament dans le traitement d'une tumeur solide ROR1-positive, la tumeur ROR1-positive étant optionnellement un cancer du sein ou un cancer du poumon.

15. Anticorps bispécifique selon l'une quelconque des revendications 1 à 8 ou composition pharmaceutique selon la revendication 11, destiné(e) à une utilisation comme médicament dans le traitement d'une leucémie lymphoïde chronique de la lignée cellulaire B (B-CLL) ou d'une affection touchant les plasmocytes, l'affection touchant les plasmocytes étant optionnellement le myélome multiple (MM) ou une autre affection touchant les cellules B exprimant ROR1.


### SEQUENCE LISTING

<110> ENGMAB AG
<120> Bispecific antibodies against CD3É\233 and ROR1
<150> EP13001840.1
   <151> 2013-04-09
<160> 36
<170> BiSSAP 1.2
<210> 1
   <211> 937
   <212> PRT
   <213> Homo sapiens
<400> 1
   Met His Arg Pro Arg Arg Arg Gly Thr Arg Pro Pro Leu Leu Ala Leu
   1               5                   10                  15
   Leu Ala Ala Leu Leu Leu Ala Ala Arg Gly Ala Ala Ala Gln Glu Thr
               20                  25                  30
   Glu Leu Ser Val Ser Ala Glu Leu Val Pro Thr Ser Ser Trp Asn Ile
           35                  40                  45
   Ser Ser Glu Leu Asn Lys Asp Ser Tyr Leu Thr Leu Asp Glu Pro Met
       50                  55                  60
   Asn Asn Ile Thr Thr Ser Leu Gly Gln Thr Ala Glu Leu His Cys Lys
   65                  70                  75                  80
   Val Ser Gly Asn Pro Pro Pro Thr Ile Arg Trp Phe Lys Asn Asp Ala
                   85                  90                  95
   Pro Val Val Gln Glu Pro Arg Arg Leu Ser Phe Arg Ser Thr Ile Tyr
               100                 105                 110
   Gly Ser Arg Leu Arg Ile Arg Asn Leu Asp Thr Thr Asp Thr Gly Tyr
           115                 120                 125
   Phe Gln Cys Val Ala Thr Asn Gly Lys Glu Val Val Ser Ser Thr Gly
       130                 135                 140
   Val Leu Phe Val Lys Phe Gly Pro Pro Pro Thr Ala Ser Pro Gly Tyr
   145                 150                 155                 160
   Ser Asp Glu Tyr Glu Glu Asp Gly Phe Cys Gln Pro Tyr Arg Gly Ile
                   165                 170                 175
   Ala Cys Ala Arg Phe Ile Gly Asn Arg Thr Val Tyr Met Glu Ser Leu
               180                 185                 190
   His Met Gln Gly Glu Ile Glu Asn Gln Ile Thr Ala Ala Phe Thr Met
           195                 200                 205
   Ile Gly Thr Ser Ser His Leu Ser Asp Lys Cys Ser Gln Phe Ala Ile
       210                 215                 220
   Pro Ser Leu Cys His Tyr Ala Phe Pro Tyr Cys Asp Glu Thr Ser Ser
   225                 230                 235                 240
   Val Pro Lys Pro Arg Asp Leu Cys Arg Asp Glu Cys Glu Ile Leu Glu
                   245                 250                 255
   Asn Val Leu Cys Gln Thr Glu Tyr Ile Phe Ala Arg Ser Asn Pro Met
               260                 265                 270
   Ile Leu Met Arg Leu Lys Leu Pro Asn Cys Glu Asp Leu Pro Gln Pro
           275                 280                 285
   Glu Ser Pro Glu Ala Ala Asn Cys Ile Arg Ile Gly Ile Pro Met Ala
       290                 295                 300
   Asp Pro Ile Asn Lys Asn His Lys Cys Tyr Asn Ser Thr Gly Val Asp
   305                 310                 315                 320
   Tyr Arg Gly Thr Val Ser Val Thr Lys Ser Gly Arg Gln Cys Gln Pro
                   325                 330                 335
   Trp Asn Ser Gln Tyr Pro His Thr His Thr Phe Thr Ala Leu Arg Phe
                   340                 345                 350
   Pro Glu Leu Asn Gly Gly His Ser Tyr Cys Arg Asn Pro Gly Asn Gln
               355                 360                 365
   Lys Glu Ala Pro Trp Cys Phe Thr Leu Asp Glu Asn Phe Lys Ser Asp
           370                 375                 380
   Leu Cys Asp Ile Pro Ala Cys Asp Ser Lys Asp Ser Lys Glu Lys Asn
   385                 390                 395                 400
   Lys Met Glu Ile Leu Tyr Ile Leu Val Pro Ser Val Ala Ile Pro Leu
                       405                 410                 415
   Ala Ile Ala Leu Leu Phe Phe Phe Ile Cys Val Cys Arg Asn Asn Gln
                   420                 425                 430
   Lys Ser Ser Ser Ala Pro Val Gln Arg Gln Pro Lys His Val Arg Gly
               435                 440                 445
   Gln Asn Val Glu Met Ser Met Leu Asn Ala Tyr Lys Pro Lys Ser Lys
           450                 455                 460
   Ala Lys Glu Leu Pro Leu Ser Ala Val Arg Phe Met Glu Glu Leu Gly
   465                 470                 475                 480
   Glu Cys Ala Phe Gly Lys Ile Tyr Lys Gly His Leu Tyr Leu Pro Gly
                       485                 490                 495
   Met Asp His Ala Gln Leu Val Ala Ile Lys Thr Leu Lys Asp Tyr Asn
                   500                 505                 510
   Asn Pro Gln Gln Trp Thr Glu Phe Gln Gln Glu Ala Ser Leu Met Ala
               515                 520                 525
   Glu Leu His His Pro Asn Ile Val Cys Leu Leu Gly Ala Val Thr Gln
           530                 535                 540
   Glu Gln Pro Val Cys Met Leu Phe Glu Tyr Ile Asn Gln Gly Asp Leu
   545                 550                 555                 560
   His Glu Phe Leu Ile Met Arg Ser Pro His Ser Asp Val Gly Cys Ser
                       565                 570                 575
   Ser Asp Glu Asp Gly Thr Val Lys Ser Ser Leu Asp His Gly Asp Phe
                   580                 585                 590
   Leu His Ile Ala Ile Gln Ile Ala Ala Gly Met Glu Tyr Leu Ser Ser
               595                 600                 605
   His Phe Phe Val His Lys Asp Leu Ala Ala Arg Asn Ile Leu Ile Gly
           610                 615                 620
   Glu Gln Leu His Val Lys Ile Ser Asp Leu Gly Leu Ser Arg Glu Ile
   625                 630                 635                 640
   Tyr Ser Ala Asp Tyr Tyr Arg Val Gln Ser Lys Ser Leu Leu Pro Ile
                       645                 650                 655
   Arg Trp Met Pro Pro Glu Ala Ile Met Tyr Gly Lys Phe Ser Ser Asp
                   660                 665                 670
   Ser Asp Ile Trp Ser Phe Gly Val Val Leu Trp Glu Ile Phe Ser Phe
               675                 680                 685
   Gly Leu Gln Pro Tyr Tyr Gly Phe Ser Asn Gln Glu Val Ile Glu Met
           690                 695                 700
   Val Arg Lys Arg Gln Leu Leu Pro Cys Ser Glu Asp Cys Pro Pro Arg
   705                 710                 715                 720
   Met Tyr Ser Leu Met Thr Glu Cys Trp Asn Glu Ile Pro Ser Arg Arg
                       725                 730                 735
   Pro Arg Phe Lys Asp Ile His Val Arg Leu Arg Ser Trp Glu Gly Leu
                   740                 745                 750
   Ser Ser His Thr Ser Ser Thr Thr Pro Ser Gly Gly Asn Ala Thr Thr
               755                 760                 765
   Gln Thr Thr Ser Leu Ser Ala Ser Pro Val Ser Asn Leu Ser Asn Pro
           770                 775                 780
   Arg Tyr Pro Asn Tyr Met Phe Pro Ser Gln Gly Ile Thr Pro Gln Gly
   785                 790                 795                 800
   Gln Ile Ala Gly Phe Ile Gly Pro Pro Ile Pro Gln Asn Gln Arg Phe
                       805                 810                 815
   Ile Pro Ile Asn Gly Tyr Pro Ile Pro Pro Gly Tyr Ala Ala Phe Pro
                   820                 825                 830
   Ala Ala His Tyr Gln Pro Thr Gly Pro Pro Arg Val Ile Gln His Cys
               835                 840                 845
   Pro Pro Pro Lys Ser Arg Ser Pro Ser Ser Ala Ser Gly Ser Thr Ser
       850                 855                 860
   Thr Gly His Val Thr Ser Leu Pro Ser Ser Gly Ser Asn Gln Glu Ala
   865                 870                 875                 880
   Asn Ile Pro Leu Leu Pro His Met Ser Ile Pro Asn His Pro Gly Gly
                   885                 890                 895
   Met Gly Ile Thr Val Phe Gly Asn Lys Ser Gln Lys Pro Tyr Lys Ile
               900                 905                 910
   Asp Ser Lys Gln Ala Ser Leu Leu Gly Asp Ala Asn Ile His Gly His
           915                 920                 925
   Thr Glu Ser Met Ile Ser Ala Glu Leu
       930                 935
<210> 2
   <211> 112
   <212> PRT
   <213> Oryctolagus cuniculus
<400> 2
   Glu Leu Val Leu Thr Gln Ser Pro Ser Val Ser Ala Ala Leu Gly Ser
   1               5                   10                  15
   Pro Ala Lys Ile Thr Cys Thr Leu Ser Ser Ala His Lys Thr Asp Thr
               20                  25                  30
   Ile Asp Trp Tyr Gln Gln Leu Gln Gly Glu Ala Pro Arg Tyr Leu Met
           35                  40                  45
   Gln Val Gln Ser Asp Gly Ser Tyr Thr Lys Arg Pro Gly Val Pro Asp
       50                  55                  60
   Arg Phe Ser Gly Ser Ser Ser Gly Ala Asp Arg Tyr Leu Ile Ile Pro
   65                  70                  75                  80
   Ser Val Gln Ala Asp Asp Glu Ala Asp Tyr Tyr Cys Gly Ala Asp Tyr
                   85                  90                  95
   Ile Gly Gly Tyr Val Phe Gly Gly Gly Thr Gln Leu Thr Val Thr Gly
               100                 105                 110
<210> 3
   <211> 12
   <212> PRT
   <213> Oryctolagus cuniculus
<400> 3
   Thr Leu Ser Ser Ala His Lys Thr Asp Thr Ile Asp
   1               5                   10
<210> 4
   <211> 7
   <212> PRT
   <213> Oryctolagus cuniculus
<400> 4
   Gly Ser Tyr Thr Lys Arg Pro
   1               5
<210> 5
   <211> 9
   <212> PRT
   <213> Oryctolagus cuniculus
<400> 5
   Gly Ala Asp Tyr Ile Gly Gly Tyr Val
   1               5
<210> 6
   <211> 121
   <212> PRT
   <213> Oryctolagus cuniculus
<400> 6
   Gln Glu Gln Leu Val Glu Ser Gly Gly Arg Leu Val Thr Pro Gly Gly
   1               5                   10                  15
   Ser Leu Thr Leu Ser Cys Lys Ala Ser Gly Phe Asp Phe Ser Ala Tyr
               20                  25                  30
   Tyr Met Ser Trp Val Arg Gln Ala Pro Gly Lys Gly Leu Glu Trp Ile
           35                  40                  45
   Ala Thr Ile Tyr Pro Ser Ser Gly Lys Thr Tyr Tyr Ala Thr Trp Val
       50                  55                  60
   Asn Gly Arg Phe Thr Ile Ser Ser Asp Asn Ala Gln Asn Thr Val Asp
   65                  70                  75                  80
   Leu Gln Met Asn Ser Leu Thr Ala Ala Asp Arg Ala Thr Tyr Phe Cys
                   85                  90                  95
   Ala Arg Asp Ser Tyr Ala Asp Asp Gly Ala Leu Phe Asn Ile Trp Gly
               100                 105                 110
   Pro Gly Thr Leu Val Thr Ile Ser Ser
           115                 120
<210> 7
   <211> 5
   <212> PRT
   <213> Oryctolagus cuniculus
<400> 7
   Ala Tyr Tyr Met Ser
   1               5
<210> 8
   <211> 17
   <212> PRT
   <213> Oryctolagus cuniculus
<400> 8
   Thr Ile Tyr Pro Ser Ser Gly Lys Thr Tyr Tyr Ala Thr Trp Val Asn
   1               5                   10                  15
   Gly
<210> 9
   <211> 12
   <212> PRT
   <213> Oryctolagus cuniculus
<400> 9
   Asp Ser Tyr Ala Asp Asp Gly Ala Leu Phe Asn Ile
   1               5                   10
<210> 10
   <211> 125
   <212> PRT
   <213> Mus musculus
<400> 10
   Glu Val Gln Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Asn Lys Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Lys Asn Thr
   65                  70                  75                  80
   Ala Tyr Leu Gln Met Asn Asn Leu Lys Thr Glu Asp Thr Ala Val Tyr
                   85                  90                  95
   Tyr Cys Val Arg His Gly Asn Phe Gly Asn Ser Tyr Ile Ser Tyr Trp
               100                 105                 110
   Ala Tyr Trp Gly Gln Gly Thr Leu Val Thr Val Ser Ser
           115                 120                 125
<210> 11
   <211> 109
   <212> PRT
   <213> Mus musculus
<400> 11
   Gln Thr Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Ser Gly
               20                  25                  30
   Tyr Tyr Pro Asn Trp Val Gln Gln Lys Pro Gly Gln Ala Pro Arg Gly
           35                  40                  45
   Leu Ile Gly Gly Thr Lys Phe Leu Ala Pro Gly Thr Pro Ala Arg Phe
       50                  55                  60
   Ser Gly Ser Leu Leu Gly Gly Lys Ala Ala Leu Thr Leu Ser Gly Val
   65                  70                  75                  80
   Gln Pro Glu Asp Glu Ala Glu Tyr Tyr Cys Ala Leu Trp Tyr Ser Asn
                   85                  90                  95
   Arg Trp Val Phe Gly Gly Gly Thr Lys Leu Thr Val Leu
               100                 105
<210> 12
   <211> 10
   <212> PRT
   <213> Mus musculus
<400> 12
   Gly Phe Thr Phe Asn Lys Tyr Ala Met Asn
   1               5                   10
<210> 13
   <211> 19
   <212> PRT
   <213> Mus musculus
<400> 13
   Arg Ile Arg Ser Lys Tyr Asn Asn Tyr Ala Thr Tyr Tyr Ala Asp Ser
   1               5                   10                  15
   Val Lys Asp
<210> 14
   <211> 14
   <212> PRT
   <213> Mus musculus
<400> 14
   His Gly Asn Phe Gly Asn Ser Tyr Ile Ser Tyr Trp Ala Tyr
   1               5                   10
<210> 15
   <211> 14
   <212> PRT
   <213> Mus musculus
<400> 15
   Gly Ser Ser Thr Gly Ala Val Thr Ser Gly Tyr Tyr Pro Asn
   1               5                   10
<210> 16
   <211> 7
   <212> PRT
   <213> Mus musculus
<400> 16
   Gly Thr Lys Phe Leu Ala Pro
   1               5
<210> 17
   <211> 9
   <212> PRT
   <213> Mus musculus
<400> 17
   Ala Leu Trp Tyr Ser Asn Arg Trp Val
   1               5
<210> 18
   <211> 15
   <212> PRT
   <213> Homo sapiens
<400> 18
   Trp Asn Ile Ser Ser Glu Leu Asn Lys Asp Ser Tyr Leu Thr Leu
   1               5                   10                  15
<210> 19
   <211> 10
   <212> PRT
   <213> Artificial Sequence
<220>
   <223> artificial linker sequence
<400> 19
   Gly Gly Gly Gly Ser Gly Gly Gly Gly Ser
   1               5                   10
<210> 20
   <211> 14
   <212> PRT
   <213> Homo sapiens
<400> 20
   Lys Ser Gln Lys Pro Tyr Lys Ile Asp Ser Lys Gln Ala Ser
   1               5                   10
<210> 21
   <211> 125
   <212> PRT
   <213> Mus musculus
<400> 21
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Thr Tyr
               20                  25                  30
   Ala Met Asn Trp Val Arg Gln Ala Pro Gly Lys Gly Leu Glu Trp Val
           35                  40                  45
   Ser Arg Ile Arg Ser Lys Tyr Asn Asn Tyr Ala Thr Tyr Tyr Ala Asp
       50                  55                  60
   Ser Val Lys Gly Arg Phe Thr Ile Ser Arg Asp Asp Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ser
           115                 120                 125
<210> 22
   <211> 109
   <212> PRT
   <213> Mus musculus
<400> 22
   Gln Ala Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Thr Ser
               20                  25                  30
   Asn Tyr Ala Asn Trp Val Gln Glu Lys Pro Gly Gln Ala Phe Arg Gly
           35                  40                  45
   Leu Ile Gly Gly Thr Asn Lys Arg Ala Pro Gly Thr Pro Ala Arg Phe
       50                  55                  60
   Ser Gly Ser Leu Leu Gly Gly Lys Ala Ala Leu Thr Leu Ser Gly Ala
   65                  70                  75                  80
   Gln Pro Glu Asp Glu Ala Glu Tyr Tyr Cys Ala Leu Trp Tyr Ser Asn
                   85                  90                  95
   Leu Trp Val Phe Gly Gly Gly Thr Lys Leu Thr Val Leu
               100                 105
<210> 23
   <211> 5
   <212> PRT
   <213> Mus musculus
<400> 23
   Thr Tyr Ala Met Asn
   1               5
<210> 24
   <211> 19
   <212> PRT
   <213> Mus musculus
<400> 24
   Arg Ile Arg Ser Lys Tyr Asn Asn Tyr Ala Thr Tyr Tyr Ala Asp Ser
   1               5                   10                  15
   Val Lys Gly
<210> 25
   <211> 14
   <212> PRT
   <213> Mus musculus
<400> 25
   His Gly Asn Phe Gly Asn Ser Tyr Val Ser Trp Phe Ala Tyr
   1               5                   10
<210> 26
   <211> 14
   <212> PRT
   <213> Mus musculus
<400> 26
   Gly Ser Ser Thr Gly Ala Val Thr Thr Ser Asn Tyr Ala Asn
   1               5                   10
<210> 27
   <211> 7
   <212> PRT
   <213> Mus musculus
<400> 27
   Gly Thr Asn Lys Arg Ala Pro
   1               5
<210> 28
   <211> 9
   <212> PRT
   <213> Mus musculus
<400> 28
   Ala Leu Trp Tyr Ser Asn Leu Trp Val
   1               5
<210> 29
   <211> 451
   <212> PRT
   <213> Mus musculus
<400> 29
   Gln Glu Gln Leu Val Glu Ser Gly Gly Arg Leu Val Thr Pro Gly Gly
   1               5                   10                  15
   Ser Leu Thr Leu Ser Cys Lys Ala Ser Gly Phe Asp Phe Ser Ala Tyr
               20                  25                  30
   Tyr Met Ser Trp Val Arg Gln Ala Pro Gly Lys Gly Leu Glu Trp Ile
           35                  40                  45
   Ala Thr Ile Tyr Pro Ser Ser Gly Lys Thr Tyr Tyr Ala Thr Trp Val
       50                  55                  60
   Asn Gly Arg Phe Thr Ile Ser Ser Asp Asn Ala Gln Asn Thr Val Asp
   65                  70                  75                  80
   Leu Gln Met Asn Ser Leu Thr Ala Ala Asp Arg Ala Thr Tyr Phe Cys
                   85                  90                  95
   Ala Arg Asp Ser Tyr Ala Asp Asp Gly Ala Leu Phe Asn Ile Trp Gly
               100                 105                 110
   Pro Gly Thr Leu Val Thr Ile Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Ala Ala Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Gly Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 30
   <211> 236
   <212> PRT
   <213> Mus musculus
<400> 30
   Met Gly Trp Ser Cys Ile Ile Leu Phe Leu Val Ala Thr Ala Thr Gly
   1               5                   10                  15
   Val His Ser Glu Leu Val Leu Thr Gln Ser Pro Ser Val Ser Ala Ala
               20                  25                  30
   Leu Gly Ser Pro Ala Lys Ile Thr Cys Thr Leu Ser Ser Ala His Lys
           35                  40                  45
   Thr Asp Thr Ile Asp Trp Tyr Gln Gln Leu Gln Gly Glu Ala Pro Arg
       50                  55                  60
   Tyr Leu Met Gln Val Gln Ser Asp Gly Ser Tyr Thr Lys Arg Pro Gly
   65                  70                  75                  80
   Val Pro Asp Arg Phe Ser Gly Ser Ser Ser Gly Ala Asp Arg Tyr Leu
                   85                  90                  95
   Ile Ile Pro Ser Val Gln Ala Asp Asp Glu Ala Asp Tyr Tyr Cys Gly
               100                 105                 110
   Ala Asp Tyr Ile Gly Gly Tyr Val Phe Gly Gly Gly Thr Gln Leu Thr
           115                 120                 125
   Val Leu Gly Gln Pro Lys Ala Ala Pro Ser Val Thr Leu Phe Pro Pro
       130                 135                 140
   Ser Ser Glu Glu Leu Gln Ala Asn Lys Ala Thr Leu Val Cys Leu Ile
   145                 150                 155                 160
   Ser Asp Phe Tyr Pro Gly Ala Val Thr Val Ala Trp Lys Ala Asp Ser
                   165                 170                 175
   Ser Pro Val Lys Ala Gly Val Glu Thr Thr Thr Pro Ser Lys Gln Ser
               180                 185                 190
   Asn Asn Lys Tyr Ala Ala Ser Ser Tyr Leu Ser Leu Thr Pro Glu Gln
           195                 200                 205
   Trp Lys Ser His Arg Ser Tyr Ser Cys Gln Val Thr His Glu Gly Ser
       210                 215                 220
   Thr Val Glu Lys Thr Val Ala Pro Thr Glu Cys Ser
   225                 230                 235
<210> 31
   <211> 694
   <212> PRT
   <213> Mus musculus
<400> 31
   Gln Glu Gln Leu Val Glu Ser Gly Gly Arg Leu Val Thr Pro Gly Gly
   1               5                   10                  15
   Ser Leu Thr Leu Ser Cys Lys Ala Ser Gly Phe Asp Phe Ser Ala Tyr
               20                  25                  30
   Tyr Met Ser Trp Val Arg Gln Ala Pro Gly Lys Gly Leu Glu Trp Ile
           35                  40                  45
   Ala Thr Ile Tyr Pro Ser Ser Gly Lys Thr Tyr Tyr Ala Thr Trp Val
       50                  55                  60
   Asn Gly Arg Phe Thr Ile Ser Ser Asp Asn Ala Gln Asn Thr Val Asp
   65                  70                  75                  80
   Leu Gln Met Asn Ser Leu Thr Ala Ala Asp Arg Ala Thr Tyr Phe Cys
                   85                  90                  95
   Ala Arg Asp Ser Tyr Ala Asp Asp Gly Ala Leu Phe Asn Ile Trp Gly
               100                 105                 110
   Pro Gly Thr Leu Val Thr Ile Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Gly Gly Gly Gly Ser Gly Gly Gly Gly Ser Glu Val Gln Leu Leu
   225                 230                 235                 240
   Glu Ser Gly Gly Gly Leu Val Gln Pro Gly Gly Ser Leu Arg Leu Ser
                   245                 250                 255
   Cys Ala Ala Ser Gly Phe Thr Phe Ser Thr Tyr Ala Met Asn Trp Val
               260                 265                 270
   Arg Gln Ala Pro Gly Lys Gly Leu Glu Trp Val Ser Arg Ile Arg Ser
           275                 280                 285
   Lys Tyr Asn Asn Tyr Ala Thr Tyr Tyr Ala Asp Ser Val Lys Gly Arg
       290                 295                 300
   Phe Thr Ile Ser Arg Asp Asp Ser Lys Asn Thr Leu Tyr Leu Gln Met
   305                 310                 315                 320
   Asn Ser Leu Arg Ala Glu Asp Thr Ala Val Tyr Tyr Cys Val Arg His
                   325                 330                 335
   Gly Asn Phe Gly Asn Ser Tyr Val Ser Trp Phe Ala Tyr Trp Gly Gln
               340                 345                 350
   Gly Thr Leu Val Thr Val Ser Ser Ala Ser Val Ala Ala Pro Ser Val
           355                 360                 365
   Phe Ile Phe Pro Pro Ser Asp Glu Gln Leu Lys Ser Gly Thr Ala Ser
       370                 375                 380
   Val Val Cys Leu Leu Asn Asn Phe Tyr Pro Arg Glu Ala Lys Val Gln
   385                 390                 395                 400
   Trp Lys Val Asp Asn Ala Leu Gln Ser Gly Asn Ser Gln Glu Ser Val
                   405                 410                 415
   Thr Glu Gln Asp Ser Lys Asp Ser Thr Tyr Ser Leu Ser Ser Thr Leu
               420                 425                 430
   Thr Leu Ser Lys Ala Asp Tyr Glu Lys His Lys Val Tyr Ala Cys Glu
           435                 440                 445
   Val Thr His Gln Gly Leu Ser Ser Pro Val Thr Lys Ser Phe Asn Arg
       450                 455                 460
   Gly Glu Cys Asp Lys Thr His Thr Cys Pro Pro Cys Pro Ala Pro Glu
   465                 470                 475                 480
   Ala Ala Gly Gly Pro Ser Val Phe Leu Phe Pro Pro Lys Pro Lys Asp
                   485                 490                 495
   Thr Leu Met Ile Ser Arg Thr Pro Glu Val Thr Cys Val Val Val Asp
               500                 505                 510
   Val Ser His Glu Asp Pro Glu Val Lys Phe Asn Trp Tyr Val Asp Gly
           515                 520                 525
   Val Glu Val His Asn Ala Lys Thr Lys Pro Arg Glu Glu Gln Tyr Asn
       530                 535                 540
   Ser Thr Tyr Arg Val Val Ser Val Leu Thr Val Leu His Gln Asp Trp
   545                 550                 555                 560
   Leu Asn Gly Lys Glu Tyr Lys Cys Lys Val Ser Asn Lys Ala Leu Gly
                   565                 570                 575
   Ala Pro Ile Glu Lys Thr Ile Ser Lys Ala Lys Gly Gln Pro Arg Glu
               580                 585                 590
   Pro Gln Val Tyr Thr Leu Pro Pro Cys Arg Asp Glu Leu Thr Lys Asn
           595                 600                 605
   Gln Val Ser Leu Trp Cys Leu Val Lys Gly Phe Tyr Pro Ser Asp Ile
       610                 615                 620
   Ala Val Glu Trp Glu Ser Asn Gly Gln Pro Glu Asn Asn Tyr Lys Thr
   625                 630                 635                 640
   Thr Pro Pro Val Leu Asp Ser Asp Gly Ser Phe Phe Leu Tyr Ser Lys
                   645                 650                 655
   Leu Thr Val Asp Lys Ser Arg Trp Gln Gln Gly Asn Val Phe Ser Cys
               660                 665                 670
   Ser Val Met His Glu Ala Leu His Asn His Tyr Thr Gln Lys Ser Leu
                       675                 680                 685
   Ser Leu Ser Pro Gly Lys
       690
<210> 32
   <211> 451
   <212> PRT
   <213> Mus musculus
<400> 32
   Gln Glu Gln Leu Val Glu Ser Gly Gly Arg Leu Val Thr Pro Gly Gly
   1               5                   10                  15
   Ser Leu Thr Leu Ser Cys Lys Ala Ser Gly Phe Asp Phe Ser Ala Tyr
               20                  25                  30
   Tyr Met Ser Trp Val Arg Gln Ala Pro Gly Lys Gly Leu Glu Trp Ile
           35                  40                  45
   Ala Thr Ile Tyr Pro Ser Ser Gly Lys Thr Tyr Tyr Ala Thr Trp Val
       50                  55                  60
   Asn Gly Arg Phe Thr Ile Ser Ser Asp Asn Ala Gln Asn Thr Val Asp
   65                  70                  75                  80
   Leu Gln Met Asn Ser Leu Thr Ala Ala Asp Arg Ala Thr Tyr Phe Cys
                   85                  90                  95
   Ala Arg Asp Ser Tyr Ala Asp Asp Gly Ala Leu Phe Asn Ile Trp Gly
               100                 105                 110
   Pro Gly Thr Leu Val Thr Ile Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Ala Ala Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Gly Ala Pro Ile
                   325                 330                 335
   Glu Lys Thr Ile Ser Lys Ala Lys Gly Gln Pro Arg Glu Pro Gln Val
               340                 345                 350
   Cys Thr Leu Pro Pro Ser Arg Asp Glu Leu Thr Lys Asn Gln Val Ser
           355                 360                 365
   Leu Ser Cys Ala Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Val Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 33
   <211> 214
   <212> PRT
   <213> Mus musculus
<400> 33
   Gln Ala Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Thr Ser
               20                  25                  30
   Asn Tyr Ala Asn Trp Val Gln Glu Lys Pro Gly Gln Ala Phe Arg Gly
           35                  40                  45
   Leu Ile Gly Gly Thr Asn Lys Arg Ala Pro Gly Thr Pro Ala Arg Phe
       50                  55                  60
   Ser Gly Ser Leu Leu Gly Gly Lys Ala Ala Leu Thr Leu Ser Gly Ala
   65                  70                  75                  80
   Gln Pro Glu Asp Glu Ala Glu Tyr Tyr Cys Ala Leu Trp Tyr Ser Asn
                   85                  90                  95
   Leu Trp Val Phe Gly Gly Gly Thr Lys Leu Thr Val Leu Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Lys Lys
           195                 200                 205
   Val Glu Pro Lys Ser Cys
       210
<210> 34
   <211> 467
   <212> PRT
   <213> Mus musculus
<400> 34
   Gln Glu Gln Leu Val Glu Ser Gly Gly Arg Leu Val Thr Pro Gly Gly
   1               5                   10                  15
   Ser Leu Thr Leu Ser Cys Lys Ala Ser Gly Phe Asp Phe Ser Ala Tyr
               20                  25                  30
   Tyr Met Ser Trp Val Arg Gln Ala Pro Gly Lys Gly Leu Glu Trp Ile
           35                  40                  45
   Ala Thr Ile Tyr Pro Ser Ser Gly Lys Thr Tyr Tyr Ala Thr Trp Val
       50                  55                  60
   Asn Gly Arg Phe Thr Ile Ser Ser Asp Asn Ala Gln Asn Thr Val Asp
   65                  70                  75                  80
   Leu Gln Met Asn Ser Leu Thr Ala Ala Asp Arg Ala Thr Tyr Phe Cys
                   85                  90                  95
   Ala Arg Asp Ser Tyr Ala Asp Asp Gly Ala Leu Phe Asn Ile Trp Gly
                   100                 105                 110
   Pro Gly Thr Leu Val Thr Ile Ser Ser Ala Ser Thr Lys Gly Pro Ser
               115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Gly Gly Gly Gly Ser Gly Gly Gly Gly Ser Glu Val Gln Leu Leu
   225                 230                 235                 240
   Glu Ser Gly Gly Gly Leu Val Gln Pro Gly Gly Ser Leu Arg Leu Ser
                   245                 250                 255
   Cys Ala Ala Ser Gly Phe Thr Phe Ser Thr Tyr Ala Met Asn Trp Val
               260                 265                 270
   Arg Gln Ala Pro Gly Lys Gly Leu Glu Trp Val Ser Arg Ile Arg Ser
           275                 280                 285
   Lys Tyr Asn Asn Tyr Ala Thr Tyr Tyr Ala Asp Ser Val Lys Gly Arg
       290                 295                 300
   Phe Thr Ile Ser Arg Asp Asp Ser Lys Asn Thr Leu Tyr Leu Gln Met
   305                 310                 315                 320
   Asn Ser Leu Arg Ala Glu Asp Thr Ala Val Tyr Tyr Cys Val Arg His
                   325                 330                 335
   Gly Asn Phe Gly Asn Ser Tyr Val Ser Trp Phe Ala Tyr Trp Gly Gln
               340                 345                 350
   Gly Thr Leu Val Thr Val Ser Ser Ala Ser Val Ala Ala Pro Ser Val
           355                 360                 365
   Phe Ile Phe Pro Pro Ser Asp Glu Gln Leu Lys Ser Gly Thr Ala Ser
       370                 375                 380
   Val Val Cys Leu Leu Asn Asn Phe Tyr Pro Arg Glu Ala Lys Val Gln
   385                 390                 395                 400
   Trp Lys Val Asp Asn Ala Leu Gln Ser Gly Asn Ser Gln Glu Ser Val
                   405                 410                 415
   Thr Glu Gln Asp Ser Lys Asp Ser Thr Tyr Ser Leu Ser Ser Thr Leu
               420                 425                 430
   Thr Leu Ser Lys Ala Asp Tyr Glu Lys His Lys Val Tyr Ala Cys Glu
           435                 440                 445
   Val Thr His Gln Gly Leu Ser Ser Pro Val Thr Lys Ser Phe Asn Arg
       450                 455                 460
   Gly Glu Cys
   465
<210> 35
   <211> 702
   <212> PRT
   <213> Mus musculus
<400> 35
   Gln Glu Gln Leu Val Glu Ser Gly Gly Arg Leu Val Thr Pro Gly Gly
   1               5                   10                  15
   Ser Leu Thr Leu Ser Cys Lys Ala Ser Gly Phe Asp Phe Ser Ala Tyr
               20                  25                  30
   Tyr Met Ser Trp Val Arg Gln Ala Pro Gly Lys Gly Leu Glu Trp Ile
           35                  40                  45
   Ala Thr Ile Tyr Pro Ser Ser Gly Lys Thr Tyr Tyr Ala Thr Trp Val
       50                  55                  60
   Asn Gly Arg Phe Thr Ile Ser Ser Asp Asn Ala Gln Asn Thr Val Asp
   65 70 75 80
   Leu Gln Met Asn Ser Leu Thr Ala Ala Asp Arg Ala Thr Tyr Phe Cys
   85 90 95
   Ala Arg Asp Ser Tyr Ala Asp Asp Gly Ala Leu Phe Asn Ile Trp Gly
   100 105 110
   Pro Gly Thr Leu Val Thr Ile Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Asp Gly Gly Gly Gly Ser Gly Gly Gly Gly Ser Gln Glu Gln Leu Val
   225 230 235 240
   Glu Ser Gly Gly Arg Leu Val Thr Pro Gly Gly Ser Leu Thr Leu Ser
   245 250 255
   Cys Lys Ala Ser Gly Phe Asp Phe Ser Ala Tyr Tyr Met Ser Trp Val
   260 265 270
   Arg Gln Ala Pro Gly Lys Gly Leu Glu Trp Ile Ala Thr Ile Tyr Pro
   275 280 285
   Ser Ser Gly Lys Thr Tyr Tyr Ala Thr Trp Val Asn Gly Arg Phe Thr
   290 295 300
   Ile Ser Ser Asp Asn Ala Gln Asn Thr Val Asp Leu Gln Met Asn Ser
   305 310 315 320
   Leu Thr Ala Ala Asp Arg Ala Thr Tyr Phe Cys Ala Arg Asp Ser Tyr
   325 330 335
   Ala Asp Asp Gly Ala Leu Phe Asn Ile Trp Gly Pro Gly Thr Leu Val
   340 345 350
   Thr Ile Ser Ser Ala Ser Thr Lys Gly Pro Ser Val Phe Pro Leu Ala
   355 360 365
   Pro Ser Ser Lys Ser Thr Ser Gly Gly Thr Ala Ala Leu Gly Cys Leu
   370 375 380
   Val Lys Asp Tyr Phe Pro Glu Pro Val Thr Val Ser Trp Asn Ser Gly
   385 390 395 400
   Ala Leu Thr Ser Gly Val His Thr Phe Pro Ala Val Leu Gln Ser Ser
   405 410 415
   Gly Leu Tyr Ser Leu Ser Ser Val Val Thr Val Pro Ser Ser Ser Leu
   420 425 430
   Gly Thr Gln Thr Tyr Ile Cys Asn Val Asn His Lys Pro Ser Asn Thr
   435 440 445
   Lys Val Asp Lys Lys Val Glu Pro Lys Ser Cys Asp Gly Gly Gly Gly
   450 455 460
   Ser Gly Gly Gly Gly Ser Glu Val Gln Leu Leu Glu Ser Gly Gly Gly
   465 470 475 480
   Leu Val Gln Pro Gly Gly Ser Leu Arg Leu Ser Cys Ala Ala Ser Gly
   485 490 495
   Phe Thr Phe Ser Thr Tyr Ala Met Asn Trp Val Arg Gln Ala Pro Gly
   500 505 510
   Lys Gly Leu Glu Trp Val Ser Arg Ile Arg Ser Lys Tyr Asn Asn Tyr
   515 520 525
   Ala Thr Tyr Tyr Ala Asp Ser Val Lys Gly Arg Phe Thr Ile Ser Arg
   530 535 540
   Asp Asp Ser Lys Asn Thr Leu Tyr Leu Gln Met Asn Ser Leu Arg Ala
   545 550 555 560
   Glu Asp Thr Ala Val Tyr Tyr Cys Val Arg His Gly Asn Phe Gly Asn
                   565                 570                 575
   Ser Tyr Val Ser Trp Phe Ala Tyr Trp Gly Gln Gly Thr Leu Val Thr
               580                 585                 590
   Val Ser Ser Ala Ser Val Ala Ala Pro Ser Val Phe Ile Phe Pro Pro
           595                 600                 605
   Ser Asp Glu Gln Leu Lys Ser Gly Thr Ala Ser Val Val Cys Leu Leu
       610                 615                 620
   Asn Asn Phe Tyr Pro Arg Glu Ala Lys Val Gln Trp Lys Val Asp Asn
   625                 630                 635                 640
   Ala Leu Gln Ser Gly Asn Ser Gln Glu Ser Val Thr Glu Gln Asp Ser
                   645                 650                 655
   Lys Asp Ser Thr Tyr Ser Leu Ser Ser Thr Leu Thr Leu Ser Lys Ala
               660                 665                 670
   Asp Tyr Glu Lys His Lys Val Tyr Ala Cys Glu Val Thr His Gln Gly
           675                 680                 685
   Leu Ser Ser Pro Val Thr Lys Ser Phe Asn Arg Gly Glu Cys
       690                 695                 700
<210> 36
   <211> 246
   <212> PRT
   <213> Homo sapiens
<400> 36
   Met Gly Trp Ser Cys Ile Ile Leu Phe Leu Val Ala Thr Ala Thr Gly
   1               5                   10                  15
   Val His Ser Asp Lys Thr His Thr Cys Pro Pro Cys Pro Ala Pro Glu
               20                  25                  30
   Ala Ala Gly Gly Pro Ser Val Phe Leu Phe Pro Pro Lys Pro Lys Asp
           35                  40                  45
   Thr Leu Met Ile Ser Arg Thr Pro Glu Val Thr Cys Val Val Val Asp
       50                  55                  60
   Val Ser His Glu Asp Pro Glu Val Lys Phe Asn Trp Tyr Val Asp Gly
   65                  70                  75                  80
   Val Glu Val His Asn Ala Lys Thr Lys Pro Arg Glu Glu Gln Tyr Asn
                   85                  90                  95
   Ser Thr Tyr Arg Val Val Ser Val Leu Thr Val Leu His Gln Asp Trp
               100                 105                 110
   Leu Asn Gly Lys Glu Tyr Lys Cys Lys Val Ser Asn Lys Ala Leu Gly
           115                 120                 125
   Ala Pro Ile Glu Lys Thr Ile Ser Lys Ala Lys Gly Gln Pro Arg Glu
       130                 135                 140
   Pro Gln Val Cys Thr Leu Pro Pro Ser Arg Asp Glu Leu Thr Lys Asn
   145                 150                 155                 160
   Gln Val Ser Leu Ser Cys Ala Val Lys Gly Phe Tyr Pro Ser Asp Ile
                   165                 170                 175
   Ala Val Glu Trp Glu Ser Asn Gly Gln Pro Glu Asn Asn Tyr Lys Thr
               180                 185                 190
   Thr Pro Pro Val Leu Asp Ser Asp Gly Ser Phe Phe Leu Val Ser Lys
           195                 200                 205
   Leu Thr Val Asp Lys Ser Arg Trp Gln Gln Gly Asn Val Phe Ser Cys
       210                 215                 220
   Ser Val Met His Glu Ala Leu His Asn His Tyr Thr Gln Lys Ser Leu
   225                 230                 235                 240
   Ser Leu Ser Pro Gly Lys
                   245